# EUROPEAN PATENT SPECIFICATION

(11) **EP 3 681 266 B1**
(45) Date of publication and mention of the grant of the patent: **23.10.2024**
(21) Application number: 18853938.1
(22) Date of filing: 10.09.2018
(51) Int. Cl.: A01G 22/00, C05F 11/00, A01C 21/00, A01H 6/20, A23K 10/30, B01D 53/62, B01D 53/84, C10G 3/00, A01C 7/00

(54) **A METHOD FOR PRODUCING A LOW CARBON INTENSITY BIOFUEL**
VERFAHREN ZUR HERSTELLUNG EINES BIOKRAFTSTOFFS MIT NIEDRIGER KOHLENSTOFFINTENSITÄT
PROCÉDÉ DE PRODUCTION D'UN BIOCARBURANT À FAIBLE INTENSITÉ DE CARBONE

(30) Priority: 11.09.2017 US 201762556575 P
(43) Date of publication of application: 22.07.2020
(73) Proprietor: Nuseed Global Innovation Ltd., M3 3JE (GB)
(72) Inventor: FABIJANSKI, Steven, Rockland, Ontario K4K 0H3 (CA); LINDENBAUM, Michael, Beaconsfield, Québec H9W 5G9 (CA); BENALI, Mejda, Nepean, Ontario K2J 5Z5 (CA)
(74) Representative: Zacco Sweden AB
(86) International application number: PCT/CA2018/051111
(87) International publication number: WO 2019/046968

(56) References cited:
- MASSIMO CARDONE ET AL: "Brassica carinata as an alternative oil crop for the production of biodiesel in Italy: agronomic evaluation, fuel production by transesterification and characterization", BIOMASS AND BIOENERGY, vol. 25, no. 6, 1 December 2003 (2003-12-01), AMSTERDAM, NL, pages 623 - 636, XP055595473, ISSN: 0961-9534, DOI: 10.1016/S0961-9534(03)00058-8
- ZEGADA-LIZARAZU W ET AL: "Energy crops in rotation. A review", BIOMASS AND BIOENERGY, PERGAMON, AMSTERDAM, NL, vol. 35, no. 1, 1 January 2011 (2011-01-01), pages 12 - 25, XP027577811, ISSN: 0961-9534, [retrieved on 20100909], DOI: 10.1016/J.BIOMBIOE.2010.08.001
- JOHNSON, E. N. ET AL.: "Effects of nitrogen fertilizer application on seed yield, N uptake, N use efficiency, and seed quality of Brassica carninata", CANADIAN JOURNAL OF PLANT SCIENCE, vol. 93, no. 6, 2013, pages 1073 - 1081, XP055582006
- PAN, X. ET AL.: "The effect of cultivar, seeding rate and applied nitrogen on Brassica carinata seed yield and quality in contrasting environments", CANADIAN JOURNAL OF PLANT SCIENCE, vol. 92, no. 5, 2012, pages 961 - 971, XP055582007

## Description

### Field of invention

The invention is in the field of agriculture and teaches a novel method comprising cultivation of a *Brassica carinata* oilseed crop as a replacement for fallow or existing cover crops used in crop rotations, utilizing new agricultural practices that preserve the soil benefits of traditional cover crop or fallow rotation but enable the harvest of an oil rich grain that provides feedstock for producing low carbon intensity biofuel while concomitantly allowing for greater reductions of lifecycle Greenhouse Gas (GHG) emissions, and for sequestration of carbon in soil

### Background

Over-reliance on fossil fuel for transportation, energy generation, home heating, industrial power source, *etc.,* has resulted in an ever-increasing rate of CO₂ and GHG emission and accumulation in the atmosphere. This has led to the threat of global warming and the undesirable consequences thereof. One strategy to reduce the rate of increase of CO₂ and other greenhouse gases in the atmosphere would be to reduce reliance on fossil fuels by replacing them with more sustainable fuels such as those derived from plant oils and biomass, which are less carbon intensive over their entire lifecycle.

In order to control greenhouse gas emissions, governments have enacted regulations that attempt to curtail the rate of growth of carbon emissions to agreed- upon target levels within their jurisdictions. To allow for these regulations to be enforceable, mechanisms and methodologies to accurately audit the lifecycle greenhouse gas emissions produced for specific fuels "from well to tank" have been developed. Concomitantly, measures to induce emitters to respect these targets (carbon taxes, cap and trade systems) are also being put in place. The net result of these measures has been to establish a pricing system for carbon that must be borne by those that emit carbon.

Industries such as those which produce and rely on fuels and energy sources are particularly targeted by these policies. Fuel producers and, by extension, biofuel producers are thus heavily incented to identify feedstocks, fuels and manufacturing processes to respect the imposed targets and minimize the impact of carbon pricing on their bottom line.

The need to reduce carbon emissions and the strong incentives to industry to achieve carbon emission reductions in their sectors has been important factors leading to the development of new low Carbon Intensity pathways. However, pricing of the next generation fuels in relation to conventional fuels has served as an impediment to their larger scale adoption. With the advent of carbon pricing, some of this disincentive has been removed and it follows that increasing the differential in Carbon Intensity of biofuels versus conventional fuels may be one factor that can significantly impact price differentials.

Carbon Intensity (CI) is defined as "a measure of the greenhouse gas (GHG) emissions of a fuel, determined using Life-Cycle Assessment (LCA). LCA identifies and estimates all GHG emissions in producing a fuel; from the growing or extraction of raw materials, to the production of the fuel, through to the end use of the fuel. Carbon intensity is reported as the mass of carbon dioxide equivalent greenhouse gases emitted per unit of energy contained in the fuel, in units of grams of carbon dioxide equivalent per megajoule of energy (gCO2e/MJ)" ("Determination of Carbon Intensity for the Renewable and Low Carbon Fuel Requirements Regulation (Information Bulletin RLCF-006)" Section 2, page 3 entitled "What is carbon Intensity?" Published by the Department of Energy and Mines, Government of British Columbia. Issued December 2010, revised July 2013). In 2017, the baseline CI values reported for fossils fuels, according to the European Union Renewable Energy Directive (EU-RED), and for biodiesel, according the US Renewable Fuel Standard (US RFS) were, respectively, 83.8 (EU-RED) g CO_{2eq}/MJ energy produced and 91.8 g CO_{2eq}/MJ energy produced (US RFS), as reported in Table 3 of DeJong *et al.,* 2017. Those skilled in the art are aware that CI values for both fossil fuels and biofuels can change as LCA models and production methods evolve. The current CI for various biofuel pathways may be found at (https://www.arb.ca.gov/fuels/lcfs/fuelpathways/current-pathways-01 102017. xlsx).

Agricultural production provides an appropriate methodology for producing the next generation biofuels. Modern agriculture produces food, feed and fiber at enormous scale and can be mobilized to provide feedstocks for fuel production, without any need for development of new production technologies or infrastructure. One attractive feature of agricultural production is its exploitation of a plant's ability to utilize and fix atmospheric carbon dioxide via photosynthesis and so acts as an important sink for carbon. The carbon accumulated in an annual crop's biomass will eventually be remobilized, part of it as harvested material, and the remainder as crop residues (leaves, stalks, stems, roots) which undergo degradation by soil borne bacteria and fungi. Some of this soil assimilated carbon is used as an energy source by soil microbes and will ultimately be respired as gaseous carbon dioxide, however some will also be retained stably in the soil, an important sink for sequestering of carbon and reduction of emissions into the atmosphere. Of all the environmental pools of carbon, soil is second in size only to the oceans, and comprises an estimated content of more than 2.3 GT of organic carbon (Jobbagy and Jackson, 2000) representing more than 4 times the amount of carbon accumulated in total plant biomass. An added benefit of restoring carbon to soils is the subsequent improvement of soils fertility and structure.

However, while annual crops sequester carbon during their life span and also return a significant portion of the accumulated carbon to the soil for longer term sequestration, their cultivation can also directly and indirectly result in emission of CO₂ and CO₂ equivalent greenhouse gases. These emissions occur throughout the crop's cultivation, subsequent conversion of the crop to feedstock, conversion of the feedstock to liquid fuel, storage and transportation of feedstock and finished fuel and finally distribution and utilization of the fuel. The greenhouse gas emissions associated with the elevation of the crop occur comprise the stages of seed development, field preparation, manufacturing and application of crop inputs (fertilizers, pesticide/herbicides/ seed treatments), crop seeding, crop maintenance and crop harvest, storage of the harvested material and storage and transport to the processing plant.

To account for the flux of carbon dioxide and other greenhouse gases over the entire lifecycle of an energy crop's cultivation, harvest, and conversion to biofuel, auditing methodologies such as the "Greenhouse Gases, Regulated Emissions, and Energy Use in Transportation (GREET)" model (Wang 1996), GHGenius (S&T Squared. GHGenius, Model Version 4.03; www. ghgenius.ca; S&T squared Consultants Inc. for Natural Resources Canada: Delta, British Columbia, 2017), based on a previously developed model (DeLucchi 1991), BioGrace (www.biograce.net; Neeft *et al.,* 2012) and others have been developed. These allow for more valid comparisons between the overall GHG impact of biofuel production and utilization versus that of fossil fuels and also permit comparisons to be made between biofuels manufactured from different types of energy crops. The ability to accurately model and predict GHG emissions over the entire lifecycle of biofuel production has enabled a value to be assigned the production of carbon. As a consequence of carbon pricing, National and international agreements have been negotiated to achieve reduction of carbon/GHG emissions to specified targets. The Renewable Energy Directive (RED) in the EU and the Renewable Fuel Standard (RFS) and California Low Carbon Fuel Standard (CA-LCFS) in the USA are examples of such policies.

Table 1 compares published carbon intensity (CI) values for selected Biofuel pathways and compares them to those of conventional gasoline and/or diesel fuel. As can be seen, the FAME (Fatty Acid Methyl Ester) biodiesel pathways have CI values ranging from 67.32 to 51.35 g CO_{2eq}/MJ compared to a CI of 102.4 g CO_{2 eq} /MJ for conventional diesel, demonstrating the significant reduction in CI afforded by FAME Biodiesel pathways over their petroleum-based equivalents. Furthermore, with a CI of 44 g CO_{2 eq} /MJ, renewable or green diesel produced via hydrotreating of rapeseed oil affords additional reduction in overall pathway carbon intensity over that of the FAME process.

**Table 1: Carbon Intensities of selected biofuel pathways**

| **Feedstock** | **Fuel Pathway** | **Carbon Intensity (g CO_{2 eq} /MJ)** | **Data Source** | **Model used** |
|---|---|---|---|---|
| Petroleum | Gasoline | 93.3 | DeJong, *et al.,* 2017 | GREET |
| Petroleum | Diesel | 91.8 | | |
| Canola/ rapeseed oil | Biodiesel (FAME) | 67.32 | CA ARB* | |
| Canola/ rapeseed oil | Biodiesel (FAME) | 51.35 | | |
| Canola/ rapeseed oil | Biodiesel (FAME) | 52 | | BIOGRACE |
| Canola/ rapeseed oil | HVO*** | 44 | | BIOGRACE |

| | | | | |
|---|---|---|---|---|
| *https://www.arb.ca.gov/fuels/lcfs/fuelpathways/current-pathways-01102017.xlsx **http://eur-lex.europa.eu/legal-content/EN/ALL/?uri=CELEX%3A32009L0028 *** Hydrotreated Vegetable Oil | | | | |

In a study focused solely on GHG emissions from cultivation of canola in the Canadian prairies over the period of 1986 to 2006, the authors (Shrestha, *et al.,* 2014) demonstrated that the GHG emissions decreased on a per area basis by 40% and on a grain-dry matter basis by 65% in that time interval. The decrease was due to a combination of factors including reduced land use change, increased grain yields and increased sequestration of soil organic carbon through improved land management. In 2006, soil carbon sequestration in this region represented on average almost 500 kg CO₂/ha.

However, there still exists a need for a dedicated feedstock crop whose production can be scaled to meet demand as a high-quality feedstock for liquid biofuels such as biodiesel, green diesel and jet fuel replacements. Whereas other high yielding and productive oilseed crops have been suggested as potential sources of feedstock, the most established species and varieties, such as canola type *Brassicas* or soybean, produce edible oils which command a cost premium relative to dedicated biofuel feedstocks and which would also reduce supply of edible oils.

For example, diversion of canola or low erucic rapeseed to production of significant amounts of biofuel would almost certainly lead to land use change in order to make up for the shortfall in edible oil production. Moreover, the price premium accorded high quality edible oils might be expected to drive the price of canola feedstock for biofuel applications to uncompetitive levels.

Soybean, whose oil has been used as a feedstock for biofuel production, is a cool season legume crop grown throughout much of North America, South America and Asia. As a source of edible oil, soybeans also currently account for more than 60 percent of the edible oils consumed in the U.S. (Data taken from Table 20: United States Oilseeds and Products Supply and Distribution Local Marketing Year and Table 21: United States Soybeans and Products Supply and Distribution Local Marketing Year; (https://apps.fas.usda.gov/psdonline/circulars/oilseeds.pdf)). Competition between its use as an edible oil and as a biofuel feedstock has led to price volatility, which likely diminishes the economic case for its desirability as biofuel feedstock (Wisner 2010). Moreover, substantial diversion of edible oils into biofuel applications would almost certainly trigger indirect land use change emissions as a consequence.

Palm oil, another major feedstock for production of biofuels, is grown in Asia and South America. However, palm oil faces significant hurdles in many jurisdictions due to land-use changes incurred due setting up palm plantations in sensitive ecosystems. Use of palm oil has been associated with high levels of GHG emissions due to the massive deforestation resulting from establishment of monoculture palm plantations. The so-called certified sustainable palm oil, or palm oil produced per the standards of the Roundtable of Sustainable Palm Oil (RSPO), is distinguished from non-certified palm oil by the producer's commitment to preserve and conserve high value natural forests. The sustainable palm oil is, however, considerably more expensive than non-certified palm oil which constitutes a disincentive to its use as bio-fuel feedstock.

A member of the Brassicaceae (formerly Cruciferae) family, *Brassica carinata* is also known as carinata, Ethiopian mustard, Abyssinian mustard, African Sarson and Gomenzer. In addition to *B. carinata,* the *Brassica* genus includes several economically important oilseed crop species: *B. juncea* (L). Czern. (brown mustard), *B. napus* L. (rape, Argentine canola), *B. nigra* (L.) W.D.J. Koch (black mustard), and *B. rapa* L. (field mustard, Polish canola) and also includes *B. oleracea* L. food crops, including cabbage, broccoli, cauliflower, Brussels sprouts, kohlrabi and kale. The six *Brassica* species are closely related genetically, as described in the Triangle of U (Nagaharu 1935). The native range of *Brassica carinata* comprises the central highland region of Ethiopia; however, recent efforts have exploited the inherent genetic variation in carinata to produce varieties that are productive in more diverse agricultural settings, including semi-arid zones or regions where more marginal agricultural land may predominate.

*Brassica carinata* produces abundant spherical seed, 1-1.5 mm in diameter (Mnzava and Schippers 2007) varying from yellow to yellow-brown to brown in color (Alemaw 1987, Rahman and Tahir 2010). The seeds are rich in oil, containing 37-44% oil content based on seed dry weight, depending on the cultivar and growing conditions. The seed protein content is also high, at 25-30% expressed as seed dry weight (Pan *et al.,* 2012). Unlike canola, *Brassica carinata* produces a non-edible oil.

In Spain and Italy, carinata seed oil has been used for biofuel (Cardone *et al.,* 2002, CARDONE, M et al.: "Brassica carinata as an alternative oil crop for the production of biodiesel in Italy: agronomic evaluation, fuel production by transesterification and characterization",BIOMASS AND BIOENERGY, vol. 25, no. 6, 1 December 2003 (2003-12-01), pages 623-636, Bouaid *et al.,* 2005, Gasol *et al.,* 2007, Gasol *et al.,* 2009) and as a bio-industrial feedstock with many uses (*i.e.,* in lubricants, paints, cosmetics, plastics). In North America, carinata has been assessed as a biofuel feedstock (Drenth *et al.,* 2014, Drenth *et al.,* 2015), and crude oil produced from *B. carinata* seed has been used to produce green or renewable diesel, biodiesel and bio-jet fuel (Drenth *et al.* 2014). In October 2012, experimental aviation flights by the National Research Council of Canada using the world's first 100% bio-jet fuel were successful ("ReadiJet 100% biofuels flight - one of 2012's 25 most important scientific events", Popular Science Magazine, 2012(12).

Blackshaw and co-workers compared several oilseed species for suitability as sources of FAME biodiesel in Western Canada (Blackshaw *et al.,* 2011). In trials carried out at 5 sites in western Canada (during the years 2008-2009), a number of oilseed species and varieties including 3 varieties of canola (comprising one each of *Brassica napus, Brassica rapa* and *Brassica juncea* canola types), *Brassica carinata, Camelina sativa,* oriental mustard (juncea), yellow mustard *(Sinapis alba),* soybean and flax were evaluated for yield and oil feedstock quality. Based on the results of these studies, *Brassica carinata* exceeded *Brassica napus* canola (the check line) yields in only 1 of nine 9 site -years, which constituted the lowest aggregate yield ranking of all entries tested in these trials, whereas in oil content, *Brassica carinata* ranked third lowest (higher only than condiment mustard and soybean). However, it should be noted that in this study the carinata variety used was a heterogeneous "common" variety and not a commercial elite variety.

By contrast, in a comparison of *Brassica* oilseed varieties carried out in Minnesota in 2012-2013, Gesch *et al.* (2015) purported to demonstrate that the new commercial carinata varieties produced comparable grain yields to commercial canola type *Brassica napus* varieties while producing almost twice the above-ground biomass of the napus varieties. Gesch *et al.* teach that the lower seed to above-ground biomass ratio (Harvest Index) of the carinata crops and suggests that there is scope for grain yield improvement through selective breeding. However, Gesch *et al.* do not teach that the higher biomass of *Brassica carinata* can provide benefits in terms of potential for return of additional carbon to the soil.

Johnson and colleagues teach that carinata grain and biomass yields correlate positively with increasing nitrogen fertilizer application, and under the conditions studied (up to 160-200 kg N/ha, depending on experiment) the maximal yields of straw and grain did not plateau (Johnson *et al.,* 2013). This might be taken to indicate that very high levels of nitrogen might be required for production of carinata grain; however, they also purportedly demonstrated that under conditions of high preexisting soil nitrogen mineralization, high grain yields could be obtained without added nitrogen fertilizer. On the other hand, Johnson *et al.* provided no teachings related to the potential positive effect of including *Brassica carinata* in crop rotations with legume crops such as lentils, peas or soybean which fix nitrogen and increase soil nitrogen mineralization, reducing the requirement for nitrogen fertilizer and the carbon intensity of carinata production.

As a first attempt at establishing the carbon footprint for cultivation of *Brassica carinata,* a GHG life cycle analysis was carried out on a bioenergy cropping system for carinata based on the use of the entire harvested above-ground biomass (including grain) as a lignocellulosic power generation system (Gasol *et al*., 2007). Based on an estimate of the carbon associated with its extensive root system, Gasol *et al.* teach that as much as 631 kg CO₂/ha could be translocated into the soil, contributing to a reduction of atmospheric CO₂ equivalent emissions of up to 71% relative to the baseline natural gas power generation system. However, Gasol *et al.* did not consider the potential for additional return of nutrients to the soil by return of above-ground biomass after harvest and collection of carinata grain, nor did they consider the use of the grain for extraction of feedstock for biofuel manufacturing and meal co-product for use as high protein animal feed additive.

While the references cited above teaching that *Brassica carinata* may be a suitable dedicated feedstock crop for biofuel production, how this feedstock might be produced from carinata in a multiplicity of regions, soil conditions and crop rotations so as to achieve the lowest possible and most advantageous Carbon Intensity for a biofuel pathway is unknown.

### Summary

As a means to reduce reliance on use of fossil fuels and the consequential increase in greenhouse gas emissions and to contribute to sustainable agriculture, the invention described herein comprises methods for cultivation of *Brassica carinata*, a crop which yields an oil from its harvested seed that is a feedstock for production of biofuel to replace fossil fuels and as well produces a high-quality protein rich meal as by-product that can be used in commercial livestock feed rations.
The present invention is as set out in the appended claims. More specifically, the invention describes cultivation methods to produce the crop using optimal agronomic and land management practices applied in a multiplicity of climate zones and regions, allowing for substantial reduction in atmospheric CO₂ and GHG emissions relative to an equivalent quantity of fossil fuel.

*Brassica carinata* can be sustainably cultivated in a variety of environments for production of high quality biofuel feedstock while simultaneously
a. reducing GHG emissions associated with the production of the feedstock as well as subsequent manufacturing of biofuel;
b. increasing the carbon content of soil in which it is cultivated;
c. providing conditions for improved yields of crops with which it is produced in rotation; and
d. achieving the preceding with little or no increase in land use change.

These attributes allow for credits to be accrued via schemes or programs designed to assign value to emitted carbon such as the RFS program in the US and the RED program in the EU. Such programs also allow the value of carbon produced in the production and utilization of fuels to be monetized in such a way as to reduce the current differential in price between fossil fuels and alternative biofuels. The end result is the recognition of carbon as a primary commodity of value. Similarly, the notion of carinata as a crop that is being produced and valued as source of a particular commodity *(i.e.,* as feedstock for biofuel production) is being replaced with one whose value represents a desirable balance of carbon release versus carbon abatement. In this light, the production of carinata represents a new category of agricultural production, namely one that can be described as carbon farming.

The current invention provides cultivation of carinata in specific climatic and soil zones, and geographic regions, using particular agricultural and land management practices to provide sustainable feedstock for biofuel and feed while providing measurable benefits in the form of reduced greenhouse gas emissions, improved soil structure and improved performance of following crops which are grown with carinata in rotation.

Unlike canola, *Brassica carinata* produces a non-edible oil and its production can be carried out on marginal lands or as part of a crop rotation replacing summer or winter fallow, which would entail minimal displacement of food crops and little or no concomitant land use change.

In embodiments, there are provided methods for cultivation of *Brassica carinata* varieties to enable short daylength winter cropping in temperate or subtropical regions and long day length summer cropping in cool temperate dry regions.

In some embodiments, there are provided conditions of cultivation whereby carinata is maintained on previously cultivated land, as a replacement for fallow and in a rotation following or preceded by pulse, legume or cereal.

In still other embodiments, there are provided agronomic and land management practices for cultivation and harvest of *Brassica carinata* oilseed grain including usage of fertilizer, herbicide and pesticide applications, seeding rates and seeding depths to achieve optimal grain and biomass.

In additional embodiments, there are provided land management practices for carinata cultivation, such as return of above-ground and below-ground carinata plant biomass to the field to maximize soil carbon levels. The extent of carbon accumulation achievable with carinata is an unexpected finding. Whereas other oilseeds such as canola are optimized for grain production by breeding for varieties that that will channel the plants energy input into seed production at the expense of biomass production, *Brassica carinata* achieves high levels of grain production and biomass production concurrently. The increased biomass so produced incorporates a larger amount of carbon and can subsequently return a larger portion of this carbon to the soil post-harvest.

In other embodiments, there are provided conditions for cultivation of *Brassica carinata* to yield a grain whose oil is used as a feedstock for biofuel manufacturing, such as for HVO, while producing a meal as by-product of oil extraction with protein, carbohydrate, fiber and energy as described to be used as animal feed.

In other embodiments, there are provided methods for producing feedstock for the production of a low carbon intensity biofuel. The carbon intensity of the biofuel produced from feedstock thus produced can be negative, providing for enhanced greenhouse gas reduction.

In some environments, where the winter may be too severe to support cultivation of crops, *Brassica carinata* may be planted immediately after the winter as soon as soil temperatures permit, as part of a rotation where the *Brassica carinata* replaces a spring/summer fallow that would normally follow the crop harvested before the intervening winter.

In some embodiments, the method comprises planting a *Brassica carinata* variety immediately following the harvest or concomitant with the harvest of a first crop for sequential crop production without an intervening fallow period

According to the invention, the method further comprises processing the grain to produce oil whereby the oil is used as a feedstock to produce low carbon intensity biofuels.

In some embodiments, the method further comprises processing the grain such that after extraction of the oil fraction there remains a low fiber, protein rich meal fraction that can be used as a protein rich feed additive for livestock production.

In some embodiments, a new crop that is not *Brassica carinata* is planted immediately after or concomitant with the harvest of *Brassica carinata* without an intervening fallow period, hence increasing the productivity of the land while adding additional carbon to the soil. As a result, the overall greenhouse gas emissions associated with agriculture are reduced.

It is generally understood by those skilled in the art of agricultural production that a fallow period is a common practice in many regions. It is also generally understood by those skilled in the art that a fallow period can often involve leaving the land untreated for a period of time equal to the typical period when the first crop is grown, or that a fallow period can also comprise the planting of a cover crop to control soil erosion or aid in the prevention of the growth of undesirable plants such as weeds. In each case, the term fallow is used to broadly describe a period of time when the land is not used for the production of a first crop, but rather is managed to either have no planted crop or seeded with a plant or crop that is simply used to provide a plant cover over the ground. For each region of agriculture, the timing and duration of fallow will be different as climate can vary and practices change from region to region and this is generally apparent to those skilled in the agricultural arts; however, fallow is a term to describe a portion of time when the ground is considered to be non-productive.

There are a number of cover crops that have been used during a fallow period, ranging from wheat, rye, other grasses and even crops that produce oil in the seed like *Brassica napus, Brassica juncea, Camelina* and *Lesquerella* (meadowfoam). However, crops such as wheat and rye and other grasses do not produce oil that can be used for low carbon intensity fuels, whereas crops such as *Camelina* and *Lesquerella* do not produce significant levels of biomass to enable enough carbon to be captured and provide for the greenhouse gas savings that is seen for *Brassica carinata.* For example, it was shown (Gesch *et al.,* 2015) that *Brassica carinata* can produce up to 2 times the biomass of *Brassica napus* and more than 4.5 times the biomass of *Camelina* under typical planting conditions where the use of fallow is common. In the present invention, we have demonstrated an unexpected and advantageous result that *Brassica carinata* can be substituted for a fallow period and provide for more carbon to be added to the soil, with the added advantage of recovering a grain that can be used for the production of low carbon intensity fuels.

It is an object of the present invention to provide a method where fallow is avoided and replaced with a *Brassica carinata* using practices that maximize the capture of atmospheric carbon that is added to the soil upon harvest. These practices can include reduced fertilizer and reduced use of added water for example irrigation. The unique properties of *Brassica carinata,* which include enhanced tolerance to extreme climatic changes such as frost events or heat events, make it possible to cultivate *Brassica carinata* in regions where other oilseeds cannot grow or will not yield a harvestable product such as a grain that contains a high percentage of oil in the grain.

In typical fallow protocols, the plant material that grows during fallow is simply tilled into or killed by herbicides at the end of the fallow period, to be incorporated into soil. The present invention provides for a more advantageous use of land, more atmospheric carbon being provided to the soil through the *Brassica carinata* biomass, and the added advantage of harvesting a grain that comprises an oil that can be used to produce low carbon intensity fuels.

In some embodiments, the invention provides a method for producing grain for use in the production of a plant-based oil feedstock for low carbon intensity biofuels; for adding carbon in soil; and/or acquiring a carbon credit.

### Description of the Drawings

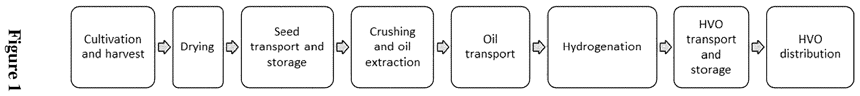

Figure 1 shows the steps in the production of HVO (Hydrotreated Vegetable Oil) from cultivated *Brassica carinata* grain that are within the "well to tank" system boundary of the BioGrace GHG emissions calculator. The allocation factor for the first three steps is 0.613. The yield of HVO is 0.58 MJ/MJ carinata seed.

### Definitions

In the Description, Examples, and Tables which follow, a number of terms are used. To aid in a clear and consistent understanding of the invention, the following definitions are provided.

Biofuels are fuels produced from feedstocks derived from a biological (plant, animal or bacterial) hydrocarbon source as opposed to fuels derived from a fossil source. Types of biofuels include those classified as
a. First generation: in general, first generation biofuels are those derived from biomass obtained from food crops, for example ethanol produced from fermentation of starches and sugars or biodiesel produced from transesterification of edible vegetable oils.
b. Second generation: second generation biofuels are those made from feedstocks derived from biomass of dedicated non-food energy crops or from harvest residues of food crops. In the former category are feedstock oils from dedicated energy crops such as jatropha while the latter category includes lignin, cellulose and hemicellulose from corn stover, sugarcane bagasse etc.
**c. Advanced or third generation biofuels:** biofuels produced from algal feedstocks.

Biomass is defined broadly as organic derived material that comprises a living, or recently living organism. Above-ground plant biomass comprises the entirety of biomass associated with the portion of a plant that is above the soil surface at the time of sampling. Likewise, below-ground plant biomass comprises the entirety of biomass associated with the portion of a plant that is below the soil surface at the time of sampling. It follows that total plant biomass is defined as the sum of above-ground biomass and all below-ground biomass at the time of sampling.

Carbon intensity (CI) refers to the quantity of greenhouse gas (GHG) emissions produced by a unit quantity of fuel over its entire lifecycle compared to the energy evolved when that unit of fuel is burned. GHG production is determined by a thorough life cycle analysis (LCA) which enumerates all GHG emissions released in a unit of fuel's production and utilization. For biofuel, this will include all emissions produced as a result of growing the crop and subsequently transporting the harvested material, subsequent conversion of the harvested material to fuel feedstock, storage and transport of transport of the feedstock, production of fuel from the feedstock, storage and distribution of the fuel, through to the end use of the fuel. CI is reported as the mass of carbon dioxide equivalent greenhouse gases emitted per unit of energy contained in the fuel, in units of grams of carbon dioxide equivalent per megajoule of energy produced (gCO₂e/MJ).

**Carinata** refers to seeds or plants of the species *Brassica carinata* containing both the B genome from *Brassica nigra* and the C genome from *Brassica oleracea* (Nagahuru, 1935).

**Cereals, or cereal crop(s),** is the term applied to grasses that are cultivated for their grain including, but not limited to, barley, corn, oats, rice, rye, and wheat.

**Climate zones, climate (climatic) regions, climate,** as used herein, are terms referring to geographical divisions of the earth's surface that delineate regions based on similarities in historical average temperature, precipitation and seasonal variations. The climate zones used in this application are derived from those described in the EU document entitled " COMMISSION DECISION of 10 June 2010 on guidelines for the calculation of land carbon stocks for the purpose of Annex V to Directive 2009/28/EC", which in turn are based on climate zones established by the IPCC (IPCC 2006, 2006 IPCC Guidelines for National Greenhouse Gas Inventories, Prepared by the National Greenhouse Gas Inventories Programme, Eggleston H.S., Buendia L., Miwa K., Ngara T. and Tanabe K. (eds). Published: IGES, Japan.). The zones are defined based on commonalities in elevation, mean annual temperature (MAT), mean annual precipitation (MAP), mean annual precipitation to potential evapotransporation ratio (MAP:PET), and frost occurrence. There are 12 climate regions: Tropical, montane; Tropical, wet; Tropical, moist; Tropical, dry; Warm temperate, moist; Warm temperate, dry; Cool temperate, moist; Cool temperate, dry; Boreal, moist; Boreal, dry; Polar, moist; and Polar, dry (described in Table 2 below).

**Table 2: IPCC climate zone definitions**

| **Climate Zone** | **Defining characteristics** |
|---|---|
| Tropical Montane | • Mean annual temperature greater than 18°C |
| | • Up to 7 days of frost per year |
| | • Elevation greater than 1000m above sea level |
| Tropical Wet | • Mean annual temperature greater than 18°C |
| | • Up to 7 days of frost per year |
| | • Mean annual precipitation greater than 200cm |
| | • Less than 3 months dry, during winter |
| Tropical Moist | • Mean annual temperature greater than 18°C |
| | • Up to 7 days of frost per year |
| | • Mean annual precipitation between 100 and 200cm |
| | • Less than 3-5 months dry during winter |
| Tropical Dry | • Mean annual temperature greater than 18°C |
| | • Up to 7 days of frost per year |
| | • 5-8 months dry during winter |
| Warm Temperate Moist | • Mean annual temperature greater than 10°C |
| | • Humid; no dry season |
| | • Ratio of mean annual precipitation to potential evapotranspiration greater than 1 |
| Warm Temperate Dry | • Mean annual temperature greater than 10°C |
| | • Seasonally dry; winter rains |
| | • Ratio of potential evapotranspiration to mean annual precipitation is greater than 1 |
| Cool Temperate Moist | • Mean annual temperature greater than 10°C (4-8 months at a temperature greater than 10°C) |
| | • Ratio of mean annual precipitation to potential evapotranspiration greater than 1 |
| Cool Temperate Dry | • Mean annual temperature greater than 10°C (4-8 months at a temperature greater than 10°C) |
| | • Ratio of potential evapotranspiration to mean annual precipitation is greater than 1 |
| Boreal Moist | • 3 months or less at a temperature of 10°C or greater |
| | • Ratio of mean annual precipitation to potential evapotranspiration greater than 1 |
| Boreal Dry | • 3 months or less at a temperature of 10°C or greater |
| | • Ratio of potential evapotranspiration to mean annual precipitation is greater than 1 |
| Polar Moist | • All months at a temperature of less than 10°C |
| | • Ratio of mean annual precipitation to potential evapotranspiration greater than 1 |
| Polar dry | • All months at a temperature of less than 10°C |
| | • Ratio of potential evapotranspiration to mean annual precipitation is greater than 1 |

**Combining** refers to the process of reaping and collecting the seed pods from the matured crop, threshing the seed pods to release the seed (grain), and winnowing to separate and recover the grain from the now empty seed pods, stems, and branches (collectively referred to as chaff). These once distinct operations are today often "combined" by use of a multifunctional mechanized apparatus, appropriately known as a "combine" harvester.

**Cover crops** are annual plants grown primarily to enrich or improve the soil. They act by preventing soil degradation due to erosion and leaching of nutrients from the root crops root zone. They can also help to conserve soil moisture, act as reservoirs to maintain soil nutrients, improve soil structure by increasing its carbon content and act as disease breaks to prevent persistence of plant pathogens. By their nature, cover crops are planted during one season to conserve or improve the soils potential to support the growth of the next season's crop. Typically, cover crops are not grown in the expectation of yielding a harvestable material that has an inherent economic value, such as the ability to be used for production of food, fuel or fiber. Rather the plant matter produced by the cover crop is usually incorporated into the soil during or by the end of its lifecycle. By way of contrast, *Brassica carinata,* while providing similar benefits to soil as well as to succeeding crops, is harvested to yield an oil rich grain which can be processed to a feedstock used in biofuel production, as well as a protein rich meal that can be used in animal feed applications, and which provides a direct economic return to the farmer.

**Cultivation:** Cultivation refers to the conditions under which a crop is seeded, maintained and harvested. For carinata, factors for cultivation include the following:
a. **Time of seeding:** carinata is a mid- to long-season crop that requires a slightly longer growing season than other mustard types. Hence seeding early provides the best results. The ideal seeding date depends greatly on geography and weather. However, in general soils should be at least 40 F or higher before planting.
   i. Canadian Prairies and US northern tier: typical planting occurs in spring between early April to late May.
   ii. South Eastern US: typical planting occurs in fall between October and December.
   iii. South America (Uruguay): the optimal planting time occurs in fall or winter (*i.e.,* typically between beginning of May to end of June).
b. **Seeding:** Soil type and previous cropping history will influence the type of tillage necessary to prepare the seedbed. Reduced or minimum tillage may increase water conservation, soil organic matter, fuel efficiency, and erosion control. Carinata can be planted into conventionally or minimally tilled soil, or it may be no-till planted in standing stubble. Seeding is carried out at a rate designed to achieve plant densities in a range from 80 to 180 plants per square meter. *B. carinata* may be seeded at a consistent depth of 0.5 to 5.0 cm-for example, at a depth of 1.35 to 2.5 cm.
c. **Fertility:** The fertility requirements of carinata are similar to other mustards and canola. Adequate availability of nitrogen, phosphorous, potassium and sulfur are required to achieve the true yield potential. Manure may be used as a source of organic nitrogen to replace some or all of the recommended inorganic nitrogen for growth of *Brassica carinata* in specific soil composition. Fertilizer rates vary with growing zone and soil fertility.
d. **Tillage:** Carinata can be planted into conventionally tilled soil where conventional tillage or full-tillage comprises a substantial soil inversion repeated several times yearly such that few plant residues remain at the soil surface at the time of seeding. Alternatively, carinata can planted into soil that is maintained under conservation tillage practices whereby the extent and frequency of tillage is substantially reduced with respect to conventional tillage (so- called medium or low tillage soil management) or it may be no-till planted in standing stubble. A fuller description of each tillage practice is provided below, in the definition of land management practices.
e. **Moisture:** For good stand establishment, carinata requires adequate soil moisture at seeding and through emergence but can tolerate reduced moisture thereafter and stands up well to the semi-arid mid-summer conditions of the Southern Canadian prairies.
f. **Temperature:** Carinata is a temperate climate crop but which has been adapted to the more extreme conditions experienced in the southern Canadian prairies and Northern Tier US states. During initial stand formation, carinata can recover from short term frost conditions and tolerates higher heat during flowering and seed set better than other *Brassica* oilseeds.
g. **Herbicides:** Carinata is an aggressive crop and will out-compete many weeds if it establishes well. Some weed species, however, if allowed to establish early and persist, can affect quality and yield of all crops, including carinata. Examples of weeds that can adversely affect carinata yield and quality include cochia, wild mustard, and wild radish. Weed management is thus an important aspect of modern agricultural practice and comprises several different but complementary approaches including physical methods to remove weeds before seed can be set, such as cultivation, tilling and rogueing of fields as well as use of chemical agents or herbicides to suppress or kill weedy species before they become established and/or are able to set and release their seed. Herbicides are a class of pesticide and comprise a large group of chemical compounds that interfere with specific biological processes of the plants in such a way as to block their growth and survival. Herbicides are grouped into classes defined by the biological process they interact with. These can include inhibition of lipid biosynthesis, inhibition of amino acid biosynthesis, hormonal regulation of plant growth, inhibition of photosynthesis, inhibition of nitrogen metabolism, inhibition of plant pigments biosynthesis or function, agents which can disrupt cell membranes and agents which inhibit seedling growth (Sherwani *et al.,* 2015). In general, different compounds and herbicide classes may display varying degrees of efficacy against certain weedy species. Moreover, some crop species may display more tolerance to certain classes of herbicide than others. Thus, in a particular geographical region, the use of a particular herbicide for weed control may be dictated by the nature of the crop being cultivated and the native weeds encountered in the region. A particular herbicide might thus be designated as being registered for use with a crop based on its record of performance and proven ability to control relevant weeds without significantly impacting crop yield. The registered usage also specifies specific methods of application of the herbicide, including recommended concentration of herbicide, use of appropriate diluents, adjuvants, surfactants, etc., method of delivery (*i.e.,* spray versus granular), timing of application at appropriate crop stage to ensure least crop damage, timing of application and number of applications to ensure optimal weed control, location of application (foliar or soil application), recommended weather conditions for optimal weed control. Some examples of herbicides recommended for use with *Brassica carinata* grown in SE USA are listed (https://agrisoma.com/ckfinder/userfiles/files/2017_18_SE_Handbook.pdt). The aforementioned is cited as an example and is not meant to limit in any way the scope of the invention.
h. **Fungicides** constitute a class of pesticide comprising a diverse set of chemical agents which can prevent or reduce the severity of plant infection by pathogenic fungi. As is the case for herbicides, there are numerous classes of fungicides. FRAC (Fungicide Resistance Action Committee; http://www.frac.info/home) list 12 classes based on the different biochemical pathways that the fungicides within a class targets, as well as a 13^{th} class which comprises fungicides with unknown modes of action. Fungicides are also distinguished by their modes of delivery and sites of action: some fungicides are sprayed onto the plants surfaces while others are applied to the soil surfaces either in granular form or as a liquid flooding the soil surface. Fungicides applied to the soils tend to be absorbed via the roots and are transported to all plant tissues via xylem. Fungicides that are foliar can be either local-*i*.*e*., protecting only the surfaces that they contact, systemic-*i*.*e*., absorbed by the upper plant surfaces but then transported by xylem to all above-ground tissues, or partially systemic-*i*.*e*., they can be locally absorbed but can only be transported short distances to protect a somewhat more extensive surface than the initial point of fungicide contact. Also, as with the case of herbicides, a registration system for fungicides exists which restricts the use of particular fungicides to specific crops and fungal disease applications where their application has been shown to be most efficacious and safe. Fungal diseases of *Brassica* oil seed crops can reduce the yield and the quality of harvested grain. Depending on the nature and severity of the fungal pathogen infection, the impacts can range from minor to complete crop loss. Fungicides can help mitigate the risk of losses incurred by fungal infection, but the costs of fungicide spraying are significant enough to require cost benefit and risk assessment type analyses to be carried out before deciding to proceed. Examples of economically significant fungal diseases of *Brassicas* and mustard oilseeds include
   i. ***Sclerotinia* stem rot** is caused by a fungus whose spores infect *Brassica* primarily during flowering stages and whose incidence is associated with periods of high humidity. Lesions are formed on the stems which can eventually kill the plant. Fungicides are available which can control the severity of the infection but must be applied at specific periods of the plant lifecycle *(i.e.,* at early to mid-flowering) for best effect. Often multiple applications within this window of time are necessary.
   ii. **Alternaria** is a fungal disease of *Brassicas* that affects plants at all growth stages from early seedling through to maturity although mature plants are more susceptible. The greatest economic impact is on grain yield and quality. Foliar fungicide application during the late flowering stage is an effective way to mitigate the more detrimental effects of the disease on grain yield and quality.
   iii. **Blackleg,** a fungal disease of *Brassica* oilseed crops, infects plants at all stages but early stage infections have the most serious consequences, often culminating in plants with necrotic lesions on their lower stems that can virtually sever the plants at the base. Fungicides are only partially effective, having a minor protective effect when applied at an early plant growth stage.
   iv. **Clubroot** is a soil-borne fungus that affects the roots of *Brassica* oilseed crops. The spores can persist for long periods in the soil and there is currently no effective fungicidal treatment. Management may require using rotations which limit the frequency of *Brassica* planting.
i. **Insecticides** are a third group of pesticide compounds designed to reduce or eliminate crop loss due the predation of crop species by insects. Like herbicides and fungicides, insecticides are classified according to their mode of action and the biochemical pathways that they target. One classification scheme (IRAC MoA) advocated by the Insecticide Resistance Action Committee (IRAC; http://www.irac-online.org) lists 29 classes of insecticides grouped by the common biochemical processes and pathways that the insecticide compounds target. Like herbicides and fungicides, insecticide function and persistence can also be influenced by their sites of action, *i.e.,* whether they are only active on the surface of plants as applied, or whether they function as systemic agents. Further differentiation among some insecticide groups may be apparent based on whether they exhibit selectivity for specific insect types due to distinctive aspects of that insect's biology, Given that some insects serve a beneficial role, such as controlling plant pests, serving as plant pollinators and improving the nutrient content of soil, it is important that insecticides not be applied indiscriminately, but rather are used in a way that limits their actions as much as possible to the desired target species. Thus, modalities such as timing of application, amount and route of application, and restrictions on the types of insecticides used and the crops they may be used are all incorporated into the registered usage criteria of insecticide as a means of ensuring their safety and efficacy. Listed below are examples of insect species that can have significant negative economic impact on the yields and grain quality of *Brassicas* and mustard oilseed crops:
j. **Flea beetle** is a very common pest of *Brassica* oilseed and mustard crops, feeding on both leaves and stems. When the flea beetle is present at early stages of crop development, it can result in severe thinning of the crop stand which will ultimately have a significant impact on the grain yield. There are insecticide products that can be applied as a foliar spray that are effective in controlling early stage infestations.
k. The adult **Diamondback Moth** will lay her eggs on the surface of *Brassica* oilseed and mustard leaves and on hatching, the resultant larva will attack leaves and stems of the crops. Larva from eggs hatched at later stages will also feed on seed pods. The most impactful infestations are those which begin at early plant stages since the lifecycle of the moth can allow up to for generations during a typical *Brassica* oilseed crop season, resulting in ever increasing cycles of damage to the crop. In such cases insecticide treatment is most effective when applied at the early stages of the season when the larva are first observed.
l. Both the adult and the larval **Cabbage Seedpod Weevil** can inflict significant damage on *Brassica* oilseed crops, mostly by compromising flowering and seed pod development. The adults will feed on flower buds, severely weakening them and rendering them more susceptible to heat induced damage and loss. Adults will lay their eggs in seed pods and the larva will fed on the developing seed. When the larvae mature to adulthood, they will emerge from the pod and continue feeding on the seeds through the pod wall. Insecticide application at the early flowering stage is necessary control the primary infestation to break the progression to secondary infestation.
m. **Seed treatments:** Often, specific fungicides and insecticides can be formulated with chemical agents and binders to form a composition that can be applied to the surface of a seed. This seed treatment forms a stable coating over the seed surface. The resultant treated seed can then be packaged and sold to the farmer. When the seed is subsequently planted, the fungicide and insecticide will be present at the optimal dose to allow the emerging plant to withstand early season insect and fungal infestations, when the plant is most vulnerable and allowing the plantlet to form a better more vigorous stand. The benefits include improved crop yield potential and reduced requirement for early season spraying.

**Emission intensity** is average emission rate of a given pollutant from a given source relative to the intensity of a specific activity. As a specific example carbon intensity is the amount of carbon (often expressed in terms of grams of CO₂) released during the production of energy (expressed in mega joules).

**Fallow** refers to the agricultural practice of leaving agricultural land barren of crops or vegetation for one or more growing seasons following a period when the land has been intensively farmed. The goal of a fallow is to improve the likelihood of a better yielding crop in the subsequent season. A fallow season affords the farmer the opportunity to address weed issues in the field. Weeds may be allowed to grow in the fallow field and then be eradicated either by physical means or by herbicide treatment. This can be repeated several times during the course of a single fallow season and thus depletion of the weed seed bank can be achieved more effectively than by use of pre-seed, in-crop and postharvest measures. Fallow periods can also benefit the soil by allowing it to accumulate moisture to replace that which was depleted by the previous crop. During fallow, organic material from plant residues and roots of previous crops can be broken down more thoroughly, further benefitting the soil by improving its structure and nutrient content. Fallow periods also allow the soil microbiome and other soil organisms to replenish their numbers, while the break from crop production may also allow for depletion of specific plant pathogens that rely on crop species as a host. While it is understood by those in the art that fallow periods built into a crop rotation may have considerable benefit to the health and yields of subsequent and future crops, it is also understood that in the immediate term, the fallow field does not offer the farmer an opportunity for economic return from cash crops. By contrast, cultivation of *Brassica carinata* as a cover crop in place of fallow can provide many of the soil and rotational benefits described above but with the additional benefits of providing the farmer with the possibility of significant economic return from the harvest of the valuable oilseed grain. Cultivation of *Brassica carinata* as a cover crop in place of fallow also benefits the environment by providing a feedstock for low carbon intensity biofuel production as well as by affording the opportunity to offset lifecycle GHG emissions by the capture and transfer of considerable amounts of carbon to the soil.

**Feedstock,** as utilized herein, refers to oils derived from crushing of the oilseed and subjected to a cursory purification to render them as suitable and sufficient primary raw material for the production of biofuels through specified methods.

**Fertilizers (inorganic fertilizers, chemical fertilizers, mineral fertilizers)** are manufactured nutrients added to soil by the farmer/producer to supplement existing soil-based nutrients with the goal of optimizing the growth, yield and performance of cultivated plants and crops. Nitrogen (N), phosphorus (P) and potassium (K) are the major elemental macronutrient constituents of fertilizers. The chemical constituents commonly used as chemical fertilizers include, but are not limited to:
- Nitrogen fertilizers: ammonia (NH₃), sodium nitrate (NaNO₃), ammonium nitrate (NH₄NO₃), calcium ammonium nitrate (Ca(NO₃)₂NH₄NO₃), monoammonium phosphate or MAP (NH₄H₂PO₄), diammonium phosphate or DAP ((NH₄)₂HPO₄), and urea (CO(NH₂)₂).
- Phosphorus fertilizers: phosphorus pentoxide (P₂O₅), superphosphate or OSP (monocalcium phosphate or Ca(H₂PO₄)₂), rock phosphate, MAP and DAP
- Potassium fertilizers: potash, potassium oxide (K₂O), potassium chloride (KCl), potassium nitrate (KNO₃), potassium sulfate (K₂SO₄), mono-potassium phosphate (KH₂PO₄), and di- potassium phosphate (K₂HPO₄).
While the chemical forms of N, P and K in fertilizers may vary, in order to allow comparisons between fertilizers containing different forms of the three elements, the proportions are standardized as follows: nitrogen content is expressed in terms of elemental nitrogen, phosphorous content is expressed in terms of P₂O₅ equivalent, and potassium content is expressed in terms of K₂O equivalent. Conversion factors allow for the weights of different mineral forms to be converted to the appropriate standard weight. Testing of soil for preexisting nutrient content prior to seeding is the most reliable way to determine optimal levels of fertilizer application. Overapplication of fertilizer at levels beyond what is required based on crop requirements and soil nutrient content is not advisable for several reasons. Often, the additional expense incurred by application of excess fertilizer does not translate to increased crop yield to cover the expense. As well, excess nutrients can have deleterious effects on crop growth. For example, excessive nitrogen after bolting of *Brassica* oilseed crops can result in overgrowth of foliage at the expense of flowering and seed set. Moreover, excess nitrogen-based fertilizer can be released from soil either as material leached in the water table and subsequently into water courses or through direct volatilization. This leached- and/or volatilized material can be converted through an indirect process to N₂O (see below) and thus contribute to GHG emissions. Excess nitrogen and phosphate fertilizers leached from the soil into the water table can enter bodies of fresh water (lakes and rivers) and reach levels sufficient or cause eutrophication and deoxygenation, resulting in damage to the aquatic environment.

**Grain,** in reference to *Brassica carinata,* refers to the seed harvested at maturity and sold as a source of oil and meal products.

**Greenhouse gases (GHG)** are the subset of gaseous byproducts emitted through anthropogenic sources, such as from combustion of hydrocarbon fuels or by release of volatile components of hydrocarbon-containing products, that act to increase global warming by contributing to atmospheric entrapment of radiant solar energy. The major greenhouse gases are CO₂ (carbon dioxide), CH₄ (methane), N₂O (nitrous oxide) and CFC (chloroflourocarbons). Emissions of CFC's, a class of compounds used in aerosol propellants and refrigerants, are generally a result of their direct release. N₂O emission can occur via combustion of hydrocarbon fuels as well as by release from applied fertilizer. The most important GHG are CO₂, CH₄ and N₂O. GHGs are ranked in terms of their global warming potential (GWP), or ability to stimulate global warming on a concentration basis. If compared in terms of their relative contribution to global warming (global warming potential) on a per weight basis, N₂O is 265 times more potent than CO₂ and CH₄ is 28 times more potent than CO₂ (Values taken from the IPCC Fifth Assessment Report: IPCC, 2014: Climate Change 2014: Synthesis Report. Contribution of Working Groups I, II and III to the Fifth Assessment Report of the Intergovernmental Panel on Climate Change [Core Writing Team, R.K. Pachauri and L.A. Meyer (eds.)]. IPCC, Geneva, Switzerland, 151 pp). Based on the aforementioned relative potencies, GHG emission is often expressed in terms of CO₂ equivalent emission (combining and normalizing the effects of the three GHGs released during energy production relative to CO₂). GHG emissions and their effects on global warming are usually linked to combustion of fossil fuels but GHG can also be released by burning of biomass-based fuels. In the latter case, the GHG emission is offset by the CO₂ that is assimilated by plants and crops via photosynthesis.

**Harvest or harvesting,** as used herein, refers to the act of collecting the portion of a plant that has matured sufficiently over the course of a growing season and that has value as a source of food, feed, fibre, feedstock, structural material or as a propagule for the plant itself. Carinata is harvested, for example, by mechanical harvesting, ideally when seed maturity is reached (seed, pods and stalks turn from green to yellow, seed moisture is 9.5% or less). Carinata can be combine harvested by straight cutting or, if need be can be swathed at an early stage, allowed to dry naturally or with the aid of a desiccant, then the dried swath can be combined. Canola, which has a higher tendency to lean over ("Lodge") when mature, is often "swathed" prior to combining. Swathing mean cutting the canola near the base of the plant and allowing the plant to lie flat in field for several days to allow the grain to reach the appropriate dryness. Once dry, the swath is then harvested by combining. Another variation is called "pushing", which is similar to swathing except the plant is physically pushed over on its side and allowed to dry for several days prior to it being combine harvesting. For all of these variations in harvesting, the common final stage is combine harvesting. However, since carinata has a much sturdier stalk than canola, the preferred method for harvest of carinata is direct combining at maturity, rather than swathing or pushing followed by combining. Direct combining allows harvest by a single pass through the rows in the field. A single-pass harvest produces less CO₂ than a harvest where swathing or pushing is carried out in addition to the combining, because of the reduction in fuel use.

**Harvest Index** (Hay, 1995) refers to a measure of the ratio between the weight of grain harvested from a plant at maturity to that of the remaining above-ground plant material which can include stems and branches, remaining associated leaves and the empty seed pods (chaff).

**Land Use Change (LUC):** The term Land Use Change, used in an environmental sciences context, refers to changes in the use of land that results in significant changes in stored carbon and concomitant changes in atmospheric CO₂ levels and those of other GHG. Examples where LUC results in increased CO₂ and GHG emissions include clearing of forest to increase arable land available for agricultural production and clearing of grasslands to increase arable land for agricultural production. Examples of LUC leading to reductions in atmospheric CO₂ and GHG levels include allowing previously cultivated land to return to their natural state.

**Land management practices:** For the purposes of this application, the term "land management practices" refers, for a given land use, to those practices or changes in those practices that affect soil carbon, nutrient and water levels and may also alter the levels of atmospheric CO₂ and other GHG. These may include: types of tillage practice and treatment of crop residues, types and amount of fertilizer (or other inputs) used and use of specific crop rotations or fallow seasons. Land management and land input types are found in Table 3 of EC document entitled: COMMISSION DECISION of 10 June 2010 on guidelines for the calculation of land carbon stocks for the purpose of Annex V to Directive 2009/28/EC, including
i. **full-tillage:** substantial soil disturbance with full inversion and/or frequent (within year) tillage operations. At planting time, little (e.g. < 30 %) of the surface is covered by residues;
ii. **reduced tillage:** primary and/or secondary tillage but with reduced soil disturbance (usually shallow and without full soil inversion) and normally leaves surface with > 30 % coverage by residues at planting.;
iii. **no till (or no tillage):** Direct seeding without primary tillage, with only minimal soil disturbance in the seeding zone. Herbicides are typically used for weed control;
iv. **low till (or low tillage):** Low residue return occurs when there is due to removal of residues (via collection or burning), frequent bare-fallowing, production of crops yielding low residues (e.g. vegetables, tobacco, cotton), no mineral fertilization or nitrogen-fixing crop;
v. medium till (or medium tillage): Representative for annual cropping with cereals where all crop residues are returned to the field. If residues are removed, then supplemental organic matter (e.g. manure) is added. Also requires mineral fertilization or nitrogen-fixing crop in rotation;
vi. high with manure: Represents significantly higher carbon input over medium carbon input cropping systems due to an additional practice of regular addition of animal manure; and
vii. high without manure: Represents significantly greater crop residue inputs over medium carbon input cropping systems due to additional practices, such as production of high residue yielding crops, use of green manures, cover crops, improved vegetated fallows, irrigation, frequent use of perennial grasses in annual crop rotations, but without manure applied (see row above).

Legumes (or leguminous crops) are plants of the family Fabaceae (or Leguminosae), which are grown primarily for their grain; dried forms are referred to as pulses. Legumes are also grown as forage. An important characteristic of legume crops are their roots which have uniquely evolved structures known as root nodules that can be colonized by symbiotic, nitrogen fixing rhizobacteria. These symbiotic bacteria confer on legumes the ability to fix atmospheric nitrogen as ammonia, which subsequently is used by the plant in biosynthesis of amino acids and proteins. When the plant dies, the nitrogen stored as protein is returned to the soil and ultimately converted to NO₃, which is then made available to other plants.

Lifecycle assessment (LCA) is "a systematic set of procedures for compiling and examining the inputs and outputs of materials and energy and the associated environmental impacts directly attributable to the functioning of a product or service system throughout its life cycle" (ISO 14040.2 Draft: Life Cycle Assessment - Principles and Guidelines). "LCA is a technique for assessing the potential environmental aspects and potential aspects associated with a product (or service), by compiling an inventory of relevant inputs and outputs, evaluating the potential environmental impacts associated with those inputs and outputs and interpreting the results of the inventory and impact phases in relation to the objectives of the study" (ISO 14040.2 Draft: Life Cycle Assessment - Principles and Guidelines). Aspects of LCA include:
- **Functional unit,** which defines and delimits what is being studied, the process steps involved, specifies the appropriate inputs and outputs and provides a basis for comparisons between alternative fuels, fuel manufacturing processes or feedstocks.
- **System boundaries,** which define which process(es) should be included in the analysis of a particular system: for transportation fuel, the system boundary most often used is termed well to wheel and comprises all steps from the extraction of the raw material, its processing, transportation, storage, distribution network and final combustion in the vehicle engine. A variant of the well to wheel system is "well to tank" which comprises all steps of the well to wheel system except for the combustion of the fuel in the vehicle engine.
- **Allocation methods,** which are used to partition the emissions of a process when one or several co- products, along with the main product are produced during a process. A particular example of this is the allocation that occurs in a well-to-tank analysis of biofuels developed from oilseeds up to the point that the oil is extracted from the oilseed. In the BioGrace model employed herein, account is taken of the fact that crushing of the oilseed produces not only oil (the biofuel feedstock) but also a protein rich meal byproduct. Prior to the processing of the oilseed into separate meal and oil fractions, an allocation factor is applied to all process emissions which reflects the proportion of energy associated with the oil portion of the oilseed based on the oil's LHV (lower heating value). After separation of oil and meal fractions all energy usage and emissions are allocated to the oil while the meal contributes no further.

**Low Carbon Intensity biofuel, or low CI biofuel,** as used herein, is a biofuel whose production results in lower carbon intensity than that of a corresponding petroleum-based fuel according to one or more renewable fuel regulations. For example, using the values set forth in Table 1 of DeJong et al, a low CI biofuel will have a CI value less than 83.8 g CO_{2 eq}/MJ in accordance with EU-RED and a low CI biodiesel will have a CI value less than 91.8 g CO_{2 eq} /MJ in accordance with US RFS.

**Low GHG biofuel,** as used herein, is a biofuel whose production results in lower GHG emissions than the production of a corresponding petroleum-based fuel, as determined using one or more LCA models, according to one or more renewable fuel regulations.

**Macronutrients,** as used herein, refer to nitrogen (N), phosphorus (P), potassium (K) and sulfur (S). Nitrogen as a major constituent of amino acids, proteins and chlorophyll, contributes primarily to growth of leaves and foliage. Phosphorous, found in DNA/RNA polymers, nucleoside precursors and coenzymes, membrane phospholipids, etc. is required for root and flower formation as well as seed and fruit development and maturation. Potassium is an important regulator of water movement, turgor, flowering and fruiting.

**Manure,** as used herein, refers to organic matter, mostly derived from animal feces, which can be used as organic fertilizer in agriculture. Manures contribute to the fertility of the soil by adding organic matter and nutrients, such as nitrogen, that are utilized by bacteria, fungi and other organisms in the soil. Most animal manure consists of feces. A common form of animal manure is farmyard manure (FYM) which may also contain plant material (often straw), which has been used as bedding for animals and has absorbed the feces and urine. Manure from different animals has different qualities and requires different application rates when used as fertilizer. For instance, sheep manure is high in nitrogen and potash, cattle manure is a good source of nitrogen as well as organic carbon. Chicken litter is concentrated in both nitrogen and phosphate.

**Maturity** is defined as the stage where pod seed fill has been completed, the pods and the seeds have lost all green coloration and seed moisture is less than 9%. At this point, most, if not all, of the leaves have been lost, the stalks and stems have all turned to yellow, and the plant is considered to be dead.

**Micronutrients:** In addition to the major macronutrients (N, P, and K), lesser amounts of secondary macronutrients, including calcium (Ca), magnesium (Mg) and sulfur (S) and trace amounts of micronutrients (such as boron, copper, iron, manganese, zinc) may also contribute to optimal plant growth and yield.

**N₂O emissions:** Managed agricultural soils can release nitrous oxide (N₂O), a potent greenhouse gas 265 times more active than CO₂. The nitrous oxide can be released directly or indirectly. Direct nitrous oxide emissions can ensue through Nitrification and denitrification of soil nitrogen by microorganisms. The soil nitrogen can originate from application of synthetic N fertilizers (urea, ammonia or nitrate based), application of organic fertilizer (mulches, manure), natural animal or poultry wastes (dung/urine), decomposition of plant/crop residues, ongoing mineralization/ demineralization of soil organic matter. Indirect nitrous oxide emissions result from a multistep process. The first step involves emission of ammonia or nitrate/nitrite (NOx) based gases into the atmosphere. These emissions can arise from a number of sources: direct volatilization of nitrogen containing compounds found in synthetic fertilizers, organic fertilizers or animal wastes; burning of plant wastes /crop debris; and burning of fuel for farm machinery. The second step involves the deposition of these atmospheric nitrogen compounds (ammonium, NOx) via rainfall, for example, onto surfaces of soil or bodies of water and the final step involves the subsequent conversion via denitrification! nitrification to N₂O and emission into the atmosphere. A secondary source of ammonium and NOx for indirect emission of nitrous oxide involves the leaching of nitrogen-based fertilizers, organic fertilizers or livestock wastes from the soil into the water table and then to bodies of water, whereupon they can be converted via denitrification/nitrification into N₂O. Formulas for calculation of direct and indirect N₂O emissions from agricultural lands, based in the mass amounts of synthetic fertilizer, organic fertilizer, above and below-ground biomass at harvest, on farm-fuel use as well as degree of soil saturation during the growing season are provided by the BioGrace model (and are based on those described in IPCC 2006, 2006 IPCC Guidelines for National Greenhouse Gas Inventories, Prepared by the National Greenhouse Gas Inventories Programme, Eggleston H.S., Buendia L., Miwa K., Ngara T. and Tanabe K. (eds). Published: IGES, Japan.)

**Seed pods** are the specialized structures that contain the seed during its development and maturation into grain. Seed pods serve as the seeds protection from the external environment and provide the energy and nutrients for seed development. When the seed reaches full maturity, the seed pod becomes dry and brittle and having lost all its chlorophyll assumes a yellow coloration. At this time as well the seed pod becomes susceptible to dehiscence, the physical opening of the structure, allowing release of the mature seed (Grain).

**Sequential crop production** is the practice of growing two or more crops sequentially on the same piece of land in a farming year and enables farmers to extend use of the land into a season, *i.e.,* winter where crops are not normally cultivated. This allows the farmer to earn additional income. Sequential crop production does not incur land-use change, since the land is already cleared and used for agricultural production. Moreover, use of a cover crop such as *Brassica carinata* as a sequential crop allows the farmer to accrue the soil benefits of a fallow period and earn income on the sales of the carinata grain.

**Soil** consists of minerals, organic matter, gases, liquids, and various animal and plant organisms. Soil is generated by the interaction, over time, of climate, geological, hydrological and atmospheric forces on the minerals that compose the earth's crust. Given sufficient time, soil will develop layers, or horizons that differ in structure and composition determined by the relative proportions of sand, silt, and clay.

**Soil Carbon:** Soil contains organic and inorganic (mineral) forms of carbon. The organic carbon fraction can consist of dead and decaying matter or comprise live plant, insect fungal or microbial matter. A standard **Soil organic carbon stock (SOC_{ST}) value** can be estimated based on the known carbon content of region's reference soil type modified by the effect of the regions climate (based on the climate classification scheme described earlier). Table 3 below, adapted from Table 1 of EC document entitled: COMMISSION DECISION of 10 June 2010 on guidelines for the calculation of land carbon stocks for the purpose of Annex V to Directive 2009/28/EC, summarizes estimated SOC_{ST} values (tonnes of carbon per hectare in the 0 to 0.3 M soil depth layer) for topsoil classes in a given climatic region.

**Table 3: Topsoil SOC_{ST} values for mineral soil types in specific climate zones**

| **Climate type** | **Soil Organic Carbon content of different soil types (T/ha)** | | | |
|---|---|---|---|---|
| | **High activity clay soil** | **Low activity clay soil** | **Sandy soils** | **Spodic soils** |
| Cold temperate dry | 50 | 33 | 14 | - |
| Cold temperate moist | 95 | 85 | 71 | 115 |
| Warm temperate dry | 38 | 24 | 19 | - |
| warm temperate moist | 88 | 63 | 34 | - |
| Tropical dry | 38 | 35 | 31 | - |
| Tropical moist | 65 | 47 | 39 | - |

A **Soil Organic Carbon (SOC)** value, taking into account factors such as land use, land management, and agricultural inputs can then be calculated for the land under cultivation (SOC = SOC_{ST} × F_{LU} × F_{MG} × Fi, where SOC = soil organic carbon measured as mass of carbon per hectare; SOC_{ST} = standard soil organic carbon in the 0-30 centimeter topsoil layer measured as mass of carbon per hectare and determined as described above; F_{LU} = land use factor reflecting the difference in soil organic carbon associated with the type of land use compared to the standard soil organic carbon; F_{MG} = management factor reflecting the difference in soil organic carbon associated with the principle management practice compared to the standard soil organic carbon; Fi = input factor reflecting the difference in soil organic carbon associated with different levels of carbon input to soil compared to the standard soil organic carbon. Table 4, adapted from Table 2 of EC document entitled: COMMISSION DECISION of 10 June 2010 on guidelines for the calculation of land carbon stocks for the purpose of Annex V to Directive 2009/28/EC, provides values for F_{LU}, F_{MG}, Fi for crops cultivated in different climate zones under specified land uses, land management practices and input usage levels.

**Table 4: Values for F _{LU}, F _{MG} and F _{I}**

| **Climate zone** | **Land use** | **Land management** | **Land Inputs** | **F_{LU}** | **F_{MG}** | **F_{I}** |
|---|---|---|---|---|---|---|
| Cool/ Warm Temperate dry | cultivated | Full tillage | Low | 0.80 | 1.00 | 0.95 |
| | | | Medium | 0.80 | 1.00 | 1.00 |
| | | | High w/ manure | 0.80 | 1.00 | 1.37 |
| | | | High w/o manure | 0.80 | 1.00 | 1.04 |
| | | Medium tillage | Low | 0.80 | 1.02 | 0.95 |
| | | | Medium | 0.80 | 1.02 | 1.00 |
| | | | High w/ manure | 0.80 | 1.02 | 1.37 |
| | | | High w/o manure | 0.80 | 1.02 | 1.04 |
| | | No tillage | Low | 0.80 | 1.10 | 0.95 |
| | | | Medium | 0.80 | 1.10 | 1.00 |
| | | | High w/ manure | 0.80 | 1.10 | 1.37 |
| | | | High w/o manure | 0.80 | 1.10 | 1.04 |
| Cool/Warm Temperate moist | cultivated | Full tillage | Low | 0.69 | 1.00 | 0.92 |
| | | | Medium | 0.69 | 1.00 | 1.00 |
| | | | High w/ manure | 0.69 | 1.00 | 1.44 |
| | | | High w/o manure | 0.69 | 1.00 | 1.11 |
| | | Medium tillage | Low | 0.69 | 1.02 | 0.92 |
| | | | Medium | 0.69 | 1.02 | 1.00 |
| | | | High w/ manure | 0.69 | 1.02 | 1.44 |
| | | | High w/o manure | 0.69 | 1.02 | 1.11 |
| | | No tillage | Low | 0.69 | 1.10 | 0.92 |
| | | | Medium | 0.69 | 1.10 | 1.00 |
| | | | High w/ manure | 0.69 | 1.10 | 1.44 |
| | | | High w/o manure | 0.69 | 1.10 | 1.11 |
| Tropical dry | cultivated | Full tillage | Low | 0.58 | 1.00 | 0.95 |
| | | | Medium | 0.58 | 1.00 | 1.00 |
| | | | High w/ manure | 0.58 | 1.00 | 1.37 |
| | | | High w/o manure | 0.58 | 1.00 | 1.04 |
| | | Medium tillage | Low | 0.58 | 1.02 | 0.95 |
| | | | Medium | 0.58 | 1.02 | 1.00 |
| | | | High w/ manure | 0.58 | 1.02 | 1.37 |
| | | | High w/o manure | 0.58 | 1.02 | 1.04 |
| | | No tillage | Low | 0.58 | 1.10 | 0.95 |
| | | | Medium | 0.58 | 1.10 | 1.00 |
| | | | High w/ manure | 0.58 | 1.10 | 1.37 |
| | | | High w/o manure | 0.58 | 1.10 | 1.04 |
| Tropical moist | cultivated | Full tillage | Low | 0.48 | 1.00 | 0.92 |
| | | | Medium | 0.48 | 1.00 | 1.00 |
| | | | High w/ manure | 0.48 | 1.00 | 1.44 |
| | | | High w/o manure | 0.48 | 1.00 | 1.11 |
| | | Medium tillage | Low | 0.48 | 1.02 | 0.92 |
| | | | Medium | 0.48 | 1.02 | 1.00 |
| | | | High w/ manure | 0.48 | 1.02 | 1.44 |
| | | | High w/o manure | 0.48 | 1.02 | 1.11 |
| | | No tillage | Low | 0.48 | 1.10 | 0.92 |
| | | | Medium | 0.48 | 1.10 | 1.00 |
| | | | High w/ manure | 0.48 | 1.10 | 1.44 |
| | | | High w/o manure | 0.48 | 1.10 | 1.11 |

If the cultivation methods, land management practices, or inputs associated with a particular agricultural land base have changed, and it is desired to know the consequence of the change(s) on the carbon stocks, then one could similarly calculate SOC values for the **reference scenario (SOC_{R})** as well as for the **actual Scenario (SOC_{A})** and use the values to calculate **Soil Carbon Accumulation (E_{sca})** using the formula E_{sca} = -(SOC_{R} - SOC_{A})*3.664 / 20 years, or the tonnes of carbon (as CO₂)/ year over a 20 year period. If the E_{sca} value is negative, it represents a loss of soil carbon whereas if positive it represents a net accumulation of soil carbon.

**Soil Classification:** Soils are classified based on "The *World Reference Base for Soil Resources* (WRB)" which proposed 30 *'Soil Reference Groups'.* These 30 reference soil types are distributed among 10 'sets' as described below.
- Set #1 includes all organic soil. Organic soils (Histosols) are those that are unusually rich in organic matter in various stages of decomposition, and where the decomposition rate has been hindered by prolonged exposure to low temperatures and/or wet conditions. The remaining mineral soil groups are each allocated to one of nine sets based on their most particular identifying factors that are key for their formation and differentiation.
- SET #2 contains all types of mineral soils that are particularly conditioned by human influences. This set consists of one reference soil group: the ANTHROSOLS.
- SET #3 includes mineral soils whose formation is conditioned by the properties of their parent material. The set includes three reference soil groups: ANDOSOLS, which are of volcanic origin and regions; ARENOSOLS, which comprise the sandy soil of desert areas, beaches, inland sand dunes, *etc.*; and VERTISOLS, heavy clay soils found in swampy marsh regions, river banks and basins.
- SET #4 comprises mineral soils whose formation and characteristics are influenced by their topographic/physiographic or hydrological setting. This set comprises four reference soil groups:
   ∘ The stratified FLUVISOLS found in lowlands and wetland areas;
   ∘ The non-stratified GLEYSOLS typically found in waterlogged areas;
   ∘ LEPTOSOLS, shallow soils found in elevated regions usually over rocky substrates; and
   ∘ REGOSOLS, deeper soils of elevated regions occurring over gravelly substrates.
- SET #5 comprises soils that are only moderately developed due to relatively young age and thus are representative of a quite diverse reference soil group: the CAMBISOLS.
- SET #6 comprises soils conditioned by climate of sub-humid tropical regions. The six reference soil groups in this set have in common that a long history of dissolution and transport of weathering products has produced deep and genetically mature soils:
   ∘ PLINTHOSOLS, composed of a mixture of clay and quartz (*'plinthite'*);
   ∘ FERRALSOLS, with very *low cation exchange capacity* and lacking any weatherable components;
   ∘ ALISOLS, aluminum rich with *high cation exchange capacity;*
   ∘ NITISOLS, deep and red hued soil, high in iron;
   ∘ ACRISOLS, low fertility soils with high clay content, and containing high concentrations of aluminum; and
   ∘ LIXISOLS, soils with low fertility, *low cation exchange capacity* but *high base saturation.*
- SET # 7 comprises soils conditioned by climate in arid and semi-arid regions. The five reference soil groups assembled in set #7 are:
   ∘ SOLONCHAKS, with a high content of soluble salts,
   ∘ SOLONETZ, with a high percentage of adsorbed sodium ions,
   ∘ GYPSISOLS, with a horizon of secondary gypsum enrichment,
   ∘ DURISOLS, with a layer or nodules of soil material that is cemented by silica, and
   ∘ CALCISOLS, with secondary carbonate enrichment.
- SET #8 comprises soils that occur in the steppe zone between the dry climates and the humid Temperate Zone and includes three Reference Soil Groups:
   ∘ CHERNOZEMS, with deep, very dark surface soils and carbonate enrichment in the subsoil,
   ∘ KASTANOZEMS with less deep, brownish surface soils and carbonate and/or gypsum accumulation at some depth (these soils occur in the driest parts of the steppe zone), and
   ∘ PHAEOZEMS, the dusky red soils of prairie regions with high base saturation but no visible signs of secondary carbonate accumulation.
- SET #9 holds the brownish and greyish soils of humid temperate regions and contains five Reference Soil Groups:
   ∘ acid PODZOLS with a bleached eluviation horizon over an accumulation horizon of organic matter with aluminum and/or iron,
   ∘ PLANOSOLS with a bleached topsoil over dense, slowly permeable subsoil,
   ∘ base-poor ALBELUVISOLS with a bleached eluviation horizon tonguing into a clay-enriched subsurface horizon,
   ∘ base-rich LUVISOLS with a distinct clay accumulation horizon, and
   ∘ UMBRISOLS with a thick, dark, acid surface horizon that is rich in organic matter.
- SET #10 holds the soils of permafrost regions and are included in one Reference Soil Group: the CRYOSOLS.

In the EU commission decision of June 10^{th}, 2010, "on guidelines for the calculation of land carbon stocks for the purpose of Annex V to Directive 2009/28/EC" (http://eur-lex.europa.eu/legalcontent/EN/TXT/?uri=uriserv %3AOJ.L_.2010.151.01.0019.01.ENG) the 30 reference soil groups were further grouped among 6 major categories of soil types, including sandy soils (arenosols), wetland soils (gleysols), volcanic soils (andosols), spodic soils (podzol), low activity clay soils (with low cation exchange capacity or CEC) and high activity clay soils (with high CEC).

**Straw** is defined as all above-ground plant parts (excluding the grain) that are collected by the harvesting and combining process and then having been separated from the grain, are then deposited back onto the field.

**Stubble** is defined as the post-harvest residue left in the field that comprises the material below the point of cutting by the combine harvester, and that which is not collected for subsequent grain threshing and winnowing operations. In low till or no till soil management paradigms, most or all of the stubble remains on the field when seeding of the following crop is carried out. This necessitates specialized seeding equipment, which can open an unimpeded path to the soil allowing good seed to soil contact in the midst of the stubble. This is particularly important in situations where the stubble might be especially dense, as in stubble from a previous corn crop, for example.

**Variety** refers to the botanical taxonomic designation whereby variety is ranked below species or subspecies, as well as the legal definition whereby the term "variety" refers to a commercial plant cultivar that is protected under the terms outlined in the International Convention for the Protection of New Varieties of Plants, an international treaty administered by UPOV. The term "variety" (under UPOV) describes a new, physically distinctive, uniform and stable plant cultivar developed by a plant breeder. The latter definition accords certain rights of protection and ownership among the nations that are signatory to the treaty, to the plant breeder, of the legal "variety" as long as the four aforementioned criteria are fulfilled.

### Detailed Description

Carbon emissions due to agronomic practices and land use change can contribute to the overall Carbon Intensity of a biofuel pathway. The present invention recites the use of *Brassica carinata* as a dedicated biofuel feedstock crop as well as reciting associated climate zones and crop rotations used in cultivation as well as associated agronomic practices to reduce carbon emissions during its elevation as much as possible, even to the extent that net carbon flux during cultivation and harvest favors a net decrease in atmospheric CO₂ levels *(i.e.,* a negative carbon intensity).

The present invention describes the production of *Brassica carinata,* a crop whose cultivation has not been previously demonstrated to provide utility for production feedstock which can be used to produced low carbon intensity biofuels. *Brassica carinata,* due to its unique growing habit and its ability to resist frost, drought and heat, offers these advantages that were previously unknown. In this invention, and the examples and description herein, the practical utility of *Brassica carinata* as a rotation option in a number of cropping scenarios in a multitude of production practices has been demonstrated as working examples. Examples are provided whereby under optimal conditions, a net negative overall carbon intensity for production of feedstock oil and meal can be achieved that can offset carbon intensity incurred during biofuel manufacturing and distribution and results in return of a substantial amount of carbon per hectare per year to soil.

*Brassica carinata* has a unique growth habit amongst the oilseed Brassicaceae, producing a mature plant that is more highly branched than other *Brassica* oil seed species (Gesch *et al.,* 2015). Comparing biomass between current commercial *Brassica napus* and *Brassica carinata* varieties, above-ground biomass accumulation was found to be 1.8 to 2-fold higher per unit area for *Brassica carinata* varieties relative to the advanced commercial *Brassica napus* varieties. In the case of *Brassica carinata,* yields of grain approach those of the most advanced canola type *Brassica napus* varieties while producing almost double the amount of above-ground biomass (Gesch *et al.,* 2015).

*Brassica carinata's* deep and extensive tap root system can extend as far as 60-90 cm below the soil surface with more than 50% of the root mass in the top 30 cm (for example see Seepaul *et al.,* 2016). The taproots can penetrate through compacted soil layers, improving soil structure in the process. They can take up minerals and nutrients that might normally leech into the water table and make those nutrients available to subsequent crops that follow in the rotation. The roots also comprise a significant proportion of the plant's total biomass- as much as 20-25% of the plant's above-ground biomass as measured at maturity (Gan *et al.,* 2009a)-and account for an additional sink of carbon to be returned to the soil subsequent to harvest. The roots not only constitute a carbon sink, but also serve as a conduit whereby carbon-containing molecules can also be secreted into the environment at the root-soil interface. Carbon release by living root tissue, also known as rhizo-deposition, takes place during the growth and maturation of plants and encompasses three sources for deposition of carbon into the surrounding soil: carbon originating from sloughed root border cells, carbon originating from secreted mucilage, and carbon from small molecules that are "exuded" from root cells, the latter representing an important source of rhizo-deposited carbon (Nguyen, 2003). Estimates have been made of rhizo-deposited carbon by *Brassica napus* of almost 350 kg/ha in a single growing season (Gan *et al.,* 2009b).

The carbon content of *Brassica carinata* has been estimated at between 45% to 47% of the dry weight of its biomass (Gasol *et al.,* 2007, Duca *et al.,* 2015) and thus constitutes a significant sink of carbon accumulated both above-ground and below-ground during the growing season. At maturity, carinata grain is typically harvested by combining, which cuts and collects the above-ground plant material consisting of stems and branches where the seed pods are found. The seed pods are threshed, and the grain is collected while all the remaining material, including the now empty pods, stalks, branches and stems (collectively referred to as the plant straw) is returned to the field which, along with the remaining plant stubble, can now potentially contribute to soil carbon levels via breakdown of the residues by soil borne bacterial, fungus and mold.

If one examines the pathways for production of crop-based biofuels, the most scope for reducing the carbon intensity lies in the production of the feedstock and, in particular, at the crop production phase. Given that crops assimilate more CO₂ than they release over their life time it should be possible, with some modification to cultivation methods, to introduce a negative carbon intensity in this phase of the pathway, which would have the effect of decreasing the pathway's overall carbon intensity.

There can be considerable scope for mitigation of the CO₂ and GHG released during the crop's cultivation, harvest, storage, shipping and processing. For example, reduction of inputs, particularly inorganic nitrogen fertilizer, can have a significant effect on carbon-based emissions, both by reducing emissions associated with manufacturing of the fertilizer, and reducing soil nitrogen which, if present in excess of the crops needs, can be released into the atmosphere as nitrous oxide, a GHG that is 265 times more potent than CO₂. While nitrogen is an essential nutrient for most annual crops, its application can be fine-tuned according to the known requirements of the crop and determination of the pre-existing nitrogen levels in the soil. Moreover, leguminous annual crops, which are known to fix atmospheric nitrogen in the soil, can be used in rotations with other non-N fixing crops to reduce the latter's requirements for added N fertilizer.

Emission of GHG can also occur because of indirect land use change (ILUC). ILUC is a consequence of increased land requirement to accommodate cultivation of new energy and feedstock crops leading to displacement of food crop cultivation. To continue to meet demand for the displaced food crop, new land must be found to replace the land now being used for feedstock production. This may involve clearing of forest or grasslands, resulting in emission of a large amount of previously stable, sequestered CO₂ and other GHG in the process. Crops that can be grown successfully on underused, marginal cropland, as a cover crop or as a replacement for fallow in rotations will have a great advantage as energy or feedstock crops by reducing the potential for ILUC.

Crop rotation is an important means for reducing GHG emissions due to ILUC by increasing the efficiency of existing land use and reducing the requirement for new agricultural land. Crop rotations also take advantage of beneficial relationships between complementary crop species to improve crop yields and productivity. For example, a following crop that is of a different species than the preceding crop may prevent the long-term establishment or persistence of a disease specific and/or endemic to the preceding crop (*i.e.,* the following crop serves as a break crop). The following crop may also act as an alternative to a fallow cycle and provide the advantages of a cover crop--i.e., preventing soil erosion, helping to conserve moisture and allowing for the recycling of essential minerals and nutrients and improving the structure of the soil. Some crops such, as those of the legume species, may fix atmospheric nitrogen in the soil and reduce the requirements for exogenously added nitrogen fertilizer in the following crop(s).

Crops such as the *Brassica* species may exude compounds with anti-microbial properties *(i.e.,* glucosinolates) into the soil that may result in protection from plant pathogens for the following crop. Glucosinolates are a unique class of sulfur containing compounds synthesized by the Brassicaceae which, along with their catabolites, have potent anti-mycotic and anti-microbial activity. Synthesis of glucosinolates takes place in a number of plant compartments, including roots and release of glucosinolates and their catabolites in root exudates are believed to contribute to the ability of *Brassica* oilseed crops to provide an effective disease break when incorporated into crop rotations with cereals. Thus, yields of crops grown in rotation can often out yield crops grown as a monoculture and rotations that incorporate cover crops replacing winter fallow are more productive and sustainable that those relying on fallow. For example, in surveys of data relating to wheat cultivation in Australia, Europe and North America, Angus and co-workers (Angus *et al.,* 2011, Angus *et al.,* 2015) teach that wheat cultivation following that of *Brassica napus* or *Brassica juncea* consistently results in increased yield of the following wheat crop compared to wheat following wheat. It is understood by those skilled in the art that these are by example and are not meant to limit the scope of the invention.

In one aspect, there is provided a means to produce feedstock for the production of low carbon intensity biofuels. In particular, the invention describes methods for use of agricultural practices, including land management practices, to provide a feedstock for the production of low carbon intensity biofuels as consequence of the cultivation of a *Brassica carinata* oilseed crop in a rotation sequence. Said rotation sequence, which encompasses periods of time when usual cash crops are not grown, has been fortuitously discovered to provide significant advantages when the CI and GHG emissions associated with this cultivation are assessed under various established schemes for CI and GHG assessment.

For example, cultivation of *Brassica carinata* in the winter season in tropical and temperate climates has yielded the unexpected result of a good economic yield of *Brassica carinata* grain. Additionally, cultivation of *Brassica carinata* in dry land fallow further provides the unexpected result of a successful harvest of *Brassica carinata* grain that provides a feedstock that is suitable for the manufacturing of advanced low carbon intensity biofuels, such as hydrotreated vegetable oils (HVO), for the production of renewable diesel and jet fuels.

The present invention also provides agricultural methods, which include rotation strategies and land management practices to reduce fossil fuel inputs and maximize capture of atmospheric carbon during cultivation, to produce *Brassica carinata* seed for the production of feedstock that can be used for the production of low carbon intensity biofuels and other products. These production practices and rotation strategies have not been previously described, and the low carbon intensity and low GHG profile of the resultant harvest was not evident nor predictable.

The unique characteristics of *Brassica carinata* varieties described herein, combined with specific land management practices, seasonal timing of the rotation and the preceding crops of said rotation, allow for production of feedstock to produce low carbon intensity biofuels and other renewable products.

Use of *Brassica carinata* oilseed to produce a feedstock to produce low carbon intensity biofuels also provides a plant-based meal or protein source as a by-product following the extraction of the oil. It is noted that the same GHG savings associated with the oil component of the grain is also associated with the meal portion or the byproduct of the oil extraction. Accordingly, the present invention provides a novel low GHG protein-rich feed additive, a product that has utility in the production of livestock in a more environmentally conscious fashion. Hence the present invention describes a low GHG meal product for use as an animal feed additive.

In some embodiments of the invention, *Brassica carinata* is shown to be grown in tropical and warm temperature climates as a winter cover crop in rotation with summer crops such as beans, cotton, peanuts, or sesame, where the usual practice had been to follow with winter fallow (Seepaul *et al.,* 2015). This the first example of a *Brassica* oilseed providing a consistent yield in this geography when planted in early to late November, and is made possible by the unique ability of established carinata to survive and recover after hard frosts where other oilseed *Brassicas* such as canola could not adequately recover (Seepaul *et al.,* 2015). Oilseeds such as soybean are quite susceptible to frost kill (Hume and Jackson, 1981) and so would not be considered as a possible winter cover in this environment. Benefits of *Brassica carinata's* use as a winter cover crop in this environment include the ability to conserve winter moisture and nutrients in the soil, to mitigate leaching of nitrogen, phosphates and other residual nutrients into local waterways, and to provide a means to increase soil organic carbon (Newman *et al.,* 2010 (revised)). This introduces a new and viable winter oilseed cropping option to this region where none existed previously, offers benefits in terms of improved soil structure and additional moisture for establishment of crops planted following carinata harvest, and provides conditions for improved yields of the following crops. From a sustainability point of view, growing *Brassica carinata* as a winter cover may not necessarily displace production of food crops; since the land had been previously agricultural, there are no direct land use change consequences.

In other embodiments of the invention, *Brassica carinata* can be grown in semi-arid regions as a summer crop as part of a rotation with summer and winter cereal crops (for example winter and summer wheat). Similarly, *Brassica carinata* can be cultivated in multiyear rotations in combinations with leguminous crops (such as peas, lentils, peanuts, and soybeans) and cereals (such as corn, wheat, barley, rye, oats, or spelt) in areas with high summer temperatures (July average temperatures of 18-24°C) and limited total rainfall (less than 200-500 mm annually). In the southern hemisphere, the crop can be sown in late autumn or early winter into moist soil. In higher rainfall zones, it can be sown as late as early spring.

*Brassica* crops have long been shown to be beneficial when grown in rotations with cereals such as wheat, an important food crop amenable to production in semiarid regions due to its shorter growing season and tolerance to climate extremes. Rotations with oilseed as well as forage *Brassica* have consistently demonstrated a beneficial effect on yield of the ensuing cereal crop, due to effects on improving soil structure and moisture conservation and to the ability to provide a break to the cycle of diseases that affect cereal performance (Angus, *et al.,* 2011). The ability to break cereal disease cycles stems from *Brassica's* lack of susceptibility to many cereal diseases, but may also derive from the ability to actively discourage persistence of soil pathogens via the biofumigant activity of root exudates and residues (Kirkegaard and Sarwar, 1998). *Brassica carinata* is also amenable to conservation or no-tilling paradigms that allow for additional conservation of soil moisture as well as reducing the release of stable deposits of organic carbon from the disturbed soil layers. Once again in the semiarid environment, practice of the invention would allow for the sustainable production of biofuel feedstock from a non-food crop either as part of a rotation where its cultivation replaces fallow or is carried out on marginal land. In either case, there would be minimal direct or indirect land use change as a consequence of carinata's cultivation in this environment. The sequestering of atmospheric CO₂ as soil organic carbon would further reduce GHG lifecycle emissions with the additional benefit of providing conditions for improved yields of food crops grown in rotation.

In other embodiments of the invention, *Brassica carinata* can also be grown as a spring-seeded, fall-harvested crop in northern temperate regions as part of a rotation with summer and winter cereal crops, whereby the *Brassica carinata* follows harvest of preceding winter cereal replacing a summer fallow and is followed post-harvest by seeding of winter cereal crop. Amenable cereal crops include wheat, barley, rye or oats. As well as the benefits accrued due to replacement of fallow, the additional benefits due to increased overall productivity and reduced direct and indirect land use change means that biofuel produced from second generation (non-fuel) oilseed-based feedstocks, such as carinata oil, may meet the EU's directive that favors second generation feedstocks by allowing for double counting towards their mandatory volumes. *Brassica carinata's* greater tolerance to early season frost and its ability to better cope with higher heat and lower moisture during flowering and seed set, as well as its resistance to lodging, allows it to better withstand early and late season weather extremes (Seepaul *et al.,* 2015), making it a more reliable oilseed cropping option for producers in semi-arid regions.

Similarly, *Brassica carinata* can be cultivated in multiyear rotations in combinations with leguminous crops (such as peas, lentils, peanuts, and soybeans) and cereals (such as corn, wheat, barley, rye, oats, or spelt) in areas with high summer temperatures (July average temperatures of 18-24°C) and limited total rainfall (less than 200-500 mm annually). In the southern hemisphere, the crop can be sown in late autumn or early winter into moist soil. In higher rainfall zones, it can be sown as late as early spring.

Thus, *Brassica carinata* can be cultivated in a number of climates in rotation with a variety of summer or winter cereal, leguminous, or other crops to produce an oilseed that yields both oil feedstock for biofuel manufacturing as well as meal for livestock feed. The raw materials produced from the grain constitute virtually all the mass of the seed with little or no waste produced. The substantial plant residues left after harvest of the grain are returned to the field and contribute in large part of increasing the soil organic carbon and reducing the amount of carbon released as CO₂ to the atmosphere. Increased carbon content of the soil results in better soil structure, moisture retention and improved nutrient balance which improves the growing conditions for subsequent crops. Moreover, in rotations with other crops, carinata can provide a disease break benefiting the following crops productivity. *Brassica carinata* can also be seeded directly in the stubble left from previous crops. This practice, known as conservation-tillage or no-tillage type agriculture, conserves soil moisture in semi-arid regions, preserves soil structure and reduces the evolution of GHG from fuel use during operation of the tilling equipment. In total, cultivation of carinata provides a feedstock for biofuel production while providing measurable reductions in GHG emission (as measured via various GHG auditing models) across a number of production scenarios and geographies.

Based on its yield of oil seed, *Brassica carinata* not only provides a feedstock for the production of potential alternative to fossil fuels but, by enhanced production of biomass, can also provide an efficient mechanism for capturing and returning carbon to the soil. Soils also constitute a potential sink for sequestering of carbon and reduction of emissions into the atmosphere. Of all the environmental pools of carbon, soil is second in size only to the oceans, and comprises an estimated content of more than 2.3 GT of organic carbon (Jobbagy and Jackson, 2000) representing more than 4 times the amount of carbon accumulated in total plant biomass. Moreover, due to factors such as intensive agriculture, deforestation, erosion, etc. actual soil carbon stocks are relatively depleted in relation to their maximal capacity. It is estimated that the incremental capacity for carbon sequestration in soils may exceed 50 to 100 GT (Lal 2008a, Lal 2008b).

In one aspect of the invention, *Brassica carinata* is planted into the stubble of a harvested crop, with or without an intervening fallow, such that the preceding crop, which was not itself *Brassica carinata,* was the last crop to be harvested prior to seeding of the carinata.

In one embodiment, the preceding crop is a leguminous crop, which may include the following annual crops: beans, peas, lentils, soybeans, peanuts or alfalfa. Legumes are a useful crop choice in rotations due to their ability to fix atmospheric nitrogen, increasing the nitrogen content of the soil. Oilseed crops such as *Brassica carinata* are notable for requiring significant amounts of nitrogen for maximal yields. As a crop following legumes in rotation, *Brassica carinata* can utilize the accumulated soil nitrogen, which in turn reduces their requirement for nitrogen-containing fertilizers. It is well known that production of ammonia-based fertilizers using methods such as the Haber process results in significant emission of CO₂, which is a major co-product of the reaction. Moreover, reduction of exogenously added inorganic nitrogen fertilizer may also reduce soil emission of nitrous oxide produced by action of soil borne bacteria and microflora. Nitrous oxide, a potent greenhouse gas, one gram of which being the equivalent of 265 grams of CO₂, also contributes significantly to overall carbon intensity for plant-based biofuel pathways. As a final benefit of carinata following legumes in a rotation, the residue remaining after legume harvest is of a consistency that does not impact good carinata seed-soil contact, resulting in better emergence and establishment of the carinata crop, and allowing for the use and benefits of no-till or reduced-till agriculture.

To reduce GHG production resulting from application of excess amount of inorganic nitrogen fertilizer, in one embodiment of the invention, the land management practices comprise reducing use of inorganic nitrogen fertilizer compared to a recommended amount of nitrogen fertilizer for *Brassica carinata* for the growing environment. In some embodiments, the land management practices comprise reducing use of inorganic nitrogen fertilizer to between about 40% to about 100% of the recommended amount of nitrogen fertilizer for *Brassica carinata* in the growing environment. In some embodiments, the land management practices comprise reducing use of inorganic nitrogen fertilizer to between about 40% to about 90% of the recommended amount of nitrogen fertilizer for *Brassica carinata* in the growing environment. In still other embodiments, the land management practices comprise reducing use of inorganic nitrogen fertilizer to between about 50% to about 70% of the recommended amount of nitrogen fertilizer for *Brassica carinata* in the growing environment. Such practices of reducing use of inorganic nitrogen fertilizer would be beneficial, for example, when soil nitrogen levels prior to planting *Brassica carinata* are found to be high, such as when *Brassica carinata* is planted following harvest of a leguminous crop or after harvest of a first crop to which high amounts of nitrogen fertilizer had been applied.

In one embodiment of the invention, a *Brassica carinata* variety is planted into the stubble of a harvested crop, with or without an intervening fallow, in regions with a climate classified as being of tropical moist according to guidelines established by Directive 2009/28/EC such that all months may be frost free, with temperatures greater than 18°C in marine areas and while mainly wet, 3-5 months can be drier during winter. In some embodiments, planting of *Brassica carinata* occurs in fall or winter for harvest in spring or summer. In other embodiments, planting of *Brassica carinata* occurs in spring or summer for harvest in the fall or winter. In some embodiments, the *Brassica carinata* variety is chosen from among regionally adapted varieties selected for one or more traits selected from the group consisting of superior yield of oil per area planted, shorter time to maturity, improved frost tolerance, improved disease resistance, or resistance to pod shatter.

In another embodiment of the invention, *Brassica carinata* is planted into the stubble of a harvested crop, with or without an intervening fallow, in regions with a climate classified as being of warm temperate, moist characteristic as defined by Directive 2009/28/EC as being of moderate to high humidity year-round, with no singular dry season and with more than 8 months having a temperature of 10°C or higher. In some embodiments, planting of *Brassica carinata* occurs in fall or winter for harvest in spring or summer. In other embodiments, planting of *Brassica carinata* occurs in spring or summer for harvest in the fall or winter. In some embodiments, the *Brassica carinata* variety is chosen from among regionally adapted varieties selected for one or more traits selected from the group consisting of superior yield of oil per area planted, shorter time to maturity, tolerance to drought, improved disease resistance, or resistance to pod shatter.

Crop Rotation Scenarios: The present invention can be carried out in a number of different climate zones in which *Brassica carinata,* when planted in rotation with a first crop, is planted into the stubble of the harvested first crop. The season for planting and harvesting of the *Brassica carinata* may vary on the geography and crop rotation practices in each region. As described above, crop rotations that include cereals and *Brassica* oilseeds such as *Brassica carinata* can be beneficial for cereal yields and quality since, not being infectible or capable as serving as a host, the *Brassica* oilseed crop can provide a temporal and physical break in the cycle of diseases that affect cereals, thus not permitting those diseases to become persistent. The roots and harvest residues of *Brassica carinata* contain toxic substances such as glucosinolates that can also actively deter the spread of pathogenic organisms in the soil.
- **Scenario A**: In one embodiment of the present invention, *Brassica carinata* is planted into the stubble of a harvested crop, with or without an intervening fallow, in regions with a climate classified as being tropical, moist, with planting of *Brassica carinata* occurring in fall or winter for harvest in spring or early summer. In some embodiments, the harvested crop such a leguminous crop including, but not limited to, beans, pea, peanut, lentil, and soybean. In other embodiments, the harvested crop is a cereal crop including, but not limited to, wheat, barley, rye, oats or corn. In other embodiments, the harvested crop is cotton or sesame.
- **Scenario B**: In one embodiment of the present invention, Brassica *carinata* is planted into the stubble of a harvested crop, with or without an intervening fallow, in regions with a climate classified as being tropical, moist, whereby planting of *Brassica carinata* occurs in spring for harvest in summer or fall. In some embodiments, the harvested crop such a leguminous crop including, but not limited to, pea, lentil, and soybean. In other embodiments, the harvested crop is a cereal crop including, but not limited to, wheat, barley, rye, oats or corn.
- **Scenario C:** In one embodiment of the present invention, *Brassica carinata* is planted into the stubble of a harvested crop, with or without an intervening fallow, in regions with a climate classified as being warm temperate moist, whereby planting of *Brassica carinata* occurs in fall or winter for harvest in spring or summer. In some embodiments, the harvested crop such a leguminous crop including, but not limited to, pea, lentil, and soybean.
- **Scenario D:** In one embodiment of the present invention, *Brassica carinata* is planted into the stubble of a harvested crop, with or without an intervening fallow, in regions with a climate classified as being warm temperate moist, whereby planting of *Brassica carinata* occurs in spring or summer for harvest in the fall. In some embodiments, the harvested crop is a cereal crop including, but not limited to, wheat, barley, rye, oats or corn.
- **Scenario E:** In one embodiment of the present invention, *Brassica carinata* is planted into the stubble of a harvested cereal crop, with or without an intervening fallow, in regions with a climate classified as being warm temperate dry, whereby planting of *Brassica carinata* occurs in fall or winter for harvest in spring or summer. In some embodiments, the harvested cereal crop is corn. In other embodiments, the harvested cereal crop is wheat.
- **Scenario F:** In one embodiment of the present invention, *Brassica carinata* is planted into the stubble of a harvested cereal crop, with or without an intervening fallow, in regions with a climate classified as being as being cool temperate dry, whereby planting of *Brassica carinata* occurs in spring for harvest in summer or fall. In some embodiments, the harvested cereal crop is corn. In other embodiments, the harvested cereal crop is wheat.
- **Scenario G:** In one embodiment of the present invention, *Brassica carinata* is planted into the stubble of a harvested cereal crop, with or without an intervening fallow, in regions with a climate classified as being cool temperate moist, whereby planting of *Brassica carinata* occurs in spring for harvest in fall. In some embodiments, the harvested cereal crop is corn. In other embodiments, the harvested cereal crop is wheat.
- **Scenario H:** In one embodiment of the present invention, *Brassica carinata* is planted into the stubble of a harvested cereal crop, with or without an intervening fallow, in regions with a climate classified as being as being tropical dry, whereby planting of *Brassica carinata* occurs in fall or winter for harvest in spring or summer. In some embodiments, the harvested cereal crop is corn. In other embodiments, the harvested cereal crop is wheat.

In any of the above-described embodiments and crop rotation scenarios, the field may be subjected to reduced (medium) tillage, low tillage, or no tillage prior to seeding. As is known by those skilled in the art, seeding carinata into stubble, particularly cereal stubble, under circumstances whereby no-tillage or low tillage management practices are employed, will entail use of seeding practices and machinery designed to ensure consistent contact at the appropriate depth between the seed and soil surface in said stubble. Those skilled in the art are also aware that snow can further compact the soil and, as described previously, where low-till or no-till land management practices preclude removal of heavy cereal stubble or loosening of compacted topsoil, proper attention must be paid to seeding carinata using appropriate methods and machinery to ensure consistent seed to soil contact at the appropriate soil depth.

In any of the above-described embodiments and crop rotation scenarios, seeding of *Brassica carinata* is carried out using a seed drill or similar implement set at a depth of 0.50 cm, 0.63 cm, 1.25 cm, 1.9 cm, 2.5 cm, 3.75 cm, or 5 cm, or any depth therebetween, and at a seeding rate of 3.0 kg seed/ha, 4.0 kg seed/ha, 5.0 kg seed/ha, 5.6 kg seed/ha, 6.7 kg seed/ha, 7.8 kg seed/ha, 9.0 kg seed/ha, 10.1 kg seed/ha, 11.2 kg seed/ha, or any rate therebetween. The spacing rows may be set at 10 cm, 20 cm, 30 cm, 40 cm, 50 cm, or any distance therebetween. As is known by those skilled in the art, as described previously, where low-till or no-till land management practices precludes removal of heavy cereal stubble or loosening of compacted topsoil, proper attention must be paid to seeding carinata using appropriate methods and machinery to ensure consistent seed to soil contact at the appropriate soil depth.

In any of the above-described embodiments and crop rotation scenarios, inorganic (mineral) fertilizer is applied by top dressing, side dressing, broadcast or by foliar application. In some embodiments, the inorganic (mineral) fertilizer comprises one or more of inorganic nitrogen (N) fertilizer, phosphorus fertilizer, potassium fertilizer, and sulfur fertilizer. In some embodiments of the invention: inorganic nitrogen (N) fertilizer is applied at a rate of 30 kg/ha, 45 kg/ha, 56 kg/ha, 67 kg/ha, 78 kg/ha, 90 kg/ha, 101 kg/ha, 112 kg/ha, 123 kg/ha, 135 kg/ha, 150 kg/ha, 165 kg/ha, or any rate therebetween; phosphorus (P) fertilizer is added at a rate of 22, 34, 45, or 56 kg, or any amount therebetween, of equivalent P₂O₅ per hectare; potassium (K) is added at a rate of 30, 45, 56, 67, 78, 90, 101, or any amount therebetween, of equivalent K₂O per hectare; and sulfur (S) fertilizer is added at a rate of 11 kg/ha, 17 kg/ha, 22 kg/ha, 28 kg/ha, 34 kg/ha, 40 kg/ha, or any rate therebetween. In some embodiments, the inorganic N fertilizer and the S fertilizer are applied in a split dose, one half at planting and the other half prior to flowering, while the P and K fertilizers are applied in a single dose at planting. In loamy soils, where inorganic N fertilizer and the S fertilizer are applied in split dose, one quarter to one third of the inorganic N fertilizer and one third to one half of the S fertilizer are added at planting, with the remainder added at bolting, while the P and K fertilizers are applied in a single dose at planting. In deep sandy soils, the fertilizer can be applied in three doses: at planting or first plant emergence, one-third of the inorganic N fertilize, one-half of the S fertilizer, one-half of the K fertilizer, and all of the P fertilizer is added at planting or first plant emergence; at bolting, one-third of the inorganic N fertilizer and the remaining S and K fertilizers are added; and finally, at early flowering, the remaining N is added at early flowering.

In any of the above-described embodiments and crop rotation scenarios, manure and/or organic fertilizer can be used to provide some or all of the nitrogen fertilizer required during cultivation of carinata. Manure can be applied by broadcasting, banding, incorporation, or other methods known to one of skill in the art, using a manure spreader, lump spreader, tank wagon, or other suitable equipment known to one of skill in the art. The manure may be one or more of poultry litter, cattle feces, swine feces, or other agricultural waste material rich in nitrogen and other nutrients. As is known by one of skill in the art, the amount of manure applied to the field will depend on the composition of the manure, particularly on the nitrogen content. Typical application rates for manure range from 0.5 - 10 tonnes/ha, or any application rate therebetween. For example, the manure may be applied at a rate of about 0.5, 1, 1.5, 2, 2.5, 3, 3.5, 4, 4.5, 5, 5.5, 6, 7, 8, 9, or 10 tonnes/ha. When applied at this rate, the manure can provide from about 20% to 100%, or any percentage therebetween, of the nitrogen fertilizer required during cultivation of *Brassica carinata.* For example, the manure can provide about 20%, 25%, 30%, 35% 40%, 45%, 50%, 55%, 60%, 65%, 70%, 75%, 80%, 85%, 90%, 95%, or 100% of the nitrogen fertilizer required for cultivation of *Brassica carinata.* In some embodiments, the manure can provide from about 30% to 90%, or any percentage therebetween, of the nitrogen fertilizer required for cultivation of *Brassica carinata.* In other embodiments, the manure can provide from about 40% to 80%, or any percentage therebetween, of the nitrogen fertilizer required for cultivation of *Brassica carinata.* In other embodiments, the manure can provide from about 50% to 75%, or any percentage therebetween, of the nitrogen fertilizer required for cultivation of *Brassica carinata.*

Based on a conservative estimate of yield for carinata production in US semiarid regions of 2090 kg of grain per hectare (equivalent to 899 kg of oil per ha assuming 43%w/w oil content) by 2022, with nutrient inputs of 45-90 kg/ha of inorganic N fertilizer" 17-34 kg/ha of P fertilizer, 0-11 kg/ha K fertilizer, 3.1 kg/ha of pesticide, and 32.7 L/ha of diesel fuel, and assuming GHG emissions associated with crushing, oil storage and transport, bio-fuel manufacturing, and biofuel distribution were roughly equivalent to that of soybean and camelina, the EPA-estimated GHG aggregate emission reduction in a hypothetical carinata pathway for production of biomass biodiesel or advanced fuels such as HVO would allow a producer to obtain Type 4 or Type 5 RIN credits (EPA-HQ-OAR-2015-0093-; FRL-9926-80- OAR; Notice of Opportunity to Comment on an Analysis of the Greenhouse Gas Emissions Attributable to Production and Transport of *Brassica carinata* Oil for Use in Biofuel Production. Federal Register, Vol. 80, No. 79, Friday April 24, 2015, p 22996-23003; https://www.gpo.gov/fdsys/pkg/FR-2015-04-24/pdf/2015-09618.pdf). Thus, an aspect of this invention is that carinata represents a non-food, oilseed crop that can be cultivated in semiarid environments to provide an optimal biofuel feedstock and achieve significant reductions in GHG emissions, while improving soil quality which can support improved yield of subsequent food crops.

When grown under any of above-described embodiments or crop rotations scenarios, *B. carinata* will sequester from about 0.5 to about 5.0 tonnes of CO₂ per hectare per year, or any amount of CO₂ therebetween, into soil. For example, growth of *B. carinata* under any of above-described embodiments or crop rotations scenarios will sequester 0.5, 1.0., 1.5, 2.0, 2.5, 3.0, 3.5, 4.0, 4.5 or 5.0 tonnes of CO₂ per hectare per year, or any amount of CO₂ therebetween, into the soil.

Feedstock produced from the *B. carinata* grain harvested from B. carinata under any of above-described embodiments or crop rotations scenarios can be used to produce a low carbon intensity (low CI) biofuel, such as biodiesel or jet fuel. In some embodiments, the low CI biofuel has a carbon intensity value that is reduced by least 20, 40, 60, 80, 100, 120, 140, 160, 180, 200 or more g CO_{2eq}/MJ energy produced relative to the carbon intensity value of a corresponding conventional fuel produced from fossil fuel feedstock. In other embodiments, the low carbon intensity biofuel has a carbon intensity value that is reduced by about 50 to about 200 g CO_{2eq}/MJ energy produced, or any amount therebetween, relative to the carbon intensity value of a corresponding fuel produced from a fossil fuel feedstock. In other embodiments, the low carbon intensity biofuel has a carbon intensity value that is reduced by about 75 to about 200 g CO_{2eq}/MJ energy produced, or any amount therebetween, relative to the carbon intensity value of a corresponding fuel produced from a fossil fuel feedstock. In other embodiments, the low carbon intensity biofuel has a carbon intensity value that is reduced by 100-200 g CO_{2eq}/MJ energy produced, or any amount therebetween, relative to the carbon intensity of a corresponding fuel production a from fossil fuel feedstock.

Similarly, growth *of B. carinata* under any of above-described embodiments or crop rotations scenarios will reduce GHG Lifecycle emissions by about 60-400%, or any percentage therebetween, when used for production of green (renewable) diesel and when measured relative to GHG production during the refining and production of conventional diesel from fossil fuel feedstock. For example, growth of *B. carinata* under any of above-described embodiments or crop rotations scenarios will reduce GHG Lifecycle emissions by about 60%, 65%, 70%, 75%, 80%, 85%, 90%, 95%, 100%, 125%, 150%, 175%, 200%, 225%, 250%, 275%, 300%, 325%. 350%, 375%, or 400% when used for production of green (renewable) diesel and when measured relative to GHG production during the refining and production of conventional diesel from fossil fuel feedstock. In some embodiments or crop rotations scenarios, growth of *B. carinata* will reduce GHG Lifecycle emissions by about 75-300%, or any percentage therebetween, when used for production of green (renewable) diesel and when measured relative to GHG production during the refining and production of conventional diesel from fossil fuel feedstock. In some embodiments or crop rotations scenarios, growth *of B. carinata* will reduce GHG Lifecycle emissions by about 90-250%, or any percentage therebetween, when used for production of green (renewable) diesel and when measured relative to GHG production during the refining and production of conventional diesel from fossil fuel feedstock.

### Examples

**Example 1: *Brassica carinata* sequentially grown as winter cover following peanut in rotation.** This example demonstrates the cultivation of *Brassica carinata* as cover crop in the tropical moist climate zone for production of feedstock for low carbon intensity biofuel manufacturing and protein rich meal for livestock feed applications. As an example of cultivation in this zone, *Brassica carinata* was cultivated sequentially during winter 2015-2016 as a winter cover crop, replacing fallow, on two farms in the SE USA (North Florida). Farm One was located near Jay, Fl. and Farm 2 was located near Altha, Fl. The previous crop cultivated on both farms was peanut, a leguminous crop species.

Table 5 summarizes how the cultivation of the carinata crop was carried out on each farm. Both farms were located in the northern part of Florida, a region classified as Tropical Moist as described above and in Table 2. The soil in region of North Florida where both farms are situated has been classified as acrisol, which belongs to SET #6 of the Soil Classifications in the Definitions above.

The fields were prepared for seeding using a no-till management approach. Seeding was carried out using *Brassica carinata* AAC-A120 at rates and seeding depth within the optimal ranges suggested in the Agrisoma 2015 Growers Guide for the region (https://agrisoma.com/ckfinder/userfiles/files/2017_18_SE _Handbook.pdf). The inputs used in both farms (listed in Table 5) also fall within the suggested ranges provided in the grower's manual. In order to most accurately access the totality of GHG emissions associated with the cultivation of carinata, the farm managers recorded the fuel usage of all farm machinery used during the cultivation and harvest of the carinata crops (tabulated in Table 5). All watering was achieved via natural precipitation, thus no supplemental irrigation was required nor used. At maturity, the seed was harvested by straight cutting via combine and virtually all plant materials, aside from the collected grain were returned to the field. Net and per ha yields of harvested carinata grain (at 10% moisture content) from both farms are tabulated in Table 5.

**Table 5: Carinata cultivation details (Farm 1 and Farm 2)**

| | **Units** | **Farm 1 (FL)** | **Farm 2 (FL)** |
|---|---|---|---|
| **Cultivation details** | | | |
| Site | | North Fl. | North Fl. |
| Previous Crop | | Peanuts | Peanuts |
| Carinata area seeded | ha | 23.87 | 8.09 |
| Seeding rate | kg/ha | 5.60 | 4.48 |
| Total seeds used | kg | 133.68 | 36.25 |
| Yield | kg | 46,576.98 | 13,957.69 |
| Yield/area | kg/ha | 1,951 | 1,725 |

| **Energy sources used during cultivation** | | | |
|---|---|---|---|
| Diesel usage | MJ ha⁻¹ year⁻¹ | 928.14 | 3,704.37 |
| **Agro-chemicals** | | | |
| N-fertilizer (kg N) | kg N ha⁻¹year⁻¹ | 78.4 | 129.9 |
| Manure | kg N ha⁻¹ year⁻¹ | - | - |
| CaO- fertilizer (kg CaO) | kg CaO ha⁻¹ year⁻¹ | - | - |
| K₂O-fertiliser (kg K₂O) | kg K₂O ha⁻¹ year⁻¹ | 78.4 | 89.6 |
| P₂O₅- fertilizer (kg P₂O₅) | kg P₂O₅ ha⁻¹ year⁻¹ | 22.4 | 44.8 |
| Pesticides | kg ha⁻¹ year⁻¹ | - | - |

**Example 2: *Brassica carinata* sequentially grown as winter cover following cereal (corn) in tropical moist climate (Florida/SE USA).** This example demonstrates cultivation of *Brassica carinata* as cover crop in the tropical moist climate zone for production of feedstock for biofuel manufacturing and protein rich meal for livestock feed applications. *Brassica carinata* was cultivated sequentially during winter 2015-2016 as a winter cover crop, replacing winter fallow, on farms in the SE USA. The previous crop cultivated was corn, an example of a cereal crop species, and in a departure from erstwhile practice, the preceding corn crops' harvest residues were not incorporated by tilling. Table 6 summarizes the details of carinata cultivation, carried out in the tropical moist climate zone as described above and in Table 2. The soils in this region of North Florida are as sandy arenosolic- type (see SET #3 of the Soil Classifications in the Definitions above).

**Table 6: Carinata cultivation details**

| | **Units** | **Data** |
|---|---|---|
| **Cultivation details** | | |
| Site | | FL |
| Previous Crop | | Corn |
| Seeding rate | kg/ha | 4 |
| Yield | kg/ha | 2,114 |
| Yield ⁽¹⁾ | MJ/ha | 50,229 |

| **Energy sources used during cultivation** | | |
|---|---|---|
| Diesel usage ⁽²⁾ | MJ ha⁻¹ year⁻¹ | 1,385 |
| **Agro chemicals** | | |
| N-fertilizer (kg N) | kg N ha⁻¹ year⁻¹ | 141.1 |
| Manure | kg N ha⁻¹ year⁻¹ | - |
| CaO- fertilizer (kg CaO) | kg CaO ha⁻¹ year⁻¹ | 340.0 |
| K₂O-fertiliser (kg K₂O) | kg K₂O ha⁻¹ year⁻¹ | 89.6 |
| P₂O₅- fertilizer (kg P₂O₅) | kg P₂O₅ ha⁻¹ year⁻¹ | 88.5 |
| Pesticides | kg ha⁻¹ year⁻¹ | - |
| **Transport of grain** | | |
| Truck to grain handler | km | 33.8 |
| ⁽¹⁾Based on Lower heating Value (LHV) for rapeseed at 0% moisture of 26.4 MJ/kg, taken from: JEC E3-database (version 31-7-2008). | | |
| ⁽²⁾ Based on density of 832 kg/m³, LHV of 43.1 kg/MJ, taken from: JEC E3-database (version 31-7-2008) | | |

In the above described environment, *Brassica carinata* was seeded from mid to late November, into the stubble of a previous corn crop, typically at a depth of 1.25 -2.5 cm. Two inbred open-pollenated carinata varieties Resonance AAC-A120 (currently provisionally protected by Plant Breeders Rights in Canada, Application number 15-8718) or Avanza 641 (WO 2017/181276A) are currently recommended for this growing environment, the latter variety selected on the basis of regional adaptation high yield, lower glucosinolate content and increased frost tolerance. Seeding rate was adjusted to 4 kg/ha to achieve an optimal plant density in the range of 80-180 plants per m². Inputs used were as described in Table 6 and comprised inorganic nitrogen, potassium and phosphorus-based fertilizers in the amounts suggested. Arenosolic soils are moderately acidic, necessitating the addition of dolomitic lime (CaO). Inorganic nitrogen fertilizer was applied at a rate of 141.1 kg/ha, which although higher than typically recommended for carinata, can be justified for sandy soil types in moist tropical environments where nitrogen can be leached away from the root zones tends to leach out of the root zone.

In order to most accurately access total GHG emissions during cultivation, usage of all motorized farm fuel consumption of all motorized farm used during cultivation and harvest of the carinata crop were recorded. All watering was achieved via precipitation and so no supplemental irrigation was required nor used. Grown as a winter cover crop under shorter daylength conditions, *Brassica carinata* required slightly more than 5 months to reach maturity, at which point carinata it was harvested by straight cutting via combine. The grain was collected and virtually all plant materials aside from the collected grain were returned to the field. Per ha grain yields (at 10% moisture content) and cumulative fuel usage fuel are tabulated in Table 6.

**Example 3: *Brassica carinata* as summer cover following legume (lentil) in cool temperate dry climate (NT).** This example demonstrates the cultivation of carinata as a summer cover crop for production of feedstock for biofuel manufacturing and protein rich meal for livestock feed applications. *Brassica carinata* is cultivated as a summer cover crop, planted into the stubble of a previous lentil crop, in the cool temperate dry climate zone of the Northern Tier US states and southern prairies of Canada. Table 7 summarizes the details of cultivation, carried out in a climatic region classified as cool temperate dry as described above and in Table 2. Examples of such regions include the northern Tier States of the US as well as the southern Prairies of western Canada. Soils in these regions are classed as high activity clay soils (Commission Decision of June 2010 on guidelines for the calculation of land carbon stocks for the purpose of Annex V to Directive 2009/28/EC). Lentils, a leguminous species are an increasingly important crop in these regions and are often grown in rotations comprising cereals such as wheat and/or *Brassica* oilseeds.

In the above described environment, *Brassica carinata* are seeded from mid-April to early May, typically when soil temperature exceeds 4-5 °C into the stubble of a previous lentil crop and at a depth of 1.25 -2.5 cm. Two inbred open-pollenated carinata varieties, Resonance AAC-A120 (protected by plant breeders rights in Canada, Application date April 9, 2015 application number 15-8718) and 3A22 (US Provisional Patent Application No. 62/326111 filed April 22, 2016 and PCT International Application No. PCT/CA2017/050474 filed April 18, 2017) are currently recommended for this growing environment, the latter variety selected on the basis of regional adaptation, high yield, lower glucosinolate content and earlier maturity. Seeding rate is adjusted to achieve a plant density in the range of 80-180 plants per m² corresponding to a seeding rate between 5 and 9 kg/ha. Inputs are as described in Table 7 and comprise inorganic nitrogen, potassium and phosphorus-based fertilizers in the amounts suggested. Soil pH in these regions are usually 7.0 or higher and thus dolomitic lime application is not required. While 90 kg/ha of inorganic nitrogen is the recommended dosage, since lentils do increase soil nitrogen levels due to their roots' ability to fix atmospheric nitrogen, it is possible to reduce the amount of added nitrogen accordingly for subsequent crops. Therefore, Table 7 lists two cultivation scenarios that differ only with respect to the amounts of added inorganic nitrogen: one (scenario 1) with the normal recommended dosage and the other (scenario 2) with inorganic nitrogen application halved to take advantage of the nitrogen provided by the preceding lentil crop.

**Table 7: Carinata cultivation details**

| | **Units** | **Scenario 1** | **Scenario 2** |
|---|---|---|---|
| **Cultivation details** | | | |
| Site | | Northern Tier | Northern tier |
| Previous Crop | | Lentils | Lentils |
| Seeding rate | kg/ha | 5 | 5 |
| Yield | kg/ha | 1,800 | 1,800 |
| Yield ⁽¹⁾ | MJ/ha | 42,768 | 42,768 |

| **Energy sources used during cultivation** | | | |
|---|---|---|---|
| Diesel usage ⁽²⁾ | MJ ha⁻¹ year⁻¹ | 1000 | 1000 |
| **Agro chemicals** | | | |
| N-fertilizer (kg N) | kg N ha⁻¹ year⁻¹ | 90 | 45 |
| Manure | kg N ha⁻¹year⁻¹ | - | - |
| CaO- fertilizer (kg CaO) | kg CaO ha⁻¹year⁻¹ | - | - |
| K₂O-fertiliser (kg K₂O) | kg K₂O ha⁻¹ year^{- 1} | 80 | 80 |
| P₂O₅- fertilizer (kg P₂O₅) | kg P₂O₅ ha⁻¹ year⁻¹ | 40 | 40 |
| Pesticides | kg ha⁻¹year⁻¹ | 10 | 10 |
| **Transport of grain** | | | |
| Truck to grain handler | km | 100 | 100 |
| ⁽¹⁾Based on Lower heating Value (LHV) for rapeseed at 0% moisture of 26.4 MJ/kg, taken from: JEC E3-database (version 31-7-2008) | | | |
| ⁽²⁾ Based on density of 832 kg/m³, LHV of 43.1 kg/MJ, taken from: JEC E3-database (version 31-7-2008) | | | |

In order to most accurately access the totality of GHG emissions associated with the cultivation of the usage of all farm implements and machinery that are used during all stages of the cultivation and harvest of the carinata crops and amounts of diesel fuel used in their operation are recorded. For this purpose, a default value of 1000 MJ/ha of diesel fuel usage is used, which represents a moderately high usage of motorized farm implements. All watering is achieved via natural precipitation and so no supplemental irrigation is required nor used. When grown as a summer cover crop, *Brassica carinata* usually reaches maturity within 4 months, at which point it is harvested by straight cutting via combine. The grain is collected and virtually all plant materials aside from the collected grain are returned to the field. Net and per ha yields of harvested carinata grain (at 10% moisture content) under both nitrogen usage scenarios are tabulated in Table 7.

**Example 4: *Brassica carinata* sequentially grown as winter cover following Legume (soybean) in warm temperate, moist climate (Uruguay).** This example demonstrates the sequential cultivation of *Brassica carinata* as a winter cover crop, replacing fallow, for production of feedstock for biofuel manufacturing and protein rich meal for livestock feed applications. *Brassica carinata* was cultivated as a winter cover crop, planted into the stubble of a previous soybean crop, in the warm temperate, moist climate of Uruguay. Table 8 summarizes the details of cultivation, carried out during the winter of 2015, in a climatic region classified as warm temperate moist as described above and in Table 2. Examples of such regions include much of the arable land of Uruguay. Soils in these regions are classed as high activity clay soils (Commission Decision of June 2010 on guidelines for the calculation of land carbon stocks for the purpose of Annex V to Directive 2009/28/EC;). Lentils, a leguminous species are an increasingly important crop in these regions and are often grown in rotations comprising cereals such as wheat and/or *Brassica* oilseeds.

**Table 8: Carinata cultivation details**

| | **Units** | **Data** |
|---|---|---|
| **Cultivation details** | | |
| Site | | Uruguay |
| Previous Crop | | Soybean |
| Seeding rate | kg/ha | 7 |
| Yield | kg/ha | 2,100 |
| Yield ⁽¹⁾ | MJ/ha | 49,896 |

| **Energy sources used during cultivation** | | |
|---|---|---|
| Diesel usage ⁽²⁾ | MJ ha⁻¹ year⁻¹ | 277 |
| **Agro chemicals** | | |
| N-fertilizer (kg N) | kg N ha⁻¹ year⁻¹ | 59.7 |
| Manure | kg N ha⁻¹ year⁻¹ | - |
| CaO- fertilizer (kg CaO) | kg CaO ha⁻¹ year⁻¹ | 18.3 |
| K₂O-fertiliser (kg K₂O) | kg K₂O ha⁻¹ year⁻¹ | 67.4 |
| P₂O₅- fertilizer (kg P₂O₅) | kg P₂O₅ ha⁻¹ year⁻¹ | 61.0 |
| Pesticides | kg ha⁻¹ year⁻¹ | 13 |
| **Transport of grain** | | |
| Truck to grain handler | km | 376 |
| ⁽¹⁾Based on Lower heating Value (LHV) for rapeseed at 0% moisture of 26.4 MJ/kg, taken from: JEC E3-database (version 31-7-2008) | | |
| ⁽²⁾ Based on density of 832 kg/m³, LHV of 43.1 kg/MJ, taken from: JEC E3-database (version 31-7-2008) | | |

During mid to late May, seventeen farms in Uruguay, comprising over 2400 ha, were seeded sequentially with carinata in the stubble of a previous soybean crop and at a depth of 1.25 - 2.5 cm. Two inbred open-pollenated carinata varieties, Resonance AAC-A120 (currently provisionally protected by PBR in Canada, application number 15-8718) and Avanza 641 (US plant variety patent application in preparation) were recommended for this growing environment, the latter variety selected on the basis of regional adaptation, high yield, lower glucosinolate content, frost tolerance and earlier maturity. Table 8 tabulates the average values for seeding rate, input levels, and yields across all farms. Seeding rate was adjusted to achieve an optimum plant density, corresponding to an average seeding rate of 7 kg/ha. Inputs are as described in Table 8 and comprise the average of inorganic nitrogen, potassium phosphorus and calcium (lime) based fertilizers used by all farms. Soil pH in these regions are often moderately acidic, as low as pH 5.7 and thus dolomitic lime application was carried out in order to reduce the soil acidity. Based on the results of soil nitrogen analysis, a total seasonal average 59.7 kg/ha of nitrogen was applied. This is lower than the recommended level of applied nitrogen (90 kg/ha) but reflects the levels of preexisting soil nitrogen, likely as a result of the preceding leguminous crop. Table 8 also tabulates the average levels of pesticide (comprising pesticide, herbicide and fungicide) usage across all farms, since the manufacturing of these products consumes energy and thus contributes to GHG emissions, the BioGrace Model determined the level of CO₂eq emissions contributed by these products and combined them into the total emissions for the cultivation phase.

As previously described, in order to most accurately assess the totality of GHG emissions associated with carinata cultivation, all farm implements and machinery that are used during the cultivation and harvest of the carinata crops and amounts of diesel fuel used in their operation were recorded. The average consumption of diesel fuel across all farms was 277 MJ of fuel per ha of diesel. All watering was achieved via precipitation and so no supplemental irrigation was required nor used. Since it was grown as a winter cover crop under short daylight hour conditions, *Brassica carinata* reached maturity within 5-6 months, (one to two months longer than required under summer cultivation conditions) at which point carinata was harvested by straight cutting via combine. The grain was collected and virtually all plant materials aside from the collected grain were returned to the field. Net and per ha yields of harvested carinata grain (at 10% moisture content) are tabulated in Table 8.

Compared to other oilseed crop varieties cultivated in Uruguay, *Brassica carinata* produces both high yield and high biomass. In a study carried out in Uruguay in 2016, *Brassica carinata* variety Avanza 641 was seeded under identical conditions in triplicated plots alongside several current varieties of open pollenated and hybrid spring canola-type commercial *Brassica napus* varieties. The plots were monitored during the course of cultivation for plant density, silique density, above-ground biomass at harvest, grain yield at harvest and harvest index. The data are summarized in Table 9.

**Table 9: Yield of grain and biomass for Brassica carinata Avanza 641 grown in parallel with hybrid canola varieties**

| **Material** | **Replicate** | **Plants/m²** | **Siliques/m²** | **Biomass (kg/ha)** | **Grain Yield (kg/ha)** |
|---|---|---|---|---|---|
| *B. carinata* Avanza 641 | 1 | 41.18 | 9932.35 | 16544.12 | 4369.12 |
| *B. carinata* Avanza 641 | 2 | 66.18 | 13902.94 | 21691.18 | 5866.18 |
| *B. carinata* Avanza 641 | 3 | 51.47 | 10105.88 | 16617.65 | 4586.03 |
| *B. napus* HYOLA 50 | 1 | 27.94 | 5591.18 | 10000.00 | 2510.29 |
| *B. napus* HYOLA 50 | 2 | 26.47 | 5222.06 | 9117.65 | 2277.21 |
| *B. napus* HYOLA 50 | 3 | 20.59 | 7841.18 | 13676.47 | 3238.97 |
| *B. napus* HYOLA 575 CL | 1 | 54.41 | 7614.71 | 14044.12 | 2833.82 |
| *B. napus* HYOLA 575 CL | 2 | 45.59 | 5847.06 | 9411.76 | 1780.15 |
| *B. napus* HYOLA 575 CL | 3 | 44.12 | 5335.29 | 9044.12 | 1422.79 |
| *B. napus* RIVETTE | 1 | 27.94 | 5182.35 | 10558.82 | 2645.59 |
| *B. napus* RIVETTE | 2 | 25.00 | 3979.41 | 7455.88 | 1732.35 |
| *B. napus* RIVETTE | 3 | 25.00 | 4483.82 | 10529.41 | 2889.71 |

The Least square means (LSM) of the replicates were calculated and comparison of the means using Tukey's test carried out to determine whether any significant differences were observed among the varieties tested (see Table 10). LSM values sharing the same letter for each measurement are not significantly different.

**Table 10: Grain yields and biomass accumulation for Brassica carinata Avanza 641 compared to canola hybrids.**

| **Variety** | **Yield (kg/ha)** | | **Biomass (kg/ha)** | | **Silique/m²** | | **Plants/m²** | |
|---|---|---|---|---|---|---|---|---|
| | **LSM** | **Letters** | **LSM** | **Letters** | **LSM** | **Letters** | **LSM** | **Letters** |
| Avanza 641 | 4940 | A | 18284 | A | 11314 | A | 53 | A |
| HYOLA 50 | 2675 | B | 10931 | B | 6218 | B | 48 | A |
| HYOLA 575 CL | 2012 | B | 10833 | B | 6266 | B | 26 | B |
| RIVETTE | 2422 | B | 9515 | B | 4549 | B | 25 | B |

As can be seen, under the conditions of cultivation employed in these Uruguayan studies, *Brassica carinata* significantly out yielded even the most current hybrid spring canola varieties. Contributing to this yield advantage were traits such a silique density and plant density both of which were significantly higher for *Brassica carinata* AVANZA 641. Previous work demonstrated higher above-ground biomass production of spring planted *Brassica carinata* varieties relative to other *Brassica* oilseed species in the US Norther Tier (Gesch, *et al.* 2015). The results presented herein demonstrate that *Brassica carinata* varieties selected for short daylength winter cultivation also produce significantly higher levels of above-ground biomass than other commercial *Brassica* oilseed crops, while maintaining high yield potentials. The abundant biomass production, if managed in conjunction with land management practices such as return of harvest residues to the field, conservation tillage, maintaining stubble, can contribute to significant return of plant nutrients and carbon to the soil (see below).

**Example 5: *Brassica carinata* sequentially grown as winter cover crop following cereal (wheat) in New South Wales.** This example demonstrates the cultivation of carinata as cover crop in the warm temperate dry and tropical dry climate zones, as typified by the wheat belt of New South Wales of eastern Australia for production of feedstock for biofuel manufacturing and protein rich meal for livestock feed applications. Here, in order to take advantage of the increased moisture that the winter season affords, *Brassica* oilseeds (predominantly Canola type varieties) are seeded in the fall, winter grown and harvested in spring or early summer after a growing season of up to 5-7 months. In similar fashion, *Brassica carinata* is sequentially cultivated as a winter cover crop, replacing winter fallow, on farms in a sub-region known to receive relatively high precipitation during winter. The previous crop cultivated is wheat, an example of a cereal crop species, and in a departure from erstwhile practice, the preceding crops' harvest residues are not incorporated by tilling. Table 11 summarizes the details of carinata cultivation, carried out in the warm temperate dry climate zone as described above and in Table 2. Much of the soil in this region is classified as either Luvisol, Vertisol or Calcisol, which are described, respectively, in SET # 9, SET #3 and SET #7 in the Soil Classification definitions, above.

In the above described environment, *Brassica carinata* is sequentially seeded from mid to late April to late May, into the stubble of a previous wheat crop, typically at a depth of 1.25 - 2.5 cm. Seeding rate is adjusted to 5 kg/ha to achieve an optimal plant density in the range of 80-180 plants per m². Inputs used were as described in Table 11 and comprised inorganic nitrogen-, potassium- and phosphorus-based fertilizers in the amounts suggested. 110 kg/ha of inorganic nitrogen fertilizer was used, which although higher than typically recommended for carinata, can be justified for sandy soil types in moist tropical environments where nitrogen tends to leach out of the root zone.

**Table 11: Carinata cultivation details**

| | **Units** | **Data** |
|---|---|---|
| **Cultivation details** | | |
| Site | | Australia (NSW) |
| Previous Crop | | Wheat |
| Seeding rate | kg/ha | 5 |
| Yield | kg/ha | 2000 |
| Yield ⁽¹⁾ | MJ/ha | 47520 |

| **Energy sources used during cultivation** | | |
|---|---|---|
| Diesel usage ⁽²⁾ | MJ ha⁻¹ year⁻¹ | 1000 |

| **Agro chemicals** | | |
|---|---|---|
| N-fertilizer (kg N) | kg N ha⁻¹year⁻¹ | 110(high)/55 (low) |
| Manure | kg N ha⁻¹ year⁻¹ | - |
| CaO- fertilizer (kg CaO) | kg CaO ha⁻¹ year⁻¹¹ | - |
| K₂O-fertiliser (kg K₂O) | kg K₂O ha⁻¹ year⁻¹ | 30 |
| P₂O₅- fertilizer (kg P₂O₅) | kg P₂O₅ ha⁻¹ year⁻¹ | 30 |
| Pesticides | kg ha⁻¹ year⁻¹ | 10 |

| **Transport of grain** | | |
|---|---|---|
| Truck to grain handler | km | 376 |
| ⁽¹⁾Based on Lower heating Value (LHV) for rapeseed at 0% moisture of 26.4 MJ/kg, taken from: JEC E3-database (version 31-7-2008) | | |
| ⁽²⁾ Based on density of 832 kg/m³, LHV of 43.1 kg/MJ, taken from: JEC E3-database (version 31-7-2008) | | |

**Example 6: GHG reduction for low CI biofuel using feedstock produced from *Brassica carinata* sequentially grown as winter cover following peanut in rotation.** To calculate the GHG footprint of carinata cultivation in the example of carinata cultivation described in Example 1, the BioGrace v 1.4 model was used (http://www. Biograce.net). The model follows the sustainability criteria of the Renewable Energy Directive (2009/28/EC, RED) which are also stated in the Fuel Quality Directive (2009/30/EC). The calculations in the BioGrace Excel tool follow a Life Cycle Assessment (LCA) perspective to evaluate the GHG emissions of one MJ of fuel. This means that:
- The functional unit is "the production and use of one MJ of fuel".
- All life cycle steps from biomass production to fuel distribution are taken into account (see Table 12) and presented in the calculation sheet within a dedicated module representing one step in the biofuel pathway. For biofuels, the use phase bears no emission of GHG as the CO₂ emitted is biogenic (and the CH₄ emissions occurring when burning a fuel are insignificant).
- A module gathers the inputs' consumptions and calculates the emissions of the three main gases contributing to climate change (CO₂, CH₄, and N₂O). Details of each gas' contribution are presented in the last step of the calculation. The sum of all three gases is expressed as equivalent amount of CO₂ (CO_{2eq}) required to produce the same GHG effect (g CO_{2eq}/MJ HVO fuel produced).
- GHG emissions of each module are then summarized to obtain the GHG emission of the whole pathway.

For the purposes of this example, however, while the feedstock produced would be used primarily for production of HVO for use as drop in fuel replacement in transport and aviation fuel applications, the BioGrace model is only being used to consider the GHG emissions from the cultivation phase of a carinata based biofuel pathway, including harvest, drying and transport of the grain to a storage location, in order to establish the potential for carinata cultivation to reduce the carbon intensity of an associated fuel pathway under conditions of cultivation where carinata is grown as winter cover replacing fallow and when following Legume (peanut) in a region within the tropical moist climate zone. Nevertheless, in order to estimate GHG emissions consistently and in a way that respected the functional unit, the presumed yield of HVO for the abbreviated pathway was taken to be 0.58 MJ HVO/ MJ of carinata seed. The steps in the production of HVO from cultivated *Brassica carinata* that are within the "well to tank" system boundary of the BioGrace GHG emissions calculator are shown in Figure 1. The allocation factor for the first three steps of carinata oil is 0.613.

GHG emission for inputs and fuel used during cultivation could be estimated, based on amounts of inputs or fuels used, by multiplying by appropriate emissions factors, similarly provided in the BioGrace spreadsheet. Emissions derived from fuel used during transport of seed, oil or fuel could be calculated on the basis of similarly provided emission factors for the appropriate fuel type multiplied by distance traveled and fuel efficiency of the particular mode of transport (e.g. rail, road or sea-going vessel).

Lower heating values, expressed as MJ/kg, and obtained from a table of such values provided with the BioGrace spread sheet, were used to determine the energy contents of the grains, oils, meals at various stages in the pathway and allowed for conversion to MJ per MJ of HVO, respecting the functional unit.

Diesel used to fuel tractors and farm equipment employed during the cultivation of carinata (for field preparations, seeding, application of inputs and harvesting) as well as electrical energy used for drying of harvested carinata grain also contribute to pathway GHG emissions and these are also accounted for as part of the cultivation phase.

GHG emission for fuel used during cultivation could be estimated, based on amounts of fuels used, by multiplying by appropriate emissions factors, similarly provided in the BioGrace spreadsheet.

Manufacturing of inputs used in crop cultivation, such as fertilizers and pesticides, have associated emissions that must be included as part of the lifecycle GHG emissions of the biofuel production pathway; these are estimated based on the amount of input used in the cultivation of the crops and default emission coefficient (g of GHG produced/kg input) available for the relevant input's manufacturing process (JEC E3-database; version 31-7-2008). There is also an additional source of emissions from cultivation that requires accounting, which is a consequence of field emissions of nitrous oxide (N₂O), a greenhouse gas 265 times more potent than CO₂. Such field emissions are further divided in to three additive categories: direct N₂O emissions from the field, indirect N₂O emissions due to leaching and runoff, and indirect N₂O emissions due to NH₃ and NOx volatilization. Field emissions are a consequence of the decomposition or burning of organic matter derived from crop residues as well as a consequence of nitrogen-based fertilizer usage itself and are estimated N₂O emissions module of the BioGrace spreadsheet (as described in the Definitions section of this application).

Transportation of the grain to collection and storage points maintained by commercial grain handlers was also a potential source of GHG emissions. The nature of the transport, fuel used during transport and distance traveled were all recorded and used to determine the net GHG emissions (Table 12). For the purposes of this example only transportation of grain to local storage is considered.

**Table 12: Transportation of grain, oil and fuel**

| | **Means of transport** | **Fuel** | **Distance Farm 1 (km)** | **Distance Farm 2 (km)** |
|---|---|---|---|---|
| Grain to local Storage | Truck | Diesel | 45.05 | 80.5 |
| Grain from Storage to port 1 | Truck | Diesel | 800.17 | 505.44 |
| Grain from port 1 to port 2 | Cargo ship | Diesel | 8423 | |
| Grain to crushing plant | Truck | Diesel | 30 | |
| Oil to Refinery | Truck | Diesel | 1178 | |
| HVO to distribution center | Truck | Diesel | 150 | |
| HVO to filling station | Truck | Diesel | 150 | |

All emissions tabulated during the cultivation drying and transport phase were added to give a total emission value for the cultivation phase (see Table 13 for cultivation phase emissions for Farm 1 and Table 14 for cultivation phase emissions for farm 2). For the cultivation of carinata and subsequent transport of the grain to storage at a local grain handler, an allocation factor is applied to the emissions to account for the fact that the carinata oil fraction comprises 63% of the energy of the seed and is the seed fraction that is uniquely processed into HVO. Thus, up until the point that the oil is processed into HVO, emissions produced are multiplied by the allocation factor.

**Table 13: Emissions due to cultivation of carinata (Farm 1 from Example 1)**

| | **Allocation factor** | **Non-allocated results** | **Allocated results** | **Total** |
|---|---|---|---|---|
| | | ***All results in g CO_{2eq}*/*MJ HVO*** | | |
| Cultivation of carinata | 61.3% | 50.47 | 30.91 | |
| Carinata drying | 61.3% | 0.99 | 0.61 | |
| **Cultivation E_{ec}** | | | | **31.5** |
| Transport of grain (truck) | 61.3% | 0.28 | 0.17 | |
| **Transport E_{td}** | | | | **0.17** |

**Table 14: Emissions due to cultivation of carinata Farm 2 from Example 1)**

| | **Allocation factor** | **Non-allocated results** | **Allocated results** | **Total** |
|---|---|---|---|---|
| | | ***All results in g CO₂eq* / *MJ HVO*** | | |
| Cultivation of carinata | 61.3% | 93.66 | 57.37 | |
| Carinata drying | 61.3% | 0.99 | 0.61 | |
| **Cultivation E_{ec}** | | | | **58.0** |
| Transport of grain (truck) | 61.3% | 0.48 | 0.29 | |
| **Transport E_{td}** | | | | **0.29** |

The BioGrace model considers one additional factor in the calculation of net GHG emission-i.e., the anticipated reductions in GHG emissions that may result in the case of improved land management practices being applied in the case of cultivating the bioenergy crop relative to the baseline situation. Emission reduction, termed E_{sca}, assumes that the improved land management practices result in increased carbon sequestration in the managed land, thus offsetting a portion of the emissions produced during the cultivation, processing and transportation phases of the pathway. In the specific case of carinata cultivated as described herein, additional emission reductions are expected due to the change from full tillage to no tillage and replacement of fallow with a groundcover crop that returns a high proportion of its biomass back to the soil on harvest. The BioGrace model quantifies and assigns an E_{sca} value in units of tonnes CO₂ returned to soil/ ha/year based on these improvements (see E_{sca} value, Table 15). This is subsequently converted to tonnes of CO₂ returned to soil/ MJ of HVO biofuel produced which is then used to reduce the net emissions of the entire pathway (see Tables 16 and 17).

As can be seen for carinata cultivation on Farm 1, the CO₂ eq emissions per MJ of HVO produced are negative (-35.6 tonnes of CO₂eq/ MJ of HVO produced) if only considering the cultivation phase of the HVO production pathway, denoting a net reduction of atmospheric GHG levels per unit fuel produced due to the cultivation of carinata under the conditions of cultivation on Farm 1. On Farm 2, the CO₂ eq emissions per MJ of HVO produced are also negative: -17.6 tonnes of CO₂eq/ MJ of HVO produced. Factors that contribute to the greater reduction in emissions experienced by Farm 1 include: a) lower usage of inorganic nitrogen fertilizer which contributed to reduced field GHG emissions as well as lifecycle emissions associated with the manufacturing of the nitrogen fertilizer; b) lower fuel use for farm equipment used during cultivation even though number of cultivated hectares was actually higher.

**Table 15: Improved land management (farms 1 and 2)**

| | **Actual land use** | **Reference land use** | **Reference** |
|---|---|---|---|
| **Climate region** | Tropical moist | Tropical moist | Paragraph 6.1, Commission decision |
| **Soil type** | Low activity Clay | Low activity Clay | Paragraph 6.2, Commission decision |
| **Soil management** | no till | Full tillage | Table 3, Commission decision |
| **Input** | high w/o manure | low | Table 3, Commission decision |
| **SOC_{ST}** | 47 tonne C/ha | 47 tonne C/ha | Table 1, Commission Decision, using climate region and soil type |
| **F_{LU}** | 0.48 tonne C/ha | 0.48 tonne C/ha | Table 2, Commission Decision |
| **F_{MG}** | 1.22 tonne C/ha | 1.0 tonne C/ha | Table 2, Commission Decision |
| **F_{I}** | 1.11 tonne C/ha | 0.92 tonne C/ha | Table 2, Commission Decision |
| **SOCᵢ *** | **30.6 tonne C/ha** | **20.8 tonne C/ha** | |
| **E_{sca} **** | **1.79 tonne of CO₂/ha/year** | | |
| **SOCᵢ* = *SOC_{ST} X F_{LU} X F_{MG} XF_{I}* | | | |
| ***E_{sca}* = *(SOC_{A}- SOCR) *3.664 20* | | | |

**Table 16: Emissions due to cultivation of carinata (Farm 1 from Example 1)**

| | **Allocation factor** | **Non-allocated results** | **Allocated results** | **Total** |
|---|---|---|---|---|
| | | *All results in* g *CO*_{2*eq*} / *MJ_{HVO}* | | |
| Cultivation of carinata | 61.3% | 50.47 | 30.91 | |
| Carinata drying | 61.3% | 0.99 | 0.61 | |
| **Cultivation E_{ec}** | | | | **31.5** |
| Transport of grain (truck) | 61.3% | 0.28 | 0.17 | |
| **Transport E_{td}** | | | | **0.17** |
| Bonus or E_{sca} | 100.0% | (67.3)* | (67.3)* | (67.3)* |
| **Totals** | | **(15.6)*** | | **(35.63)*** |

| | | | | |
|---|---|---|---|---|
| *numbers in parentheses are negative | | | | |

**Table 17: Emissions due to cultivation of carinata (Farm 2 from Example 1)**

| | **Allocation factor** | **Non-allocated results** | **Allocated results** | **Total** |
|---|---|---|---|---|
| Cultivation of carinata | 61.3% | 93.66 | 57.37 | |
| Carinata drying | 61.3% | 0.99 | 0.61 | |
| **Cultivation E_{ec}** | | | | **58.0** |
| Transport of grain (truck) | 61.3% | 0.48 | 0.29 | |
| **Transport E_{td}** | | | | **0.29** |
| Bonus or E_{sca} | 100.0% | (76.2)* | (76.2)* | (76.2*) |
| **Totals** | | **52.9*** | | **(17.64)*** |

| | | | | |
|---|---|---|---|---|
| *numbers in parentheses are negative | | | | |

**Example 7: GHG reduction associated with the entire HVO production pathway** using **carinata to produce feedstock when grown sequentially as winter cover following peanut in rotation.** This example demonstrates the reduction of GHG emission achieved over the entire HVO production pathway by using feedstock from carinata grown sequentially as cover crop in the tropical moist climate zone following cultivation of a leguminous crop (peanut). As described in the previous example, *Brassica carinata* was cultivated during winter 2015-2016 as a winter cover crop, replacing winter fallow, on two farms in the SE USA. The previous crop cultivated on both farms was peanut, a leguminous crop species. In a departure from common practice, the peanut residue was not incorporated by tilling post-harvest, but instead no-till management used to allow peanut crop residue to remain on the field.

In order to calculate the GHG footprint of carinata cultivation in these examples, the BioGrace v 1.4 model was used (http://www. Biograce.net), as previously described. Table 18 summarizes the relevant modules of the BioGrace V1.4 module which account for all relevant emission produced by the pathway for HVO biofuel production (well to tank). In the example described herein, and unlike the previous example which only considered cultivation emissions, all sources of emission listed above are considered.

For the cultivation phase, emissions due to seed and field preparation, direct and indirect emissions due to application of inputs, emissions due to use of farm equipment for seeding, applying inputs, harvesting, *etc.,* emissions derived from energy usage associated with drying of grain and emissions resulting from field release of N₂O are as described in the previous example (Example 6) and are summarized in Tables 13 and 14.

**Table 18: Sources of GHG emissions accounted for by BioGrace 1.4 model**

| | |
|---|---|
| **Cultivation emissions (E_{ec})** | Cultivation of carinata |
| | carinata drying |
| **Processing emissions (Eₚ)** | Oil extraction |
| | Oil refining |
| | Hydrogenation of carinata oil |
| **Transport emissions (E_{td})** | Transport of carinata grain |
| | Transport of carinata oil |
| | Transport of HVO to depot |
| | Transport to filling station |
| **Emissions due to Land use change (Eₗ)** | |
| **Emission reductions (Bonus or E_{sca})** | |

For the processing phases, which include oil extraction and processing into biofuel, the production of hydrotreated vegetable oil (HVO) has been chosen as the most likely end use of the carinata feedstock. In the crushing and oil extraction phase, electricity to run the expeller and crushing line equipment and natural gas boiler steam generation for heating are major sources of GHG emissions that are accounted for in the LCA model. Chemical used in the extraction of the oil from meal (such as hexane) and in the degumming and refining of the extracted oil (such as NaOH and phosphoric acid) also contribute to lifecycle GHG and are accounted for as well. For processing of the oil into HVO, electricity and natural gas steam generation are the major energy sources contributing to GHG emissions, as is the hydrogen used in the hydrotreatment process itself. Typically, default emissions values are used for these processing phases as they are well-established processes that do not vary significantly. Existing default values for rapeseed oil extraction and hydrotreating have been used for the carinata pathway as these are not expected to differ significantly whether applied to rapeseed or carinata. Emissions for the processing phase are summarized in Table 19. While different total amounts of oil are produced and processed, due to the difference in grain yields, since these emissions are normalized to the total amount of HVO produced from each farm's contribution, the normalized processing emissions are equal for each farm's grain output.

**Table 19: Emissions due to processing of carinata grain into low carbon intensity biofuel**

| | **Allocation factor** | **Non-allocated results** | **Allocated results** | **Total** |
|---|---|---|---|---|
| | | ***All results in g CO*_{2,*eq*} / *MJ_{HVO}*** | | |
| Extraction of oil | 61.3% | 6.41 | 3.92 | |
| Hydrogenation of veg. oil | 100.0% | 9.34 | 9.34 | |
| **Processing Eₚ** | | | | **13.3** |

For transport-related emissions, distances traveled between farm gate, nearest grain elevator, crush plant, HVO biofuel refinery and filling station are used to estimate transport fuel requirements. Electrical generation costs at storage facilities are based on feedstock and biofuel amounts that are in turn calculated from grain yields. In this particular example, grain cultivated from farms in Northern Florida and Southern Georgia was transported to one of three collection points and from there then shipped by truck to the Port of Tampa where the grain was pooled and loaded into holds of a sea freight carrier. The grain was transported by sea to Rouen, France and then by truck to Grande Currone for crushing. The vegetable oil was then transported by truck to Antwerp, Belgium for storage and then by truck to a refinery in Donges, France for conversion to fuel by HVO processing. The distances traveled, and fuel used during transport (summarized in Table 20), were used to determine emissions produced during the transport phases for both Farm 1 (Table 21) and Farm 2 (Table 22).

For the cultivation phase, transport of carinata grain to the crusher, oilseed crushing and oil extraction steps, an allocation factor is applied to the emissions to account for the fact that the carinata oil fraction comprises 63% of the energy of the seed and is the seed fraction that is uniquely processed into HVO. Thus, up until the point that the oil is processed into HVO, emissions are multiplied by the allocation factor (0.63) while in subsequent phases the emissions are considered to be 100% of calculated values.

**Table 20: Transportation of grain, oil and fuel**

| | **Means of transport** | **Fuel** | **Distance Farm 1 (km)** | **Distance Farm 2 (km)** |
|---|---|---|---|---|
| Grain to local Storage | Truck | Diesel | 45.05 | 80.5 |
| Grain from Storage to port 1 | Truck | Diesel | 800.17 | 505.44 |
| Grain from port 1 to port 2 | Cargo ship | Diesel | 8423 | |
| Grain to crushing plant | Truck | Diesel | 15 | |
| Oil to Refinery | Truck | Diesel | 1193 | |
| HVO to distribution center | Truck | Diesel | 150 | |
| HVO to filling station | Truck | Diesel | 150 | |

**Table 21: Emissions due to transport (Farm 1)**

| | **Allocation factor** | **Non-allocated results** | **Allocated Results** | **Total** |
|---|---|---|---|---|
| | | ***All results in g CO*_{2,*eq*} / *MJ_{HVO}*** | | |
| Transport of grain (truck) | 61.3% | 0.27 | 0.17 | |
| Transport of grain (truck) | 61.3% | 4.72 | 2.89 | |
| Transport of grain (sea) | 61.3% | 6.50 | 3.98 | |
| Transport of carinata oil | 100.0% | 0.07 | 0.07 | |
| Transport of carinata oil | 100.0% | 2.91 | 2.91 | |
| Transport of HVO to depot | 100.0% | 0.41 | 0.41 | |
| Transport to filling station | 100.0% | 0.74 | 0.74 | |
| **Transport E_{td}** | | | | **8.3** |

**Table 22: Emissions due to transport (Farm 2)**

| | **Allocation factor** | **Non-allocated results** | **Allocated Results** | **Total** |
|---|---|---|---|---|
| | | ***All results in g CO*_{2,}*_{eq} l MJ_{HVO}*** | | |
| Transport of carinata grain | | 0.48 | 0.29 | |
| Transport of carinata grain | 61.3% | 2.98 | 1.83 | |
| Transport of grain (sea) | 61.3% | 6.50 | 3.98 | |
| Transport of carinata oil | 61.3% | 0.07 | 0.07 | |
| Transport of carinata oil | 100.0% | 2.91 | 2.91 | |
| Transport of HVO to depot | 100.0% | 0.41 | 0.41 | |
| Transport to filling station | 100.0% | 0.74 | 0.74 | |
| **Transport E_{td}** | | | | **8.4** |

As described previously, indirect land use change can contribute significantly to GHG emissions of a biofuel pathway and is accounted for in the BioGrace model as a potential source of greenhouse gas (GHG) emissions, which can be added to the aforementioned phases. However, in the carinata cultivation method of the present invention, no indirect land use change occurs since the cultivation of carinata replaces a fallow period in the crop rotation and does not displace any other crop.

The BioGrace model considers one additional factor in the calculation of net GHG emission-*i*.*e*., the anticipated reductions in GHG emissions that may result in the case of improved land management practices being applied for cultivation of the bioenergy crop relative to the baseline situation. Emission reduction, termed E_{sca}, assumes that the improved land management practices result in increased carbon sequestration, thus offsetting a portion of the emissions produced during the cultivation, processing and transportation phases of the pathway. In the specific case of carinata cultivated as described herein, additional emission reductions are expected due to the change from full tillage to no tillage and replacement of fallow with a cover crop that returns a high proportion of its biomass back to the soil. The BioGrace model quantifies and assigns an E_{sca} value based on these improvements, which is then subtracted from the net emissions of the entire pathway (Table 23).

**Table 23: Sum of emissions associated with pathway (HVO biofuel production from carinata oil)**

| | **Total** | |
|---|---|---|
| ***All results in g CO*_{2,*eq*} / *MJ_{HVO}*** | **Farm 1** | **Farm 2** |
| Cultivation E_{ec} | 31.5 | 58.0 |
| Processing Eₚ | 13.3 | 13.3 |
| Transport E_{td} | 8.3 | 8.4 |
| Bonus or E_{sca} | (67.3)* | (76.2)* |
| **Totals** | **(14.2)*** | **3.5** |

| | | |
|---|---|---|
| *numbers in parentheses are negative | | |

Table 23 also summarizes the GHG emissions calculated to occur throughout the carinata oil to HVO pathways when carinata was produced on Farm 1 and Farm 2. Since both farms lie within the same soil and climate zones, are in geographical proximity to one another and the crop is being processed identically to the same endpoint, it follows that the processing and transportation phases will be very similar in terms of emissions. As can be seen, the only phase that that shows difference in emissions between the two is the cultivation phase and is reflective of the variation in the practices employed at each farm. These include differences in acres seeded, seeding rate, levels of input (particularly nitrogen based), energy used in cultivation and ultimately crop yield. Nevertheless, as shown in Table 24, the resulting lifecycle GHG emissions for the carinata to HVO pathway of -14.2 gCO₂ eq/MJ (Farm 1) and 3.5 gCO₂ eq/MJ (Farm 2) are both significantly lower than lifecycle emissions associated with the production pathway for petroleum derived diesel, at 83.8 gCO₂eq/MJ (WTT Appendix 1, v3 paragraph 2.1 & 3; Z1), providing between 96 and 117% reductions in GHG emissions with respect to diesel fuel.

**Table 24: Carbon Intensity (CI) and GHG Emission reductions relative to fossil fuel benchmark**

| | **Fossil fuel (diesel)** | **Biodiesel from feedstock produced from carinata grain** | |
|---|---|---|---|
| | | **Farm 1** | **Farm 2** |
| CI (g CO_{2eq}/MJ) | 83.8* | -14.2 | 3.5 |
| GHG reduction | -- | 117% | 96% |

| | | | |
|---|---|---|---|
| *DeJong *et al.,* 2017 | | | |

**Example 8: *Brassica carinata* sequentially grown after soybean as a winter cover; effect of manure usage on GHG emissions during cultivation.** *Brassica carinata* was cultivated sequentially during winter 2015-2016 as a winter cover crop, replacing fallow, on two farms in the SE USA (Farm A located near Fort Valley, GA and Farm B located near Dublin, GA). This region falls within a climate zone classified as warm temperate moist, as described above and in Table 2. The soils found in this region of Georgia fall into the general classification of low activity clay soil type (see 2010/335/EU; COMMISSION DECISION of 10 June 2010 on guidelines for the calculation of land carbon stocks for the purpose of Annex V to Directive 2009/28/EC). Fields of both farms were seeded with *Brassica carinata* Avanza 641 according to procedures outlined in the Agrisoma Growers Guide for the region (https://agrisoma.com/ckfinder/userfiles/files/2017_18_SE _Handbook.pdf); for specific details of cultivation see Table 25. The quantities of fertilizer inputs used in both farms are listed in Table 25 and were based on the results of soil analysis to determine amounts of added nutrients to achieve the recommended ranges suggested in the grower's guide. In the case of Farm A, all nitrogen was applied in the form of inorganic nitrogen fertilizer, while in the case of Farm B, a mixture of inorganic nitrogen fertilizer and manure was employed.

To accurately access the totality of GHG emissions associated with the cultivation of carinata, the farm managers recorded the fuel usage of all farm machinery used during the cultivation and harvest of the carinata crops (Table 26). All watering at both sites was achieved via combination of natural precipitation as well as supplemental irrigation. At maturity, the seed was harvested by straight cutting via combine and virtually all plant materials, aside from the collected grain, were returned to the field. Yields per unit area of harvested carinata grain (at specified moisture contents) from both farms are tabulated in Table 25.

**Table 25: Carinata cultivation details (Farm A and Farm B)**

| **Cultivation details** | | | |
|---|---|---|---|
| Site | | Fort Valley, GA | Dublin, GA |
| Previous Crop | | Soybean | Soybean |
| Carinata area seeded | ha | 35.64 | 17.82 |
| Seeding rate | kg/ha | 4.0 | 6.0 |
| Yield | kg/ ha⁻¹ year⁻¹ | 1912.14 | 1754.81 |
| Grain Moisture | | 8.2% | 9.3% |

| **sources used Energy during cultivation** | | | |
|---|---|---|---|
| | **Units** | **Farm A** | **Farm B** |
| Diesel usage | MJ ha⁻¹ year⁻¹ | 858.93 | 862.24 |

| **Agro-chemicals** | | | |
|---|---|---|---|
| N-fertilizer (kg N) | kg N ha⁻¹ year⁻¹ | 110.63 | 135.39 |
| Manure | kg N ha⁻¹ year⁻¹ | - | 87.63 |
| CaO- fertilizer (kg CaO) | kg CaO ha⁻¹ year⁻¹ | - | - |
| K₂O-fertiliser (kg K₂O) | kg K₂O ha⁻¹ year⁻¹ | - | - |
| P₂O₅- fertilizer (kg P₂O₅) | kg P₂O₅ ha⁻¹ year⁻¹ | - | - |
| Pesticides | kg ha⁻¹ year⁻¹ | 3.54 | 4.14 |

To calculate the GHG footprint of carinata cultivation with and without the use of manure, the BioGrace v 1.4 model was used (http://www. Biograce.net), as described in Example 6, using the same principles to account for GHG emissions for cultivation inputs and fuel use during cultivation and transport. For the purposes of this example, however, while the feedstock produced would be used primarily for production of HVO for use as drop in fuel replacement in transport and aviation fuel applications, the BioGrace model is only being used to consider the GHG emissions from the cultivation phase of a carinata based biofuel pathway, including harvest, drying and transport of the grain to a storage location, in order to demonstrate the potential for carinata cultivation to reduce the carbon intensity of an associated biofuel fuel pathway under conditions of cultivation where carinata is grown as winter cover replacing fallow on a farm within the warm, temperate moist climate zone as well as allowing assessment of the impact of manure used in the cultivation phase on carbon intensity of the resultant HVO biofuel. Nevertheless, in order to estimate GHG emissions consistently and in a way that respected the functional unit, the presumed yield of HVO for the abbreviated pathway was taken to be 0.58 MJ HVO/ MJ of carinata grain harvested. The allocation factor for the first three steps of carinata oil production (cultivation, drying and grain transportation) is 0.613, as described previously.

All emissions tabulated during the cultivation, drying and transport phase were added to give a total emission value (expressed as carbon intensity) for the cultivation phase (see Table 26 for cultivation phase emissions for Farm A and Farm B). As described previously, for the cultivation, drying and grain transport steps, an allocation factor is applied to the emissions to account for the fact that the carinata oil fraction comprises 63% of the energy of the seed and represents the fraction that is uniquely processed into HVO. Thus, up until the point that the carinata oil is extracted from the grain, the emissions produced are multiplied by said allocation factor, to yield so-called allocated emissions for each of the cultivation, drying and grain transport steps (see Table 27).

**Table 26: Emissions due to cultivation of carinata without (Farm A) or with (Farm B) manure use**

| | **Farm A CO_{2eq} emissions** | **Farm B CO_{2eq} emissions** |
|---|---|---|
| | ***All values in g CO_{2eq}*/*MJ HVO*** | |
| **Cultivation** | | |
| Diesel consumption | 2.77 | 3.07 |
| N fertilizer (inorganic) | 23.93 | 32.31 |
| Manure* | NA | 0 |
| Pesticides | 1.59 | 1.84 |
| Seeding material | 0.11 | 0.17 |
| N₂O field emissions | 34.13 | 66.48 |
| *Cultivation Subtotal* | 62.52 | *103.87* |
| Drying of grain | 0.70 | 0.71 |
| Transport of grain | 1.26 | 1.69 |

| | | |
|---|---|---|
| * Use of manure will not incur direct emissions due to manufacturing but will lead to indirect emissions (captured in calculation of N₂O field emissions) | | |

**Table 27: Emissions due to cultivation of carinata without (Farm A) or with (Farm B) manure use**

| | **Allocation factor** | **Farm A Non-allocated emissions** | **Farm A allocated emissions** | **Farm B Non-allocated emissions** | **Farm B allocated emissions** |
|---|---|---|---|---|---|
| | | ***All values in g CO_{2eq}*/*MJ HVO*** | | | |
| **Cultivation** | 61.3% | 62.52 | 38.29 | 103.87 | 63.62 |
| **Drying** | 61.3 % | 0.70 | 0.43 | 0.71 | 0.43 |
| **Transport** | 61.3% | 1.26 | 0.77 | 1.69 | 1.03 |
| ***Total*** | | | *39.49* | | *65.08* |

The BioGrace model considers one additional factor in the calculation of net GHG emission-*i*.*e*., the anticipated reductions in GHG emissions that may result from improved land management practices being applied during cultivation of the bioenergy crop relative to a baseline situation where management practices had not been modified. Emission reduction, termed E_{sca}, assumes that the improved land management practices result in increased carbon sequestration in the managed land, thus offsetting a portion of the emissions produced during the cultivation, processing and transportation phases of the pathway. In the specific case of carinata cultivated as described herein, additional emission reductions are expected due to the change from medium tillage to reduced tillage, replacement of fallow with a groundcover crop that returns a high proportion of its biomass back to the soil on harvest, as well as the use of manure which also contributes to soil carbon conservation. The BioGrace model quantifies and assigns an E_{sca} value in units of tonnes CO₂ returned to soil/ha/year based on these improvements (see E_{sca} values in Table 28 for Farm A and Table 29 for Farm B). This is subsequently converted to tonnes of CO₂ returned to soil/ MJ of HVO biofuel produced which serves to reduce the net emissions of the entire pathway.

**Table 28: Soil organic carbon changes due to improved land management (Farm A-no manure)**

| | **Actual land use** | **Reference land use** | **Reference** |
|---|---|---|---|
| **Climate region** | Warm temperate moist | Warm temperate moist | Paragraph 6.1, Commission decision |
| **Soil type** | Low activity Clay | Low activity Clay | Paragraph 6.2, Commission decision |
| **Soil management** | reduced till | Full tillage | Table 3, Commission decision |
| **Input** | high w/o manure | medium | Table 3, Commission decision |
| **SOC_{ST}** | 63 tonne C/ha | 63 tonne C/ha | Table 1, Commission Decision, using climate region and soil type |
| **F_{LU}** | 0.69 tonne C/ha | 0.69 tonne C/ha | Table 2, Commission Decision |
| **F_{MG}** | 1.08 tonne C/ha | 1.0 tonne C/ha | Table 2, Commission Decision |
| **F_{I}** | 1.11 tonne C/ha | 1.0 tonne C/ha | Table 2, Commission Decision |
| **SOCᵢ*** | **52.1 tonne C/ha** | **43.5 tonne C/ha** | |
| **E_{sca}**** | **1.583 tonne of CO₂/ha/year** | | |
| **Esca***** | **58.23 g CO_{2eq}/MJ_{HVO}** | | |
| **SOCᵢ* = *SOC_{ST} X F_{LU} X F_{MG} X F_{I}* | | | |
| ***E_{sca}* = (*SOC_{A}- SOC_{R}*) **3.664*/*20* | | | |
| **** g CO2eq*/*MJ of HVO produced = tonne of CO_{2eq}*/*ha*/*year X (10⁶ g*/*tonne )*/*MJ of HVO producedlhalyear, where MJ* of HVO *produced*/*ha*/*year = MJ rapeseed produced*/*ha*/*year X 55.5% (estimated pathway conversion efficiency)* | | | |

**Table 29: Soil organic carbon changes due to Improved land management (Farm B with manure)**

| | **Actual land use** | **Reference land use** | **Reference** |
|---|---|---|---|
| **Climate region** | Warm temperate moist | Warm temperate moist | Paragraph 6.1, Commission decision |
| **Soil type** | Low activity Clay | Low activity Clay | Paragraph 6.2, Commission decision |
| **Soil management** | Reduced till | Full tillage | Table 3, Commission decision |
| **Input** | High w/ manure | Medium | Table 3, Commission decision |
| **SOC_{ST}** | 63 tonne C/ha | 63 tonne C/ha | Table 1, Commission Decision, using climate region and soil type |
| **F_{LU}** | 0.69 tonne C/ha | 0.69 tonne C/ha | Table 2, Commission Decision |
| **F_{MG}** | 1.44 tonne C/ha | 1.0 tonne C/ha | Table 2, Commission Decision |
| **F_{I}** | 1.11 tonne C/ha | 1.0 tonne C/ha | Table 2, Commission Decision |
| **SOC; *** | **67.6 tonne C/ha** | **43.5 tonne C/ha** | |
| **E_{sca}**** | **4.421 tonne of CO₂/ha/year** | | |
| **E_{sca}***** | **179.43 g CO_{2eq}/MJ_{HVO}** | | |
| **SOCᵢ* = *SOC_{ST} X F_{LU} X F_{MG} X F_{I}* | | | |
| ***E_{sca}* = *(SOC_{A}- SOC_{R}) *3.664*/*20* | | | |
| **** g CO2eql MJ of HVO produced = tonne of CO_{2eq}*/*ha*/*year X (10⁶ g*/*tonne )*/*MJ of HVO produced*/*ha*/*year, where MJ* of HVO *produced*/*ha(year = MJ rapeseed produced*/*ha*/*year X 55.5% (estimated pathway conversion efficiency)* | | | |

As can be seen in Table 30, the CO_{2eq} emissions per MJ of HVO produced during the cultivation, drying and grain shipment phases for both farms are negative *(i.e.,* -18.7 tonnes of CO_{2eq}/ MJ of HVO produced for Farm A and -114.35 tonnes of CO_{2eq}/ MJ of HVO produced for Farm B), indicating that cultivation of carinata under conditions and practices described herein resulted in net reductions of atmospheric CO_{2eq}, respectively, of , substantially by increasing the levels of soil organic carbon due to net incorporation of carbon containing harvest residues, leaf litter and root material, reducing soil carbon loss by employing reduced tillage and, in the case of Farm B, by improving soil structure and carbon retention by use of manure.

**Table 30: Sum of emissions associated with pathway (HVO production from carinata feedstock) without (Farm A) or with (Farm B) manure**

| | | **Farm A emissions Allocated results** | **Farm B emissions Allocated results** |
|---|---|---|---|
| | | *All results in g* CO_{2 *eq*} / *MJ_{HVO}* | |
| **Cultivation** | | 38.29 | 63.62 |
| | **Plus Drying** | 0.43 | 0.43 |
| | **Plus Transport** | 0.77 | 1.03 |
| | **Less E_{sca}** | (58.23)* | (179.43)* |
| **Totals** | | **(18.74)*** | **(114.35)*** |

| | | | |
|---|---|---|---|
| *numbers in parentheses are negative | | | |

As can be seen in Table 26, the use of manure in Farm B entails a significant increase in CO_{2eq} emissions associated with direct and indirect N₂O emissions. Indeed, in considering only the contribution of the cultivation, drying and grain transportation steps, Farm 2 demonstrates 1.65-fold higher level of CO₂ eq emissions over those of Farm A. However, this is more than compensated for by the 2.8-fold increase in yearly soil carbon deposits observed for Farm B over Farm A (see Tables 28 and 29). Thus, in instances where a farmer may wish to cultivate carinata in soils with relatively low fertility (particular as far as nitrogen levels are concerned), it is clearly advantageous for the farmer to employ manure as a substitute for inorganic fertilizers (especially inorganic nitrogen) to achieve required fertility levels and maximal carinata yields, as one can offset and further reduce the levels of GHG emissions due to the beneficial effects of manure application on soil carbon accumulation.

While the current analysis only considers a portion of the HVO biofuel pathway, it is clear to those skilled in the art, that applying the optimal practices described herein to the cultivation of carinata to produce a feedstock for production of HVO and low carbon intensity biofuels would allow for the significant reductions accrued during the carinata cultivation, drying and grain transport phases to be applied against the emissions generated during the later phases of the pathway (*i.e.,* oil extraction, transport and storage of the feedstock oil, conversion of feedstock to HVO, HVO transport storage and distribution). Indeed, if practices described for Farm B are applied to the HVO production pathway described in Example 7, a net *negative* carbon intensity for the entire HVO production pathway would readily be achieved. The more negative the carbon intensity for the cultivation, drying and grain transportation phases can be made, the greater the range of transport options for feedstock and HVO may be considered while still minimizing overall GHG emissions of the biofuel pathway.

**Example 9: GHG reduction potential for low CI biofuel made from feedstock produced from sequential cultivation of *Brassica carinata* as winter cover following cereal (corn) in tropical moist climate (Florida/SE USA).** This example demonstrates the reduction of GHG emission achieved during cultivation of carinata sequentially grown as cover crop in the tropical moist climate zone, as exemplified by the production of carinata after cereal in North Florida (described previously in Example 2). As in previous examples, emissions due to cultivation of carinata as a winter cover crop after corn in the tropical moist climate zone were calculated using the BioGrace model, assuming HVO as end product and are summarized in Table 31 as g CO_{2eq}/MJ of HVO produced. The emissions due to the cultivation and harvest, drying and transport of the grain have been tabulated before and after application of an allocation factor which is used to account for the fact that only the oil portion of the grain contributes to the GHG emissions in this portion of the biofuel pathway. As can be seen, after application of the allocation factor, the total emissions from cultivation, drying and transportation of the grain was found to be 47.9 g CO_{2eq}/MJ of HVO produced.

**Table 31: Emissions due to cultivation of carinata**

| | **Allocation factor** | **Non-allocated results** | **Allocated results** | **Total** |
|---|---|---|---|---|
| | | *All results in* g CO_{2*eq*} / *MJ_{HVO}* | | |
| Cultivation of carinata | 61.3% | 77.18 | 47.32 | |
| Carinata drying | 61.3% | 0.97 | 0.59 | |
| **Cultivation E_{ec}** | | | | **47.9** |
| Transport of grain (truck) | 61.3% | 0.20 | 0.12 | |
| **Transport E_{td}** | | | | **0.12** |

Table 32 summarizes the benefits that can accrue because of the adoption of a corn and carinata rotation and the associated improvement of land management practices. The BioGrace model compares soil carbon accumulation before and after application of the new agricultural practice. In the baseline situation, the land is maintained tilled lightly then maintained under fallow conditions while receiving low levels of inputs, whereas in the modified situation, a carinata cover crop is cultivated under no till conditions and high levels of inputs. The net result of this change in practice is significant yearly net contribution of carbon to the existing soil stocks, due to the return of accumulated carbon from plant residues and root material remaining after harvest. The BioGrace model predicts a net increase soil carbon expressed as 1.02 tonnes of CO₂/ha/year due to carinata cultivation under improved land management practices over the baseline. Since the carbon is predominantly plant-derived via photosynthetic fixation of atmospheric CO₂, this represent a net removal of CO₂ from the atmosphere and sequestered in the soil. The net GHG emission reduction can also be expressed relative the amount of HVO produced, 36.59 g CO_{2eq}/MJ HVO, and this bonus, or E_{sca} value can be used to offset the GHG emissions that are produced during the entire course of the biofuel pathway. This is shown in Table 33 where the E_{sca} values are subtracted from the net emission accumulated from cultivation, drying and transport of the grain. As can be seen a net GHG emission of 11.42 g of CO₂eq/J HVO is produced after subtraction of the E_{sca} factor. Unlike some of the above examples of carinata cultivation, carbon intensity of the pathway comprising the cultivation of carinata as winter fallow after corn in the tropical moist climate zone, drying and transport of harvested grain to collection points remains positive even after subtraction of the E_{sca} bonus, indicating that net GHG emissions are being released. This is due in part to the high levels of nitrogen used in the cultivation of carinata in this study and associated contribution to field emissions of GHG during the cultivation phase.

If, however, nitrogen input could be reduced by 50% *(i.e.,* from 141 kg/ha to 70 kg/ha) without significantly impacting carinata yields (low nitrogen utilization), GHG emissions during cultivation phase could be reduced from 47.9 g CO_{2eq}/MJ HVO to 30.1 g CO_{2eq}/MJ HVO (Table 33) due to reduced lifecycle emissions associated with the manufacturing of the nitrogen fertilizer as well as reduction of field emissions. When transportation and E_{sca} are factored in, emissions become negative (-6.4 g CO_{2eq}/MJ HVO) indicating a net reduction of atmospheric CO₂ levels as a result of carinata cultivation, which can be used to offset emissions for other phases of the biofuel pathway. This example illustrates how maximizing the nitrogen use efficiency of carinata cultivation can significantly impact reduction of GHG emissions associated with low CI biofuel manufacturing.

**Table 32: Emission reduction due to Improved land management**

| | **Actual land use** | **Reference land use** | **Reference** |
|---|---|---|---|
| **Climate region** | Tropical moist | Tropical moist | Paragraph 6.1, Commission decision |
| **Soil type** | sandy | sandy | Paragraph 6.2, Commission decision |
| **Soil management** | no till | reduced till | Table 3, Commission decision |
| **Input** | high w/o manure | low | Table 3, Commission decision |
| **SOC_{ST}** | 39 tonne C/ha | 39 tonne C/ha | Table 1, Commission Decision, using climate region and soil type |
| **F_{LU}** | 0.48 tonne C/ha | 0.48 tonne C/ha | Table 2, Commission Decision |
| **F_{MG}** | 1.22 tonne C/ha | 1.15 tonne C/ha | Table 2, Commission Decision |
| **Fi** | 1.11 tonne C/ha | 0.92 tonne C/ha | Table 2, Commission Decision |
| **SOCᵢ *** | **25.4 tonne C/ha** | **19.8 tonne C/ha** | |
| **E_{sca} **** | **1.02 tonne of CO₂/ha/year** | | |
| **E_{sca} ***** | **36.59 g CO_{2,eq}/MJ HVO** | | |
| *SOCᵢ = SOC_{ST} * F_{LU} * F_{MG} * F_{I} | | | |
| **E_{sca}= (SOC_{A}- *SOC_{R})* *3.664/20 | | | |
| *** g CO_{2,eq/} MJ of HVO produced = tonne of CO_{2.eq}/ha/year *(10⁶ g/ton)/MJ of HVO produced/ha/year, where MJ of HVO produced/ha/year = MJ rapeseed produced/ha/year *55.5% (estimated pathway conversion *efficiency)* | | | |

**Table 33: Emissions due to cultivation of carinata**

| | **Totals** | |
|---|---|---|
| ***All results in g* CO_{2*,eq*} / *MJ_{HVO}*** | **110 kg/ha inorganic N** | **55 kg/ha inorganic N** |
| Cultivation E_{ec} | 47.9 | 30.1 |
| Transport E_{td} | 0.12 | 0.12 |
| Bonus or E_{sca} | (36.6)* | (36.6)* |
| **Totals** | **11.42** | **(6.4)*** |

| | | |
|---|---|---|
| *numbers in parentheses are negative | | |

**Example 10: GHG emissions due to cultivation of *Brassica carinata* as summer cover following legume (lentil) in cool temperate dry climate.** This example demonstrates the reduction of GHG emission achieved using carinata as a summer cover crop cultivated for production of feedstock for biofuel manufacturing. As in previous examples, emissions due to cultivation of carinata as a summer cover crop after lentils in the cool temperate, dry climate zone are calculated using the BioGrace model, assuming HVO as end product, as described previously, and summarized in Table 34 (inorganic N usage scenario 1 with 110 kg/ha) and Table 35 (inorganic N usage scenario 2 with 55 kg/ha) as g CO_{2eq}/MJ of HVO produced. The emissions due to the cultivation and harvest of the grain, drying and transport of the grain are tabulated before and after application of an allocation factor which is used to account for the fact that only the oil portion of the grain is contributing the GHG emissions in this portion of the biofuel pathway. As can be seen by comparing the data in Tables 34 and 35, GHG emissions for drying and transport of the grain are identical for both nitrogen use scenarios but differ substantially for the cultivation phase, with scenario 2 showing much lower emissions predicted by the BioGrace model. This reflects the lower field emissions resulting from lower amounts of nitrogen fertilizer being applied to the crop. Thus, the lower requirement for nitrogen-based fertilizer coupled with the ability to maintain yields is an anticipated benefit of the use of lentils and other leguminous crop species in rotations with carinata and offers additional benefits in the form of significantly reducing emissions of greenhouse gases during cultivation.

**Table 34: Emissions due to cultivation of carinata (Scenario 1)**

| | **Allocation factor** | **Non-allocated results** | **Allocated Results** | **Total** |
|---|---|---|---|---|
| | | ***All results in g* CO_{2*eq*} / *MJ_{HVO}*** | | |
| Cultivation of carinata | 61.3% | 61.1 | 37.4 | |
| Carinata drying | 61.3% | 1.02 | 0.62 | |
| **Cultivation E_{ec}** | | | | **38.1** |
| Transport of grain (truck) | 61.3% | 0.61 | 0.37 | |
| **Transport E_{td}** | | | | **0.37** |

**Table 35: Emissions due to cultivation of carinata (Scenario 2)**

| | **Allocation factor** | **Non-allocated results** | **Allocated Results** | **Total** |
|---|---|---|---|---|
| | | ***All results in g* CO_{2eq} *l MJ_{HVO}*** | | |
| Cultivation of carinata | 61.3% | 40.4 | 24.8 | |
| Carinata drying | 61.3% | 1.02 | 0.62 | |
| **Cultivation E_{ec}** | | | | **25.4** |
| Transport of grain (truck) | 61.3% | 0.61 | 0.37 | |
| **Transport E_{td}** | | | | **0.37** |

Table 36 summarizes the benefits that can accrue as a consequence of the adoption of a lentil/carinata rotation and the associated improvement of land management practices. The BioGrace model compares soil carbon accumulation before and after application of the new agricultural practice. In the baseline situation, the land is allowed to remain fallow and received low levels of inputs while in the modified situation, a carinata cover crop is cultivated. While this entails application of more inputs, the net result of the cultivation of carinata is significant yearly net contribution of carbon to the existing soil stocks, due to the return of accumulated carbon from plant residues and root material returned after harvest. The BioGrace model predicts a net increase soil carbon expressed as 0.73 tonnes of CO₂ /ha/year due to carinata cultivation, over the baseline. Since the carbon is predominantly plant derived via photosynthetic fixation of atmospheric CO₂, this represent a net removal of CO₂ from the atmosphere and sequestered in the soil. The net GHG emission reduction can also be expressed relative the amount of HVO produced, 30.32 g CO_{2eq}/MJ HVO, and this bonus of E_{sca} value can be used to offset the GHG emissions that are produced during the course of the pathway. This is shown in Table 37, where the E_{sca} values are subtracted from the net emission accumulated from cultivation, drying and transport of the grain. As can be seen in scenario 1 (high nitrogen utilization), a net GHG emission of 8.2 g of CO_{2eq}/MJ HVO is produced after addition of the E_{sca} factor; however, in scenario 2 (low nitrogen utilization), a net GHG reduction of 4.5 g CO_{2eq}/ MJ HVO is obtained. This negative carbon intensity can be used to offset emissions that can occur in other phases of the HVO production pathway, such as processing, refining and hydrotreating of the carinata oil, helping to reduce the overall emissions of the pathway. Thus, increasing the nitrogen use efficiency of the cultivation phase and improving the land management practices associated with cultivation can result in increasingly negative carbon intensity for this pathway phase and can significantly reduce overall GHG emissions of the entire biofuel pathway.

**Table 36: Improved land management ( inorganic nitrogen Scenario 1 and 2)**

| | **Actual land use** | **Reference land use** | **Reference** |
|---|---|---|---|
| **Climate region** | Cool temperate dry | Cool temperate dry | Paragraph 6.1, Commission decision |
| **Soil type** | High activity clay | High activity clay | Paragraph 6.2, Commission decision |
| **Soil management** | no till | no till | Table 3, Commission decision |
| **Input** | high w/o manure | low | Table 3, Commission decision |
| **SOC_{ST}** | 50 tonne C/ha | 50 tonne C/ha | Table 1, Commission Decision, using climate region and soil type |
| **F_{LU}** | 0.8 tonne C/ha | 0.8 tonne C/ha | Table 2, Commission Decision |
| **F_{MG}** | 1.1 tonne C/ha | 1.1 tonne C/ha | Table 2, Commission Decision |
| **F_{I}** | 1.1 tonne C/ha | 0.95 tonne C/ha | Table 2, Commission Decision |
| **SOCᵢ *** | **45.8 tonne C/ha** | **41.2 tonne C/ha** | |
| E_{sca} ** | **0.73 tonne of CO₂/ha/year** | | |
| E_{sca} *** | **30.32 g CO_{2eq}/MJ HVO** | | |
| **SOCᵢ* = *SOC_{ST} X F_{LU} X F_{MG} X F_{I}* | | | |
| * **E_{sca} = (SOC_{A}- SOC_{R}) X 3. 664*/*20* | | | |
| *** *g* CO_{2*,eq*/}*MJ of HVO produced* = *tonne of* CO_{2*,eq*}/*ha*/*year X (10⁶ g*/*ton)*/*MJ of HVO produced*/*ha*/*year, where MJ of HVO produced*/*ha*/*year* = *MJ rapeseed produced*/*ha*/*year X 55.5% (estimated overall pathway conversion efficiency)* | | | |

**Table 37: Emissions due to cultivation of carinata with high (110 kg/ha) inorganic N and low inorganic N (55 kg/ha)**

| | **Totals** | |
|---|---|---|
| ***All results in g CO_{2eq} l MJ_{HVO}*** | **High N** | **Low N** |
| Cultivation E_{ec} | 38.1 | 25.4 |
| Transport E_{td} | 0.37 | 0.37 |
| Bonus or E_{sca} | (30.32)* | (30.32)* |
| **Totals** | **8.2** | **(4.5)*** |

| | | |
|---|---|---|
| *numbers in parentheses are negative | | |

**Example 11: GHG emission reduction due to sequential cultivation of *Brassica carinata* as winter cover following Legume (soybean) in warm temperate, moist climate (Uruguay).** This example demonstrates the reduction of GHG emission achieved using carinata as a winter cover crop, replacing fallow, cultivated for production of feedstock for biofuel manufacturing. Emissions due to cultivation of carinata as a sequential winter cover crop after soybeans in the warm temperate, moist climate zone were then calculated using the BioGrace model, assuming HVO as end product, as described previously, and summarized in Table 38 as g CO_{2eq/}MJ of HVO produced. As previously described, the emissions due to the cultivation and harvest, drying and transport of the grain were tabulated before and after application of an allocation factor which is used to account for the fact that only the oil portion of the grain is contributing the GHG emissions in this portion of the biofuel pathway. As can be seen, after application of the allocation factor, the total emissions from cultivation, drying and transportation of the grain was found to be 27.1 g CO_{2eq}/MJ of HVO produced.

**Table 38: Emissions due to cultivation of carinata**

| | **Allocation factor** | **Non-allocated results** | **Allocated Results** | **Total** |
|---|---|---|---|---|
| | | ***All results in g* CO_{2*eq*} / *MJ_{HVO}*** | | |
| Cultivation of carinata | 61.3% | 41.49 | 25.44 | |
| Carinata drying | 61.3% | 0.55 | 0.34 | |
| **Cultivation E_{ec}** | | | | **25.8** |
| Transport of grain (truck) | 61.3% | 2.19 | 1.34 | |
| **Transport E_{td}** | | | | **1.3** |

Table 39 summarizes the benefits that can accrue as a consequence of the adoption of a soybean/carinata rotation and the associated improvement of land management practices. The BioGrace model compares soil carbon accumulation before and after application of the new agricultural practice. In the baseline situation, the land was allowed to remain fallow and received low levels of inputs while in the modified situation, a carinata cover crop is cultivated and higher levels of inputs were applied. While this entails application of more inputs, the net result of the cultivation of carinata is significant yearly net contribution of carbon to the existing soil stocks, due to the return of accumulated carbon from plant residues and root material returned after harvest. The BioGrace model predicted a net increase in soil carbon expressed as 1.41 tonnes of CO₂ /ha/year due to carinata cultivation, over that of the baseline scenario. Since the carbon is predominantly plant derived via photosynthetic fixation of atmospheric CO₂, this would represent a net removal of CO₂ from the atmosphere, sequestered in the soil. The net GHG emission reduction was also expressed relative the amount of HVO produced, 50.91 g CO_{2eq}/MJ HVO, and this bonus or E_{sca} value was then used in the BioGrace model to offset the GHG emissions produced during the course of the pathway. This is shown in Table 39 where the E_{sca} value has been subtracted from the net emission accumulated from cultivation, drying and transport of the grain resulting in a negative GHG emission of 23.8 g CO_{2eq}/ MJ HVO. In other words, under the conditions of cultivation used in this study, cultivation of *Brassica carinata* to produce oilseed grain can reduce the levels of atmospheric CO₂. This negative carbon intensity can be used to offset emissions that can occur in other phases of the HVO production pathway, such as processing, refining and hydrotreating of the carinata oil, helping to reduce the overall emissions of the pathway. Contributing to the negative carbon intensity of the cultivation phase are factors such as nitrogen use efficiency and improved land management practices associated with cultivation of carinata.

**Table 39: Improved land management**

| | **Actual land use** | **Reference land use** | **Reference** |
|---|---|---|---|
| **Climate region** | Warm temperate moist | Warm temperate moist | Paragraph 6.1, Commission decision |
| **Soil type** | High activity clay | High activity clay | Paragraph 6.2, Commission decision |
| **Soil management** | no till | no till | Table 3, Commission decision |
| **Input** | high w/o manure | medium | Table 3, Commission decision |
| **SOC_{ST}** | 88 tonne C/ha | 88 tonne C/ha | Table 1, Commission Decision, using climate region and soil type |
| **F_{LU}** | 0.69 tonne C/ha | 0.69 tonne C/ha | Table 2, Commission Decision |
| **F_{MG}** | 1.15 tonne C/ha | 1.15 tonne C/ha | Table 2, Commission Decision |
| **Fi** | 1.11 tonne C/ha | 1.0 tonne C/ha | Table 2, Commission Decision |
| **SOC; *** | **77.5 tonne C/ha** | **69.8 tonne C/ha** | |
| **E_{sca} **** | **1.41 tonne of CO₂/ha/year** | | |
| **E_{sca}***** | **50.91 g CO_{2eq}/MJ HVO** | | |
| **SOCᵢ* = *SOC_{ST} X F_{LU} X F_{MG} X F_{I}* | | | |
| ***E_{sca}* = *(SOC_{A}- SOC_{R}) *3.664*/*20* | | | |
| *** *g CO2eq*/*MJ of HVO produced* = *tonne of CO_{2eq}*/*ha*/*year X (10⁶ g*/*tonne )*/*MJ of HVO producedlhalyear, where MJ of HVO produced*/*ha*/*year* = *MJ rapeseed produced*/*ha*/*year X 55.5% (estimated pathway conversion efficiency)* | | | |

| **Table 40: Emissions due to cultivation of carinata** | | | |
|---|---|---|---|
| ***All results in g* CO₂*_{eq} l MJ_{HVO}*** | **Total** | | |
| Cultivation E_{ec} | 25.8 | | |
| Transport E_{td} | 1.3 | | |
| Bonus or E_{sca} | (50.91)* | | |
| **Totals** | **(23.81)*** | | |

| | | | |
|---|---|---|---|
| *numbers in parentheses are negative | | | |

**Example 12: GHG reductions as a result of sequential cultivation of *Brassica carinata* as winter cover crop following cereal (wheat) in New South Wales.** This example demonstrates the reduction of GHG emission achieved using carinata as cover crop in the warm temperate dry and tropical dry climate zones, as typified by the wheat belt of New South Wales of eastern Australia. As in previous examples, emissions due to sequential cultivation of carinata as a winter cover crop after wheat in the warm temperate climate zone are calculated using the BioGrace model, assuming HVO as end product, and are summarized in Table 41 (for high inorganic N use) and Table 42 (for low inorganic N use) as g CO_{2eq}/MJ of HVO produced. The emissions due to the cultivation and harvest of the grain, drying and transport of the grain are tabulated before and after application of an allocation factor which is used to account for the fact that only the oil portion of the grain contributes to the GHG emissions in this portion of the biofuel pathway. As can be seen, after application of the allocation factor, the total emissions from cultivation, drying and transportation of the grain is found to be 38.8 g CO_{2 eq} /MJ of HVO produced in the scenario where high amounts of inorganic nitrogen fertilizer is used during carinata cultivation and 25.5 g CO_{2eq} /MJ of HVO produced in the case of low inorganic nitrogen fertilizer usage.

**Table 41: Emissions due to cultivation of carinata (high inorganic N)**

| | **Non-allocated results** | **Allocation factor** | **Allocated results** | **Total** |
|---|---|---|---|---|
| | ***All results in g* CO_{2*eq*} / *MJ_{HVO}*** | | | |
| Cultivation of carinata | 59.71 | 61.3% | 36.57 | |
| Carinata drying | 1.57 | 61.3% | 0.96 | |
| **Cultivation E_{ec}** | | | | **37.5** |
| Transport of grain (truck) | 2.19 | 61.3% | 1.34 | |
| **Transport E_{td}** | | | | **1.3** |

**Table 42: Emissions due to cultivation of carinata (low inorganic N)**

| | **Non-allocated results** | **Allocation factor** | **Allocated results** | **Total** |
|---|---|---|---|---|
| | ***All results in g* CO_{2*,q*} / *MJ_{HVO}*** | | | |
| Cultivation of carinata | 38.01 | 61.3% | 23.28 | |
| Carinata drying | 1.57 | 61.3% | 0.96 | |
| **Cultivation E_{ec}** | | | | **24.2** |
| Transport of grain (truck) | 2.19 | 61.3% | 1.34 | |
| **Transport E_{td}** | | | | **1.3** |

Table 43 summarizes the benefits that can accrue as a consequence of the adoption of a wheat-carinata rotation and the associated improvement of land management practices under the specified climate zone and soil types. The BioGrace model compares soil carbon accumulation before and after application of the new agricultural practice. In the baseline situation, the land is maintained tilled lightly then maintained under fallow conditions while receiving low levels of inputs, whereas in the modified situation, a carinata cover crop is cultivated under no till conditions and high levels of inputs. The net result of this change in practice is significant yearly net contribution of carbon to the existing soil stocks, due to the return of accumulated carbon from plant residues and root material remaining after harvest.

The BioGrace model predicts a net increase soil carbon expressed as 0.97 tonnes of CO₂/ha/year due to carinata cultivation under improved land management practices over the baseline. Since the carbon is predominantly plant derived via photosynthetic fixation of atmospheric CO₂, this represent a net removal of CO₂ from the atmosphere and sequestered in the soil. The net GHG emission reduction can also be expressed relative the amount of HVO produced, 35 g CO_{2eq}/MJ HVO, and this bonus, or E_{sca} value can be used to offset the GHG emissions that are produced during the entire course of the biofuel pathway. This is shown in Table 44 where the E_{sca} values are subtracted from the net emission accumulated from cultivation, drying and transport of the grain. As can be seen in the case of cultivation with high inorganic nitrogen a net GHG emission of 3.8 g of CO_{2 eq} /J HVO is produced after subtraction of the E_{sca} factor. Unlike other examples of carinata cultivation, carbon intensity of the pathway comprising the cultivation of carinata as winter fallow after wheat in the warm temperate dry/tropical dry zone encompassing NSW, followed by drying and transport of harvested grain to collection points remains positive even after subtraction of the E_{sca} bonus, indicating that net GHG emissions are being released. This is due in part to the high levels of nitrogen used in the cultivation of carinata in this study contribution to field emissions of GHG during the cultivation phase.

**Table 43: Emission reduction due to improved land management**

| | **Actual land use** | **Reference land use** | **Reference** |
|---|---|---|---|
| **Climate region** | Warm Temp. Dry/ Tropical dry | | Paragraph 6.1, Commission decision |
| **Soil type** | High activity clay | | Paragraph 6.2, Commission decision |
| **Soil management** | no till | reduced till | Table 3, Commission decision |
| **Input** | high w/o manure | low | Table 3, Commission decision |
| **SOC_{ST}** | 38 tonne C/ha | 38 tonne C/ha | Table 1, Commission Decision, using climate region and soil type |
| **F_{LU}** | 0.8 tonne C/ha | 0.8 tonne C/ha | Table 2, Commission Decision |
| **F_{MG}** | 1.1 tonne C/ha | 1.1 tonne C/ha | Table 2, Commission Decision |
| **F_{I}** | 1.04 tonne C/ha | 0.95 tonne C/ha | Table 2, Commission Decision |
| **SOCᵢ *** | **34.8 tonne C/ha** | **29.5 tonne C/ha** | |
| **E_{sca} * *** | **0.97 tonne of CO₂/ha/year** | | |
| **E_{sca}***** | 35 **g CO_{2,eq}/MJ HVO** | | |
| **SOCᵢ* = *SOC_{ST}* * *F_{LU}* * *F_{MG}* * *F_{I}* | | | |
| ***E_{sca}* = *(SOC_{A}- SOC_{R}) *3.664*/*20* | | | |
| *** g C O_{2, *eq*/}*MJ of HVO produced* = *tonne of* CO_{2*,eq*}/*ha*/*year* **(10⁶ g*/*ton)*/*MJ of HVO produced*/*ha*/*year, where MJ of HVO produced*/*ha*/*year* = *MJ rape seed produced*/*ha*/*year *55.5%* (*estimated pathway conversion efficiency*) | | | |

If nitrogen input could be reduced by 50% (*i.e.,* from 110 kg/ha to 55 kg/ha) without significantly impacting carinata yields (low nitrogen utilization), GHG emissions during cultivation phase could be reduced from 37.5 g CO_{2eq}/MJ HVO to 24.2 g CO_{2eq}/MJ HVO (Table 44) due to reduced lifecycle emissions associated with the manufacturing of the nitrogen fertilizer as well as reduction of field emissions. When transportation and E_{sca} are factored in, overall emissions in the low nitrogen scenario are reduced to -9.5 CO_{2eq}/MJ HVO, representing a net decrease of atmospheric CO₂ levels as a result of carinata cultivation under these conditions. This example and the previous example serve to illustrate the effect that that differences in soil type and climate region can exert on the capacity of carinata cultivation and associated optimal practices to reduce greenhouse gas emissions.

**Table 44: Emissions due to cultivation of carinata**

| | **Totals** | |
|---|---|---|
| ***All results in g* CO_{2*eq*} / *MJ_{HVO}*** | **High inorganic N** | **Low Inorganic N** |
| Cultivation E_{ec} | 37.5 | 24.2 |
| Transport E_{td} | 1.3 | 1.3 |
| Bonus or E_{sca} | (35)* | (35)* |
| Totals | 3.8 | (9.5)* |

| | | |
|---|---|---|
| *numbers in parentheses are negative | | |

**Example 13: Effect of manure use on GHG emissions and sequestration during cultivation of *Brassica carinata.*** During winter of 2016-2017, *Brassica carinata* was cultivate on 13 independent farms located in the central portion of Georgia USA. In order to assess adherence to sustainable practices, the production of carinata on these farms was carefully audited. Data obtained on energy use and GHG emissions for all steps of the cultivation process were analyzed by use of the BioGrace GHG Biofuel GHG emissions calculator spread sheet, version 4d, as described in previous Examples. Of particular interest was to assess the effects of the use of manure (in this case chicken litter) on GHG emission levels from carinata cultivation, when used as a partial or complete replacement for inorganic nitrogen. Six of the 13 farms employed manure as fertilizer in combination with inorganic nitrogen, or in one case, as a complete replacement for inorganic nitrogen, while the remainder employed inorganic nitrogen exclusively in their fertilizer blends. Cultivation on all farms included the use of improved land management practices described herein, including reduced tillage and use of carinata as a cover crop in rotation with cereal crop, leguminous crop, cotton, or sesame.

Table 45 summarizes the data obtained from these farms. For the purposes of this study, it was assumed that the carinata grain produced would provide feedstock for production of HVO biodiesel and so intermediate GHG calculations for the pathway were normalized with respect to the energy content of HVO biodiesel, as previously described. CO₂ equivalent emissions were calculated from the cultivation data and comprised emissions from the following steps: manufacturing of inputs, farm machinery fuel use, production of commercial seed used to initiate the cultivation, drying of the seed, and transport of the seed. Direct and indirect emissions from organic and inorganic nitrogen applied to the field were also quantified and included. As a result of improved land and cultivation management practices, some portion of the CO₂ emissions were prevented from being released into the atmosphere and instead were incorporated into the soil organic carbon pool, thus reducing the net emission. This latter effect, known as Esca, can be quantified as described previously and is then subtracted from the CO_{2eq} produced by the aforementioned sources to generate a net cultivation emission for each farm. As can be seen in Table 45, all farms produced negative emissions for the cultivation phase of the pathway, indicating that the cultivation of carinata using the methods described herein effected a net removal of atmospheric CO₂. Farms employing manure as a nutrient source achieved a higher reduction of atmospheric CO₂ than those using only inorganic nutrients. One reason for this can be seen in the effect of manure use on soil carbon accumulation, where farms that employed manure demonstrated a several fold higher level of soil carbon accumulations than farms employing only inorganic nitrogen.

In the study describe herein, data was not directly obtained from latter stages of the HVO production pathway. However, once the grain is consolidated, it may be considered that the subsequent steps of the pathway would be common for all the sources of grain. Emissions associated with the energy used in extraction of oil and conversion of the oil feedstock to HVO biodiesel is well understood and is primarily a function of the amount of feedstock used. While distances and modalities of transport, distribution and storage of feedstock and finished fuel may be quite variable, for the purposes of this example default distances and type of transport fuel were employed to provide data to compute the net emissions for such an sample pathway and these were added to the aforementioned actual cultivation phase emission data previously described to obtain an over all carbon intensity for HVO produced through agricultural production of carinata and carinata feedstock. As can be seen in Table 45, in most cases the carbon intensity of the HVO produced by this default pathway is negative, indicating a net reduction in atmospheric GHG levels relative to production of diesel from a fossil fuel feedstock. The highest GHG reduction benefit is achieved from feedstock obtained from farms using manure in the cultivation phase.

It is clear that the greater one is able to reduce CO_{2eq} emissions in cultivation phase, through the improved practices described herein, including use of reduced or no-till practices, reduced irrigations, as well as the use of manure, the more one can offset emissions arising from the subsequent non-cultivation phases of the pathway that are more dependent on the variable factors of distances and modalities of transport, distribution and storage of feedstock and finished fuel.

**Table 45: Impact of use of manure on CO2Eq emissions due to Carinata cultivation and on CI of HVO diesel produced using carinata feedstock**

| | **Inputs** | | | **Cultivation** | | **CI for conversion of feedstock to HVO** | |
|---|---|---|---|---|---|---|---|
| **Farms¹** | **Manure nitrogen (kg/ha)** | **inorganic nitrogen (kg/ha)** | **%N from manure** | **Net emissions (g CO_{2eq}/MJ HVO)²** | **Soil carbon accumulation (tonne CO₂/ha/y)** | **CI³ (g CO_{2eq}/MJ)** | **GHG reduction ^{3,4}** |
| Farm D | 79 | 118 | 40 | -219.9 | 4.421 | -205.5 | 345% |
| Farm M | 70 | 134 | 34 | -164 | 3.784 | -149.5 | 278% |
| Farm A | 70 | 0 | 100 | -151.4 | 3.784 | -136.9 | 263% |
| Farm K | 70 | 134 | 34 | -124 | 3.784 | -109.5 | 231% |
| Farm E | 88 | 135 | 39 | -114.4 | 4.421 | -99.9 | 219% |
| Farm L | 70 | 90 | 44 | -103.8 | 4.421 | -89.5 | 207% |
| Farm F | 0 | 101 | 0 | -57 | 1.583 | -42.5 | 151% |
| Farm J | 0 | 125 | 0 | -56.1 | 1.583 | -41.7 | 150% |
| Farm H | 0 | 118 | 0 | -38.4 | 1.583 | -24 | 129% |
| Farm G | 0 | 137 | 0 | -29.3 | 1.583 | -14.8 | 118% |
| Farm C | 0 | 111 | 0 | -18.8 | 1.583 | -4.3 | 105% |
| Farm B | 0 | 179 | 0 | -14.2 | 1.583 | 0.4 | 100% |
| Farm I | 0 | 125 | 0 | -10.8 | 1.583 | 3.6 | 96% |

| | | | | | | | |
|---|---|---|---|---|---|---|---|
| ¹All farms listed cultivated *Brassica carinata* in central Georgia in the winter of 2016-2017 ²Comprises CO₂eq emissions from cultivation, grain drying, and grain transport less E_{sca} value, as described in Example 8 ³Based on a pathway comprising the actual cultivation data for each farm supplemented with default oil extraction and processing emission data as well as simulated oil and fuel transport, storage and distribution emission data ⁴Based on a standard CI for Petroleum diesel of 83.8 CO2 eq/MJ, as per BioGrace emissions calculator v 1.4d | | | | | | | |

### References:

Alemaw, G. (1987). REVIEW ON BREEDING OF ETHIOPIAN MUSTARD (Brassica carinata A, BRAUN). 7th International Rapeseed Congress Poznan, Poland, May 11-14, 1987, Poznan, Poland, GCIRC.
Angus, J., J. Kirkegaard, M. Peoples, M. Ryan, L. Ohlander and L. Hufton (2011). A review of break-crop benefits of Brassicas. 17th Australian Research Assembly on Brassicas, Wagga Wagga, NSW, August 2011. Wagga Wagga, NSW, NSW DPI: 123-127.
Angus, J. F., J. A. Kirkegaard, J. R. Hunt, M. H. Ryan, L. Ohlander and M. B. Peoples (2015). "Break crops and rotations for wheat." Crop and Pasture Science 66(6): 523.
Blackshaw, R., E. Johnson, Y. Gan, W. May, D. McAndrew, V. Barthet, T. McDonald and D. Wispinski (2011). "Alternative oilseed crops for biodiesel feedstock on the Canadian prairies." Canadian Journal of Plant Science 91(5): 889-896.
Bouaid, A., Y. Diaz, M. Martinez and J. Aracil (2005). "Pilot plant studies of biodiesel production using Brassica carinata as raw material." Catalysis Today 106(1-4): 193-196.
Cardone, M., M. Mazzoncini, S. Menini, V. Rocco, A. Senatore, M. Seggiani and S. Vitolo (2003). "Brassica carinata as an alternative oil crop for the production of biodiesel in Italy: agronomic evaluation, fuel production by transesterification and characterization." Biomass and Bioenergy 25(6): 623-636.
Cardone, M., M. V. Prati, V. Rocco, M. Seggiani, A. Senatore and S. Vitoloi (2002). "Brassica carinata as an alternative oil crop for the production of biodiesel in Italy: engine performance and regulated and unregulated exhaust emissions." Environ Sci Technol 36(21): 4656-4662.
DeJong, S., K. Antonissen, R. Hoefnagels, L. Lonza, M. Wang, A. Faaig, and M. Junginger (2017). "Life-cycle analysis of greenhouse gase emissions from renewable jet fuel producction". Biotechnol. Biofuels 10: 64.
DeLucchi, M. A. (1991). Emissions of Greenhouse Gases from the Use of Transportation Fuels and Electricity. ANL/ESD/TM-22, Argonne National Laboratory. 1: 155.
Drenth, A. C., D. B. Olsen, P. E. Cabot and J. J. Johnson (2014). "Compression ignition engine performance and emission evaluation of industrial oilseed biofuel feedstocks camelina, carinata, and pennycress across three fuel pathways." Fuel 136(0): 143-155.
Drenth, A. C., D. B. Olsen and K. Denef (2015). "Fuel property quantification of triglyceride blends with an emphasis on industrial oilseeds camelina, carinata, and pennycress." Fuel 153: 19-30.
Duca, D., G. Toscano, G. Riva, C. Mengarelli, G. Rossini, A. Pizzi, A. Del Gatto and E. F. Pedretti (2015). "Quality of residues of the biodiesel chain in the energy field." Industrial Crops and Products 75: 91-97.
Gan, Y. T., C. A. Campbell, H. H. Janzen, R. Lemke, L. P. Liu, P. Basnyat and C. L. McDonald (2009). "Root mass for oilseed and pulse crops: Growth and distribution in the soil profile." Can. J. Plant Sci. 89: 883-893.
Gan, Y. T., C. A. Campbell, H. H. Janzen, R. L. Lemke, P. Basnyat and C. L. McDonald (2009). "Carbon input to soil from oilseed and pulse crops on the Canadian prairies." Agriculture, Ecosystems & Environment 132(3-4): 290-297.
Gasol, C., X. Gabarrell, A. Anton, M. Rigola, J. Carrasco, P. Ciria, M. L. Solano and J. Rieradevall (2007). "Life cycle assessment of a Brassica carinata bioenergy cropping system in southern Europe." Biomass and Bioenergy 31(8): 543-555.
Gasol, C. M., S. Martinez, M. Rigola, J. Rieradevall, A. Anton, J. Carrasco, P. Ciria and X. Gabarrell (2009). "Feasibility assessment of poplar bioenergy systems in the Southern Europe." Renewable and Sustainable Energy Reviews 13(4): 801-812.
Gesch, R. W., T. A. Isbell, E. A. Oblath, B. L. Allen, D. W. Archer, J. Brown, J. L. Hatfield, J. D. Jabro, J. R. Kiniry, D. S. Long and M. F. Vigil (2015). "Comparison of several Brassica species in the north central U.S. for potential jet fuel feedstock." Industrial Crops and Products 75b: 2-7.
Hay, R. K. M. (1995). "Harvest index: a review of its use in plant breeding and crop physiology." Annals of Applied Biology 126(1): 197-216.
Hume, D. J. and A. K. H. Jackson (1981). "Frost Tolerance in Soybeans 1." Crop Science 21(5): 689-692.
Jobbagy, E. G. and R. B. Jackson (2000). "The Vertical Distribution Of Soil Organic Carbon And Its Relation To Climate And Vegetation." Ecological Applications 10(2): 423-436.
Johnson, E. N., S. S. Malhi, L. M. Hall and S. Phelps (2013). "Effects of nitrogen fertilizer application on seed yield, N uptake, N use efficiency, and seed quality of Brassica carinata." Canadian Journal of Plant Science 93(6): 1073-1081.
Kirkegaard, J. A. and M. Sarwar (1998). "Biofumigation potential of Brassicas." Plant and Soil 201(1): 71-89.
Lal, R. (2008). "Carbon sequestration." Philos Trans R Soc Lond B Biol Sci 363(1492): 815-830.
Lal, R. (2008). Crop Residues and Soil Carbon. FAO Conservation Agriculture Carbon Offset Consultation: 1-14.
Mnzava, N. A. and R. R. Schippers. (2007). "Brassica carinata A.Braun. [Internet] Record from PROTA4U." Plant Resources of Tropical Africa / Ressources vegetales de l'Afrique tropicale, from https://www.prota4u.org/protav8.asp?h=M4&t=Brassica,carinata&p=Brassica+carinata#Synonyms.
Nagaharu, U. (1935). "Genome analysis in Brassica with special reference to the experimental formation of B. napus and peculiar mode of fertilization." Japanese Journal of Botany 7: 389-452.
Neeft, J., S. t. Buck, T. Gerlagh, B. Gagnepain, D. Bacovsky, N. Ludwiczek, P. Lavelle, G. Thonier, Y. Lechón, C. Lago, I. Herrera, K. Georgakopoulos, N. Komioti, H. Fehrenbach, A. Hennecke, M. Parikka, L. Kinning and P. Wollin (2012). BioGrace Publishable final report Institute for Energy and Environmental Research (IFEU): 28.
Newman, Y. C., D. L. Wright, C. Mackowiak, J. M. S. Scholberg, C. M. Cherr and C. G. Chambliss (2010 (revised)). Cover Crops I. Extension and U. o. Florida.
Nguyen, C. (2003). "Rhizodeposition of organic C by plants: mechanisms and controls." Agronomic 23: 375-396.
Pan, X., C. D. Caldwell, K. C. Falk and R. Lada (2012). "The effect of cultivar, seeding rate and applied nitrogen on Brassica carinata seed yield and quality in contrasting environments." Canadian Journal of Plant Science 92(5): 961-971.
Rahman, M. and M. Tahir (2010). "Inheritance of seed coat color of Ethiopian mustard (Brassica carinata A. Braun)." Canadian Journal of Plant Science 90(3): 279-281.
Seepaul, R., C. M. Bliss, D. L. Wright, J. J. Marois, R. Leon, N. Dufault, S. George and S. M. Olson (2015). Carinata, the Jet Fuel Cover Crop: 2016 Production Recommendations for the Southeastern United States. Agronomy Department, IFAS Extension and U. o. Florida, University of Florida. SS-AGR-384: 1-8**.**
Seepaul, R., S. George and D. L. Wright (2016). "Comparative response of Brassica carinata and B. napus vegetative growth, development and photosynthesis to nitrogen nutrition." Industrial Crops and Products 94: 872-883.
Sherwani, S. I., I. A. Arif and H. A. Khan (2015). Modes of Action of Different Classes of Herbicides. Herbicides, Physiology of Action, and Safety. A. Price, J. Kelton and L. Sarunaite. Rijeka, InTech: Ch. 08.
Shrestha, B. M., R. L. Desjardins, B. G. McConkey, D. E. Worth, J. A. Dyer and D. D. Cerkowniak (2014). "Change in carbon footprint of canola production in the Canadian Prairies from 1986 to 2006." Renewable Energy 63: 634-641.
Wang, M. Q. (1996). GREET 1.0 -- Transportation fuel cycles model: Methodology and use,; Argonne National Lab., IL (United States): Medium: ED; Size: 74 p.
Wisner, R. (2010). Corn and Soybean Availability for Biofuels in 2010-11. AgMRC Renewable Energy & Climate Change Newsletter, Agricultural Marketing Resource Center 10.

Although the foregoing invention has been described in some detail by way of illustration and example for purposes of clarity of understanding, it is readily apparent to those of ordinary skill in the art in light of the teachings of this invention that certain changes and modifications may be made thereto without departing from the scope of the appended claims.

It must be noted that as used in this specification and the appended claims, the singular forms "a," "an," and "the" include plural reference unless the context clearly dictates otherwise. Unless defined otherwise all technical and scientific terms used herein have the same meaning as commonly understood to one of ordinary skill in the art to which this invention belongs.

The phrase "and/or," as used herein in the specification and in the claims, should be understood to mean "either or both" of the elements so conjoined, i.e., elements that are conjunctively present in some cases and disjunctively present in other cases.

Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to encompass the same meaning as "and/or" as defined above. For example, when separating items in a list, "or" or "and/or" shall be interpreted as being inclusive, i.e. , the inclusion of at least one, but also including more than one, of a number or list of elements, and, optionally, additional unlisted items.

As used herein, whether in the specification or the appended claims, the transitional terms "comprising", "including", "carrying", "having", "containing", "involving", and the like are to be understood as being inclusive or open-ended (i.e., to mean including but not limited to), and they do not exclude unrecited elements, materials or method steps. Only the transitional phrases "consisting of and "consisting essentially of, respectively, are closed or semi-closed transitional phrases with respect to claims and exemplary embodiment paragraphs herein. The transitional phrase "consisting of excludes any element, step, or ingredient which is not specifically recited. The transitional phrase "consisting essentially of" limits the scope to the specified elements, materials or steps and to those that do not materially affect the basic characteristic(s) of the invention disclosed and/or claimed herein.

## Claims

1. A method for producing a low carbon intensity biofuel, the method comprising:
a) obtaining oilseed feedstock produced by a method comprising:
i. planting a *Brassica carinata* variety as a second crop in rotation with a first crop or to replace fallow, and implementing land management practices to reduce use of fossil fuel inputs and to maximize capture of atmospheric carbon by plant material of the *Brassica carinata* variety;
ii. harvesting the *Brassica carinata* variety to obtain grain;
iii. returning about 70% to about 90% of all plant material from the *Brassica carinata* variety, aside from the grain, to the soil;
iv. extracting oil from the harvested grain to produce the oilseed feedstock, and
b) producing the low carbon intensity biofuel from the oilseed feedstock.

2. The method of claim 1, wherein said land management practices comprise one or more of:
i. no-tillage,
ii. using between 30 to 165 kg/ha of inorganic nitrogen fertilizer to cultivate the *Brassica carinata* variety, and
iii. using manure to provide from 20% to 100% of the total nitrogen fertilizer used to cultivate the *Brassica carinata* variety.

3. The method of claim 1 or 2, wherein the method for producing said oilseed feedstock further comprises planting the *Brassica carinata* variety immediately following a harvest or concomitant with the harvest of the first crop for sequential crop production without an intervening fallow period.

4. The method of any one of claims 1 to 3, wherein the method for producing said oilseed feedstock further comprises planting a new crop that can be the same as the first crop, or different from the first crop, but that is not *Brassica carinata,* immediately after or concomitant with the harvest of *Brassica carinata* without an intervening fallow period.

5. The method of any one of claims 1 to 4, wherein the first crop is:
a leguminous crop, preferably peanut, soybean, lentil, bean or pea; or
a cereal crop, preferably wheat, barley, rye, oats or corn; or
cotton; or
sesame.

6. The method of any one of claims 1 to 5, wherein:
the growing environment is in a region with a tropical moist climate, and wherein the land management practices comprise planting the *Brassica carinata* in fall or winter for harvest in spring or summer, or planting the *Brassica carinata* in spring for harvest in the fall; or
the growing environment is in a region with a tropical, dry climate, and wherein the land management practices comprise planting the *Brassica carinata* in fall or winter for harvest in spring or summer; or
the growing environment is in a region with a cool temperate, dry climate, and wherein the land management practices comprise planting the *Brassica carinata* in spring for harvest in summer or the fall; or
the growing environment is in a region with a cool temperate, moist climate, and wherein the land management practices comprise planting the *Brassica carinata* in spring for harvest in summer or the fall; or
the growing environment is in a region with a warm temperate, moist climate, and wherein the land management practices comprise planting the *Brassica carinata* in fall or winter for harvest in spring or summer; or
the growing environment is in a region with a warm temperate, dry climate, and wherein the land management practices comprise planting the *Brassica carinata* in fall or winter for harvest in spring or summer.

7. The method of any one of claims 1 to 6, wherein the harvesting is by combine harvester.

8. The method of claim 7, wherein the harvesting is by direct combining.

## Patentansprüche

1. Verfahren zum Herstellen eines Biokraftstoffs mit geringer Kohlenstoffintensität, wobei das Verfahren Folgendes umfasst:
a)Erlangen eines Ölsaat-Rohmaterials, das durch ein Verfahren hergestellt wird, das Folgendes umfasst:
i.Anpflanzen einer *Brassica carinata-Sorte* als zweite Nutzpflanze im Wechsel mit einer ersten Nutzpflanze oder als Ersatz für Brachland und Umsetzen von Landbewirtschaftungspraktiken, um den Einsatz fossiler Brennstoffe zu reduzieren und die Bindung von atmosphärischem Kohlenstoff durch Pflanzenmaterial der *Brassica* carinata-Sorte zu maximieren;
ii.Ernten der *Brassica* carinata-Sorte, um Getreide zu erlangen;
iii.Rückführen von etwa 70 % bis etwa 90 % des gesamten Pflanzenmaterials der *Brassica* carinata-Sorte, abgesehen von dem Getreide, in den Boden;
iv.Extrahieren von Öl aus dem geernteten Getreide, um das Ölsaat-Rohmaterial herzustellen, und
b)Herstellen des Biokraftstoffs mit geringer Kohlenstoffintensität aus dem Ölsaat-Rohmaterial.

2. Verfahren nach Anspruch 1, wobei die Landbewirtschaftungspraktiken eines oder mehrere der Folgenden umfassen:
i.keine Bodenbewirtschaftung,
ii.Verwenden von zwischen 30 bis 165 kg/ha anorganischem Stickstoffdünger zum Anbauen der *Brassica* carinata-Sorte und
iii.Verwenden von Dung, um 20 % bis 100 % des gesamten Stickstoffdüngers, der zum Anbauen der *Brassica* carinata-Sorte verwendet wird, bereitzustellen.

3. Verfahren nach Anspruch 1 oder 2, wobei das Verfahren zum Herstellen des Ölsaat-Rohstoffs ferner Anpflanzen der *Brassica* carinata-Sorte sofort nach einer Ernste oder gleichzeitig mit der Ernte der ersten Nutzpflanze zu aufeinanderfolgendem Nutzpflanzenherstellung ohne eine dazwischenliegende Brachzeit umfasst.

4. Verfahren nach Anspruch 1 bis 3, wobei das Verfahren zum Herstellen des Ölsaat-Rohstoffs ferner Anpflanzen einer neuen Nutzpflanze, die die gleiche wie die erste Nutzpflanze sein kann oder sich von der ersten Nutzpflanze unterscheiden kann, die aber nicht *Brassica carinata* ist, sofort nach oder gleichzeitig mit der Ernte von *Brassica carinata* ohne eine dazwischenliegende Brachzeit umfasst.

5. Verfahren nach einem der Ansprüche 1 bis 4, wobei die erste Nutzpflanze eine der Folgenden ist:
eine hülsenfruchtartige Nutzpflanze, bevorzugt Erdnuss, Sojabohne, Linse, Bohne oder Erbse; oder
eine getreideartige Nutzpflanze, bevorzugt Weizen, Gerste, Roggen, Hafer oder Mais; oder
Baumwolle; oder
Sesam.

6. Verfahren nach einem der Ansprüche 1 bis 5, wobei
die Wachstumsumgebung sich in einer Region mit einem tropischen, feuchtem Klima befindet und wobei die Landbewirtschaftungspraktiken Anpflanzen der *Brassica carinata* im Herbst oder Winter für eine Ernte im Frühjahr oder Sommer oder Anpflanzen der *Brassica carinata* im Frühjahr für eine Ernte im Herbst umfassen; oder
die Wachstumsumgebung sich in einer Region mit tropischem, trockenem Klima befindet und wobei die Landbewirtschaftungspraktiken Anpflanzen der *Brassica carinata* im Herbst oder Winter für eine Ernte im Frühjahr oder Sommer umfassen; oder
die Wachstumsumgebung sich in einer Region mit kühlem, gemäßigtem und trockenem Klima befindet und wobei die Landbewirtschaftungspraktiken Anpflanzen der *Brassica carinata* im Frühling für eine Ernte im Sommer oder Herbst umfassen;
die Wachstumsumgebung sich in einer Region mit kühlem, gemäßigtem, feuchtem Klima befindet und wobei die Landbewirtschaftungspraktiken Anpflanzen der *Brassica carinata* im Frühling für eine Ernte im Sommer oder Herbst umfassen; oder die Wachstumsumgebung sich in einer Region mit warmem, gemäßigtem, feuchtem Klima befindet und wobei die Landbewirtschaftungspraktiken Anpflanzen der *Brassica carinata* im Herbst oder Winter für eine Ernte im Frühjahr oder Sommer umfassen; oder.
die Wachstumsumgebung sich in einer Region mit warmem, gemäßigtem, trockenem Klima befindet und wobei die Landbewirtschaftungspraktiken Anpflanzen der *Brassica carinata* im Herbst oder Winter für eine Ernte im Frühjahr oder Sommer umfassen.

7. Verfahren nach einem der Ansprüche 1 bis 6, wobei das Ernten mit einem Mähdrescher erfolgt.

8. Verfahren nach Anspruch 7, wobei das Ernten durch Direktmähdreschen erfolgt.

## Revendications

1. Procédé de production d'un biocarburant à faible intensité de carbone, le procédé comprenant :
a)l'obtention d'une matière première à base d'oléagineux produite par un procédé comprenant :
i.la plantation d'une variété de *Brassica carinata* comme seconde culture en rotation avec une première culture ou pour remplacer une jachère, et la mise en place de pratiques de gestion des terres pour réduire l'utilisation d'intrants de combustible fossile et maximiser la capture du carbone atmosphérique par le matériel végétal de la variété *Brassica carinata ;*
ii.la récolte de la variété *Brassica carinata* pour obtenir des céréales ;
iii.la remise au sol d'environ 70 % à environ 90 % de tout le matériel végétal de la variété *Brassica carinata,* à l'exception des céréales ;
iv.l'extraction d'huile à partir des céréales récoltées pour produire la matière première à base d'oléagineux, et
b)la production du biocarburant à faible intensité de carbone à partir de la matière première à base d'oléagineux.

2. Procédé selon la revendication 1, dans lequel lesdites pratiques de gestion des terres comprennent un ou plusieurs des éléments suivants :
i.la culture sans labour,
ii.l'utilisation d'entre 30 et 165 kg/ha d'engrais azoté inorganique pour cultiver la variété *Brassica carinata,* et
iii. l'utilisation de fumier pour fournir de 20 à 100 % de l'engrais azoté total utilisé pour cultiver la variété *Brassica carinata.*

3. Procédé selon la revendication 1 ou 2, dans lequel le procédé de production de ladite matière première à base d'oléagineux comprend en outre la plantation de la variété *Brassica carinata* immédiatement après une récolte ou en même temps que la récolte de la première culture pour une production agricole séquentielle sans période de jachère intermédiaire.

4. Procédé selon l'une quelconque des revendications 1 à 3, dans lequel le procédé de production de ladite matière première à base d'oléagineux comprend en outre la plantation d'une nouvelle culture qui peut être la même que la première culture, ou différente de la première culture, mais qui n'est pas *Brassica carinata,* immédiatement après ou en même temps que la récolte de *Brassica carinata* sans période de jachère intermédiaire.

5. Procédé selon l'une quelconque des revendications 1 à 4, dans lequel la première culture est :
une culture de légumineuse, de préférence l'arachide, le soja, la lentille, le haricot ou le pois ; ou
une culture céréalière, de préférence du blé, de l'orge, du seigle, de l'avoine ou du maïs ; ou
le coton ; ou
le sésame.

6. Procédé selon l'une quelconque des revendications 1 à 5, dans lequel :
l'environnement de croissance se trouve dans une région dotée d'un climat tropical humide, et dans lequel les pratiques de gestion des terres comprennent la plantation de *Brassica carinata* en automne ou en hiver pour une récolte au printemps ou en été, ou la plantation de *Brassica carinata* au printemps pour une récolte à l'automne ; ou
l'environnement de croissance se trouve dans une région dotée d'un climat tropical sec, et dans lequel les pratiques de gestion des terres comprennent la plantation de *Brassica carinata* en automne ou en hiver pour une récolte au printemps ou en été ; ou
l'environnement de croissance se trouve dans une région dotée d'un climat tempéré frais et sec, et dans lequel les pratiques de gestion des terres comprennent la plantation de *Brassica carinata* au printemps pour une récolte en été ou à l'automne ; ou
l'environnement de croissance se trouve dans une région dotée d'un climat tempéré frais et humide, et dans lequel les pratiques de gestion des terres comprennent la plantation de *Brassica carinata* au printemps pour une récolte en été ou à l'automne ; ou
l'environnement de croissance se trouve dans une région dotée d'un climat tempéré chaud et humide, et dans lequel les pratiques de gestion des terres comprennent la plantation de *Brassica carinata* en automne ou en hiver pour une récolte au printemps ou en été ; ou
l'environnement de croissance se trouve dans une région dotée d'un climat tempéré chaud et sec, et dans lequel les pratiques de gestion des terres comprennent la plantation de *Brassica carinata* en automne ou en hiver pour une récolte au printemps ou en été.

7. Procédé selon l'une quelconque des revendications 1 à 6, dans lequel la récolte s'effectue par moissonneuse-batteuse.

8. Procédé selon la revendication 7, dans lequel la récolte s'effectue par moissonnage-battage.
